# EUROPEAN PATENT APPLICATION

(11) **EP 4 385 519 A1**
(43) Date of publication of application: **19.06.2024**
(21) Application number: 22855898.7
(22) Date of filing: 10.08.2022
(51) Int. Cl.: A61K 39/00, A61P 35/00, A61P 35/02, A61P 43/00, C07K 7/08

(54) **PHARMACEUTICAL COMPOSITION AND METHOD FOR TREATMENT OR PREVENTION OF CANCER**

(30) Priority: 12.08.2021 JP 2021131636
(71) Applicant: International Institute of Cancer Immunology, Inc., Osaka 564-0053 (JP)
(72) Inventor: KOIDO, Shigeo, Tokyo 167-0052 (JP); SUGIYAMA, Haruo, Minoo-shi, Osaka 562-0036 (JP)
(74) Representative: Vossius & Partner Patentanwälte Rechtsanwälte mbB
(86) International application number: PCT/JP2022/030546
(87) International publication number: WO 2023/017836

(57) **Abstract**

The present disclosure includes a pharmaceutical composition for treating or preventing a cancer in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject comprising a peptide consisting of the amino acid sequence of WAPVLDFAPPGASAYGSL (SEQ ID NO: 2) or an altered peptide thereof having an ability to induce CTLs or a pharmaceutically acceptable salt thereof.

## Description

### TECHNICAL FIELD

The present application claims the priority of Japanese Patent Application No. 2021-131636, which is incorporated herein by reference in its entirety.

The present disclosure belongs to the field of cancer immunotherapy, and particularly relates to use of cancer antigen peptides derived from WT1 protein.

### BACKGROUND ART

Cellular immunity, particularly cytotoxic T cells (also called as cytotoxic T lymphocytes, hereinafter referred to as CTLs) play an important role in cancer cell clearance by a living body. CTLs, which attack cancer cells, are derived from precursor T cells that have recognized a peptide from a cancer antigen protein complexed with an MHC class I molecule through their differentiation and proliferation. To enhance CTL functions, activation of helper T cells is also known to be important. Helper T cells are derived from precursor T cells that have recognized a complex of a cancer antigen peptide and an MHC class II molecule.

WT1 gene has been isolated as a responsible gene of Wilms tumor, which is a kidney cancer in children. Leukemia and some solid cancers are known to be associated with high expression of the gene product WT1. WT1 protein is one of cancer antigen proteins of strong interest for use in cancer vaccines. As to the WT1 protein, WT1₃₄₋₅₁ peptide (WAPVLDFAPPGASAYGSL) (SEQ ID NO: 2) is known to bind to an MHC class II molecule and induce helper T cells (Patent Document 1).

### CITATION LIST

### PATENT DOCUMENTS

Patent Document 1: WO2010/123065

### SUMMARY OF THE INVENTION

### PROBLEMS TO BE SOLVED BY THE INVENTION

An object of the present disclosure is to expand the range of subjects to which WT1₃₄₋₅₁ peptide or a pharmaceutical composition comprising the peptide is applicable.

### SOLUTIONS TO THE PROBLEMS

In one aspect, the present disclosure provides a pharmaceutical composition for treating or preventing a cancer in an HLA-A* 11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject comprising a peptide consisting of the amino acid sequence of WAPVLDFAPPGASAYGSL (SEQ ID NO: 2) or an altered peptide thereof having an ability to induce CTLs or a pharmaceutically acceptable salt thereof.

### EFFECTS OF THE INVENTION

The WT1₃₄₋₅₁ peptide or a pharmaceutical composition comprising the peptide enables the treatment or prevention of a cancer in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject.

### BRIEF DESCRIPTION OF THE DRAWINGS

Fig. 1 shows the frequency of IFN-γ-producing CD8-positive T cells when NAD cells from an HLA-A*02:01/24:02-positive subject (1 case) were stimulated with mDC/WT1₃₄₋₅₁ four times and then restimulated with imDC/WT1₃₄₋₅ or imDC.
Fig. 2 shows the frequency of IFN-γ-producing CD8-positive T cells when NAD cells from an HLA-A*02:01/24:02-positive subject (1 case) were stimulated with mDC/WT1₃₄₋₅₁ four times and then restimulated with imDC/WT1₃₇₋₄₅, or when the NAD cells were stimulated with imDC/WT1₃₇₋₄₅ only once without the four stimulations.
Fig. 3 shows the frequency of IFN-γ-producing CD8-positive T cells when NAD cells from an HLA-A* 02:06/26:03-positive subject (1 case) were stimulated with mDC/WT1₃₄₋₅₁ four times and then restimulated with imDC/WT1₃₇₋₄₅ or imDC.
Fig. 4 shows the frequency of IFN-γ-producing CD8-positive T cells when NAD cells from an HLA-A* 02:07/24:02-positive subject (1 case) were stimulated with mDC/WT1₃₄₋₅₁ four times and then restimulated with imDC/WT1₃₇₋₄₅ or imDC.
Fig. 5 shows the frequency of IFN-γ-producing CD8-positive T cells when NAD cells from an HLA-A* 26:01/24:02-positive subject (1 case) were stimulated with mDC/WT1₃₄₋₅₁ or mDC four times and then restimulated with imDC/WT1₄₀₋₄₈.
Fig. 6 shows the frequency of CD8-positive and IFN-γ-positive, CD8-positive and TNF-α-positive, or CD8-positive, IFN-γ-positive and TNF-α-positive T cells (%) when PBMCs of HLA-A* 02:01/24:02-positive subject (1 case) were stimulated with WT1₃₄₋₅₁ and the compound of formula (5) and then stimulated with WT1₃₇₋₄₅ or WT1_{40-48.}
Fig. 7 shows the frequency of HLA-A*02:01 WT1₃₇₋₄₅ tetramer-positive cells when PBMCs from an HLA-A*02:01/24:02-positive subject (1 case) were stimulated with WT1₃₄₋₅₁ and the compound of formula (5).
Fig. 8 shows the frequency of CD8-positive and IFN-γ-positive, CD8-positive and TNF-α-positive, or CD8-positive, IFN-γ-positive and TNF-α-positive T cells (%) when PBMCs of HLA-A* 02:06/24:02-positive subject (1 case) were stimulated with WT1₃₄₋₅₁ and the compound of formula (5) and then stimulated with WT1₃₇₋₄₅ or WT1₄₀₋₄₈.
Fig. 9 shows the frequency of HLA-A*02:01 WT1₃₇₋₄₅ tetramer-positive cells when PBMCs from an HLA-A* 02:06/24:02-positive subject (1 case) were stimulated with WT1₃₄₋₅₁ and the compound of formula (5).
Fig. 10 shows the frequency of CD8-positive and IFN-γ-positive, CD8-positive and TNF-α-positive, or CD8-positive, IFN-γ-positive and TNF-α-positive T cells (%) when PBMCs of HLA-A* 02:07/24:02-positive subject (1 case) were stimulated with WT1₃₄₋₅₁ and the compound of formula (5) and then stimulated with WT1₃₇₋₄₅ or WT1₄₀₋₄₈.
Fig. 11 shows the frequency of HLA-A*02:01 WT1₃₇₋₄₅ tetramer-positive cells when PBMCs from an HLA-A* 02:07/24:02-positive subject (1 case) were stimulated with WT1₃₄₋₅₁ and the compound of formula (5).
Fig. 12 shows the frequency of CD8-positive and IFN-γ-positive, CD8-positive and TNF-α-positive, or CD8-positive, IFN-γ-positive and TNF-α-positive T cells (%) when PBMCs of HLA-A* 24:02/24:02-positive subject (1 case) were stimulated with WT1₃₄₋₅₁ and the compound of formula (5) and then stimulated with WT1₃₇₋₄₅ or WT1₄₀₋₄₈.
Fig. 13 shows the frequency of HLA-A*02:01 WT1₃₇₋₄₅ tetramer-positive cells when PBMCs from an HLA-A* 24:02/24:02-positive subject (1 case) were stimulated with WT1₃₄₋₅₁ and the compound of formula (5).
Fig. 14 shows the frequency of CD8-positive and IFN-γ-positive, CD8-positive and TNF-α-positive, or CD8-positive, IFN-γ-positive and TNF-α-positive T cells (%) when PBMCs of HLA-A* 02:01/24:02-positive subject (1 case) were stimulated with WT1₃₄₋₅₁ and then stimulated with WT1₃₇₋₄₅ or WT1₄₀₋₄₈.
Fig. 15 shows the frequency of HLA-A*02:01 WT1₃₇₋₄₅ tetramer-positive cells when PBMCs from an HLA-A* 02:01/24:02-positive subject (1 case) were stimulated with WT1₃₄₋₅₁.
Fig. 16 shows the frequency of CD8-positive and IFN-γ-positive, CD8-positive and TNF-α-positive, or CD8-positive, IFN-γ-positive and TNF-α-positive T cells (%) when PBMCs of HLA-A* 02:06/24:02-positive subject (1 case) were stimulated with WT1₃₄₋₅₁ and then stimulated with WT1₃₇₋₄₅ or WT1₄₀₋₄₈.
Fig. 17 shows the frequency of HLA-A*02:01 WT1₃₇₋₄₅ tetramer-positive cells when PBMCs from an HLA-A* 02:06/24:02-positive subject (1 case) were stimulated with WT1₃₄₋₅₁.
Fig. 18 shows the frequency of CD8-positive and IFN-γ-positive, CD8-positive and TNF-α-positive, or CD8-positive, IFN-γ-positive and TNF-α-positive T cells (%) when PBMCs of HLA-A* 02:07/24:02-positive subject (1 case) were stimulated with WT1₃₄₋₅₁ and then stimulated with WT1₃₇₋₄₅ or WT1₄₀₋₄₈.
Fig. 19 shows the frequency of HLA-A*02:01 WT1₃₇₋₄₅ tetramer-positive cells when PBMCs from an HLA-A* 02:07/24:02-positive subject (1 case) were stimulated with WT1₃₄₋₅₁.
Fig. 20 shows the frequency of CD8-positive and IFN-γ-positive, CD8-positive and TNF-α-positive, or CD8-positive, IFN-γ-positive and TNF-α-positive T cells (%) when PBMCs of HLA-A* 24:02/24:02-positive subject (1 case) were stimulated with WT1₃₄₋₅₁ and then stimulated with WT1₃₇₋₄₅ or WT1₄₀₋₄₈.
Fig. 21 shows the frequency of HLA-A*02:01 WT1₃₇₋₄₅ tetramer-positive cells when PBMCs from an HLA-A* 24:02/24:02-positive subject (1 case) were stimulated with WT1₃₄₋₅₁.
Fig. 22 is a graph showing the frequency of IFN-γ-positive and/or TNF-α-positive T cells in CD8-positive T cells (%) when PBMCs from an HLA-A* 01:01-positive subject (1 case) were stimulated with WT1₃₄₋₅₁ and the compound of formula (5) and then stimulated with WT1₃₇₋₄₅ or WT1₄₀₋₄₈.
Fig. 23 shows the results of flow cytometry showing the frequency of IFN-γ-positive and/or TNF-α-positive T cells in CD8-positive T cells (%) when PBMCs from an HLA-A* 01 :01-positive subject (1 case) were stimulated with WT1₃₄₋₅₁ and the compound of formula (5) and then stimulated with WT1₃₇₋₄₅ or WT1₄₀₋₄₈.
Fig. 24 shows the frequency of HLA-A*01:01 WT1₄₀₋₄₈ tetramer-positive cells when PBMCs from an HLA-A* 01:01-positive subject (1 case) were stimulated with WT1₃₄₋₅₁ and the compound of formula (5).
Fig. 25 is a graph showing the frequency of IFN-γ-positive and/or TNF-α-positive T cells in CD8-positive T cells (%) when PBMCs from an HLA-A* 26:01-positive subject (1 case) were stimulated with WT1₃₄₋₅₁ and the compound of formula (5) and then stimulated with WT1₃₇₋₄₅ or WT1₄₀₋₄₈.
Fig. 26 shows the results of flow cytometry showing the frequency of IFN-γ-positive and/or TNF-α-positive T cells in CD8-positive T cells (%) when PBMCs from an HLA-A* 26:01-positive subject (1 case) were stimulated with WT1₃₄₋₅₁ and the compound of formula (5) and then stimulated with WT1₄₀₋₄₈.
Fig. 27 is a graph showing the frequency of IFN-γ-positive and/or TNF-α-positive T cells in CD8-positive T cells (%) when PBMCs from an HLA-B* 35:01-positive subject (1 case) were stimulated with WT1₃₄₋₅₁ and the compound of formula (5) and then stimulated with WT1₄₀₋₄₈.
Fig. 28 shows the results of flow cytometry showing the frequency of IFN-γ-positive and/or TNF-α-positive T cells in CD8-positive T cells (%) when PBMCs from an HLA-B* 35:01-positive subject (1 case) were stimulated with WT1₃₄₋₅₁ and the compound of formula (5) and then stimulated with WT1₄₀₋₄₈.
Fig. 29 shows the frequency of HLA-B* 35:01 WT1₄₀₋₄₈ tetramer-positive cells when PBMCs from an HLA-B* 35:01-positive subject (1 case) were stimulated with WT1₃₄₋₅₁ and the compound of formula (5).
Fig. 30 is a graph showing the frequency of IFN-γ-positive and/or TNF-α-positive T cells in CD8-positive T cells (%) when PBMCs from an HLA-C* 03:03-positive subject (1 case) were stimulated with WT1₃₄₋₅₁ and the compound of formula (5) and then stimulated with WT1₄₀₋₄₈.
Fig. 31 shows the results of flow cytometry showing the frequency of IFN-γ-positive and/or TNF-α-positive T cells in CD8-positive T cells (%) when PBMCs from an HLA-C* 03:03-positive subject (1 case) were stimulated with WT1₃₄₋₅₁ and the compound of formula (5) and then stimulated with WT1₄₀₋₄₈.
Fig. 32 is a graph showing the frequency of IFN-γ-positive and/or TNF-α-positive T cells in CD8-positive T cells (%) when PBMCs from an HLA-C* 05:01-positive subject (1 case) were stimulated with WT1₃₄₋₅₁ and the compound of formula (5) and then stimulated with WT1₃₇₋₄₅ or WT1₄₀₋₄₈.
Fig. 33 shows the results of flow cytometry showing the frequency of IFN-γ-positive and/or TNF-α-positive T cells in CD8-positive T cells (%) when PBMCs from an HLA-C* 05:01-positive subject (1 case) were stimulated with WT1₃₄₋₅₁ and the compound of formula (5) and then stimulated with WT1₃₇₋₄₅ or WT1₄₀₋₄₈.
Fig. 34 is a graph showing the frequency of IFN-γ-positive and/or TNF-α-positive T cells in CD8-positive T cells (%) when PBMCs from an HLA-C* 07:01-positive subject (1 case) were stimulated with WT1₃₄₋₅₁ and the compound of formula (5) and then stimulated with WT1₄₀₋₄₈.
Fig. 35 shows the results of flow cytometry showing the frequency of IFN-γ-positive and/or TNF-α-positive T cells in CD8-positive T cells (%) when PBMCs from an HLA-C* 07:01-positive subject (1 case) were stimulated with WT1₃₄₋₅₁ and the compound of formula (5) and then stimulated with WT1₄₀₋₄₈.
Fig. 36 is a graph showing the frequency of IFN-γ-positive and/or TNF-α-positive T cells in CD8-positive T cells (%) when PBMCs from an HLA-A* 01:01-positive subject (1 case) were stimulated with WT1₃₄₋₅₁ and then stimulated with WT1₃₇₋₄₅ or WT1₄₀₋₄₈.
Fig. 37 shows the results of flow cytometry showing the frequency of IFN-γ-positive and/or TNF-α-positive T cells in CD8-positive T cells (%) when PBMCs from an HLA-A* 01:01-positive subject (1 case) were stimulated with WT1₃₄₋₅₁ and then stimulated with WT1₃₇₋₄₅ or WT1₄₀₋₄₈.
Fig. 38 shows the frequency of HLA-A* 01:01 WT1₄₀₋₄₈ tetramer-positive cells when PBMCs from an HLA-A* 01:01-positive subject (1 case) were stimulated with WT1₃₄₋₅₁.
Fig. 39 is a graph showing the frequency of IFN-γ-positive and/or TNF-α-positive T cells in CD8-positive T cells (%) when PBMCs from an HLA-A* 26:01-positive subject (1 case) were stimulated with WT1₃₄₋₅₁ and then stimulated with WT1₄₀₋₄₈.
Fig. 40 shows the results of flow cytometry showing the frequency of IFN-γ-positive and/or TNF-α-positive T cells in CD8-positive T cells (%) when PBMCs from an HLA-A* 26:01-positive subject (1 case) were stimulated with WT1₃₄₋₅₁ and then stimulated with WT1₄₀₋₄₈.
Fig. 41 is a graph showing the frequency of IFN-γ-positive and/or TNF-α-positive T cells in CD8-positive T cells (%) when PBMCs from an HLA-B* 35:01-positive subject (1 case) were stimulated with WT1₃₄₋₅₁ and then stimulated with WT1₄₀₋₄₈.
Fig. 42 shows the results of flow cytometry showing the frequency of IFN-γ-positive and/or TNF-α-positive T cells in CD8-positive T cells (%) when PBMCs from an HLA-B* 35:01-positive subject (1 case) were stimulated with WT1₃₄₋₅₁ and then stimulated with WT1₄₀₋₄₈.
Fig. 43 shows the frequency of HLA-B* 35:01 WT1₄₀₋₄₈ tetramer-positive cells when PBMCs from an HLA-B* 35:01-positive subject (1 case) were stimulated with WT1₃₄₋₅₁.
Fig. 44 is a graph showing the frequency of IFN-γ-positive and/or TNF-α-positive T cells in CD8-positive T cells (%) when PBMCs from an HLA-C* 03:03-positive subject (1 case) were stimulated with WT1₃₄₋₅₁ and then stimulated with WT1₄₀₋₄₈.
Fig. 45 shows the results of flow cytometry showing the frequency of IFN-γ-positive and/or TNF-α-positive T cells in CD8-positive T cells (%) when PBMCs from an HLA-C* 03:03-positive subject (1 case) were stimulated with WT1₃₄₋₅₁ and then stimulated with WT1₄₀₋₄₈.
Fig. 46 is a graph showing the frequency of IFN-γ-positive and/or TNF-α-positive T cells in CD8-positive T cells (%) when PBMCs from an HLA-C* 05:01-positive subject (1 case) were stimulated with WT1₃₄₋₅) and then stimulated with WT1₃₇₋₄₅ or WT1₄₀₋₄₈.
Fig. 47 shows the results of flow cytometry showing the frequency of IFN-γ-positive and/or TNF-α-positive T cells in CD8-positive T cells (%) when PBMCs from an HLA-C* 05:01-positive subject (1 case) were stimulated with WT1₃₄₋₅₁ and then stimulated with WT1₃₇₋₄₅ or WT1₄₀₋₄₈.
Fig. 48 is a graph showing the frequency of IFN-γ-positive and/or TNF-α-positive T cells in CD8-positive T cells (%) when PBMCs from an HLA-C* 07:01-positive subject (1 case) were stimulated with WT1₃₄₋₅₁ and then stimulated with WT1₄₀₋₄₈.
Fig. 49 shows the results of flow cytometry showing the frequency of IFN-γ-positive and/or TNF-α-positive T cells in CD8-positive T cells (%) when PBMCs from an HLA-C* 07:01-positive subject (1 case) were stimulated with WT1₃₄₋₅₁ and then stimulated with WT1₄₀₋₄₈.

### DETAILED DESCRIPTION

The "amino acid residue" as used herein refers to a single amino acid unit among amino acids constituting a peptide or a protein molecule. The "amino acid residue" may be a natural or non-natural α-amino acid residue, β-amino acid residue, γ-amino acid residue or δ-amino acid residue; more specifically, a natural α-amino acid residue, ornithine residue, homoserine residue, homocysteine residue, β-alanine residue, γ-aminobutanoic acid or δ-aminopentanoic acid. When an "amino acid residue" is optically active, it includes L-form and D-form, and is preferably L-form.

The abbreviations of "amino acid residues" used herein stand for the followings:
Ala or A: alanine residue
Arg or R: arginine residue
Asn or N: asparagine residue
Asp or D: aspartic acid residue
Cys or C: cysteine residue
Gln or Q: glutamine residue
Glu or E: glutamic acid residue
Gly or G: glycine residue
His or H: histidine residue
Ile or I: isoleucine residue
Leu or L: leucine residue
Lys or K: lysine residue
Met or M: methionine residue
Phe or F: phenylalanine residue
Pro or P: proline residue
Ser or S: serine residue
Thr or T: threonine residue
Trp or W: tryptophan residue
Tyr or Y: tyrosine residue
Val or V: valine residue
Abu: 2-aminobutyric acid residue (also referred to as α-aminobutyric acid residue)
Orn: omithine residue
Cit: citrulline residue

The amino acid sequence of a "peptide" is described herein so that the N-terminal amino acid residue is positioned on the left side and the C-terminal amino acid residue is positioned on the right side, in accordance with a usual description method. Unless otherwise indicated, in the "peptide", the amino group of the N-terminal amino acid residue binds to a hydrogen atom (to be a free amino group) and the carbonyl group of the C-terminal amino acid residue binds to a hydroxyl group. A divalent peptide group refers to a peptide group which is able to bind to other chemical moieties via the N-terminal amino group and via the C-terminal carbonyl group. In a peptide corresponding to a partial structure of a compound represented by the formula (1), for example, the compound of formula (4) or formula (5), unless otherwise indicated, the amino group of the N-terminal amino acid residue binds to a hydrogen atom and the carbonyl group of the C-terminal amino acid residue binds to a hydroxyl group.

As used herein, the term cancer antigen peptide encompasses peptides consisting of an amino acid sequence of a portion of a cancer antigen protein, altered peptides thereof, and conjugates thereof. The cancer antigen peptide includes MHC class I-restricted peptides, MHC class II-restricted peptides, and conjugates thereof.

The term "MHC class I-restricted" means an ability of a peptide to bind to a Major Histocompatibility Complex (MHC) class I molecule and induce CTLs. The term "MHC class I-restricted peptide" herein refers to a peptide being capable of binding to an MHC class I molecule and inducing cytotoxic T cells (CTLs) *in vitro* and/or *in vivo* (that is, a peptide having an ability to induce CTLs). The term "MHC class I-restricted peptide" is also referred to as a "killer peptide".

MHC is called as a human leukocyte-type antigen (HLA) in humans. HLA molecules corresponding to MHC class I-molecules are classified into subtypes such as HLA-A, B, C, F and G. Preferable examples of MHC class I restriction include HLA-A, HLA-B and HLA-C restriction.

Allelic polymorphism is known for each HLA subtype. Examples of the allelic polymorphism include HLA-A1, HLA-A2, HLA-A24, HLA-A26, HLA-A3, HLA-A11, HLA-A33, HLA-B7, HLA-B15, HLA-B27, HLA-B35, HLA-B40, HLA-B44, HLA-C3, HLA-C5, HLA-C7, HLA-C1, HLA-C3, HLA-C4, and HLA-C6, as well as HLA-A*01:01, HLA-A*02:01, HLA-A*02:06, HLA-A*02:07, HLA-A*24:02, HLA-A*26:01, HLA-A*26:03, HLA-A*03:01, HLA-A*11:01, HLA-A*33:01, HLA-A*33:03, HLA-B*15:01, HLA-B*27:05, HLA-B*35:01, HLA-B*40:01, HLA-B*40:02, HLA-B*40:03, HLA-B*40:06, HLA-B*44:03, HLA-C*01:01, HLA-C*03:01, HLA-C*03:03, HLA-C*04:01, HLA-C*05:01, HLA-C*06:02, HLA-C*07:01, and HLA-C*07:02.

The term "MHC class II-restricted" means an ability of a peptide to bind to an MHC class II molecule to induce helper T cells. The "MHC class II-restricted peptide" refers to a peptide being capable of binding to an MHC class II molecule and inducing helper T cells *in vitro* and/or *in vivo* (that is, a peptide having an ability to induce helper T cells.). The "MHC class II-restricted peptide" is also referred herein to as a "helper peptide".

HLA molecules corresponding to MHC class II-molecules are classified into subtypes such as HLA-DR, DQ and DP. Preferable examples of MHC class II-restriction include HLA-DR restriction, HLA-DQ restriction and HLA-DP restriction.

Allelic polymorphism is known for each HLA subtype. Examples of the allelic polymorphism include DRB1*0101, DRB1*0405, DRB1*0802, DRB1*0803, DRB1*0901, DRB 1 * 1201, DRB 1 * 1403, DRB1*1501, DRB 1 * 1502, DPB1*0201, DPB 1 *0202, DPB1*0402, DPB 1 *0501, DPB1*0901, DQB 1 *0301, DQB1*0302, DQB1*0401, DQB1*0501, DQB1*0601, DQBF0602 and DRB5*0102.

As used herein, the term "altered peptide" refers to a peptide consisting of an amino acid sequence which has one or several amino acid residues altered in the amino acid sequence of the original peptide. In an altered peptide, one or a plurality of amino acid residues, for example, 1 to 9 amino acid residues, preferably 1 to 5, 1 to 4 or 1 to 3 amino acid residues, more preferably 1 to 2 amino acid residues, or most preferably one amino acid residue is deleted from, substituted in, inserted into, and/or added to the amino acid sequence of the original peptide. The number of amino acid(s) deleted from, substituted in, inserted into and/or added to the amino acid sequence of the original peptide may preferably be 1 to 5, 1 to 4, or 1 to 3, more preferably 1 to 2, or most preferably one. Amino acid substitution for altering a peptide may be made at any position of amino acid residue in the original sequence with any type of amino acid. Conservative amino acid substitution is preferred. For example, substitution of Asp for Glu; Tyr for Phe; Ile for Leu; Ser for Ala; or Arg for His may be made. Amino acid addition or deletion may preferably be made at N- or C-terminus of a peptide. However, amino acid addition or deletion may be made internally. Amino acid substitution, insertion or addition may be made with any of the twenty genetically encoded amino acids or even any non-natural amino acid.

As used herein, a peptide comprising a given amino acid sequence refers to a peptide having the given amino acid sequence which may, as usually understood, optionally have one or more additional amino acid residues attached to the N-terminal and/or C-terminal amino acid of the given sequence. For example, a peptide comprising a given amino acid sequence includes a peptide consisting of the given amino acid sequence and a peptide longer than that peptide. For example, a peptide comprising the amino acid sequence of SEQ ID NO: 2 includes a peptide consisting of the amino acid sequence of SEQ ID NO: 2, and a peptide consisting of the amino acid sequence of SEQ ID NO: 2 and one or more additional amino acid residues attached to the N-terminal and/or C-terminal amino acid of the amino acid sequence of SEQ ID NO: 2.

WT1 is a cancer antigen protein known to be highly expressed in various cancers. A representative amino acid sequence of human WT1 is shown in SEQ ID NO: 1. As used herein, the term "WT1 peptide" refers to a peptide consisting of contiguous amino acid residues in the amino acid sequence of SEQ ID NO: 1. WT1 peptides can be represented with the positions of the contiguous amino acid residues in the amino acid sequence of SEQ ID NO: 1. For example, the WT1₃₄₋₅₁ peptide consists of the amino acid sequence at positions 34-51 of SEQ ID NO: 1 (WAPVLDFAPPGASAYGSL) (SEQ ID NO: 2).

SEQ ID NO: 1
MGSDVRDLNALLPAVPSLGGGGGCALPVSGAAQWAPVLDFAPPGASAYGSLGG
PAPPPAPPPPPPPPPPHSFIKQEPSWGGAEPHEEQCLSAFTVHFSGQFTGTAGACRY

GPFGPPPPSQASSGQARMFPNAPYLPSCLESQPAIRNQGYSTVTFDGTPSYGHTP
SHHAAQFPNHSFKHEDPMGQQGSLGEQQYSVPPPVYGCHTPTDSCTGSQALLL
RTPYSSDNLYQMTSQLECMTWNQMNLGATLKGVAAGSSSSVKWTEGQSNHST
GYESDNHTTPILCGAQYRIHTHGVFRGIQDVRRVPGVAPTLVRSASETSEKRPFM
CAYPGCNKRYFKLSHLQMHSRKHTGEKPYQCDFKDCERRFSRSDQLKRHQRR
HTGVKPFQCKTCQRKFSRSDHLKTHTRTHTGKTSEKPFSCRWPSCQKKFARSDE
LVRHHNMHQRNMTKLQLAL

The WT1₃₄₋₅₁ peptide has been known to function as a helper peptide, but also functions as a killer peptide in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject. Accordingly, a peptide consisting of the amino acid sequence of SEQ ID NO: 2 or an altered peptide thereof having an ability to induce CTLs (herein also referred to as the peptide of the present disclosure) can be used for the treatment or prevention of a cancer in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject. In the present disclosure, the altered peptide of "a peptide consisting of the amino acid sequence of SEQ ID NO: 2" having an ability to induce CTLs is an HLA-A* 11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-restricted peptide. In an embodiment, the altered peptide of "a peptide consisting of the amino acid sequence of SEQ ID NO: 2" has both the ability to induce CTLs and the ability to induce helper T cells.

The HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject refers to a subject each having HLA haplotypes including HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02. The HLA alleles (HLA types) are specified by HLA typing (particularly, genotyping) ordinarily used. Examples of the "subject" include human and non-human animals such as non-human primate, ovine, canine, feline, equine, and bovine. A human subject is preferred.

In an embodiment, the subject is an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject. In an embodiment, the subject is an HLA-A* 11:01, HLA-A*02:01, HLA-A*02:06, HLA-A*02:07, HLA-A*26:01, or HLA-A*26:03-positive subject. In an embodiment, the subject is an HLA-A`02:01, HLA-A*02:06, HLA-A*02:07, or HLA-A*26:01-positive subject. In an embodiment, the subject is an HLA-A*02:01, HLA-A*02:06, or HLA-A*02:07-positive subject. In an embodiment, the subject is an HLA-A*11:01, HLA-A*26:01, or HLA-A*26:03-positive subject. In an embodiment, the subject is an HLA-A*11:01 or HLA-A*26:03-positive subject. In an embodiment, the subject is an HLA-A*11:01-positive subject. In an embodiment, the subject is an HLA-A*02:01-positive subject. In an embodiment, the subject is an HLA-A*02:06-positive subject. In an embodiment, the subject is an HLA-A*02:07-positive subject. In an embodiment, the subject is an HLA-A*26:01-positive subject. In an embodiment, the subject is an HLA-A*26:03-positive subject. In an embodiment, the subject is an HLA-A*01:01, HLA-B*15:01, HLA-B*35:01, HLA-C*03:03, HLA-C*05:01, HLA-C*07:01, or HLA-C*07:02-positive subject. In an embodiment, the subject is an HLA-A*01 :01, HLA-B*35:01, HLA-C*03:03, HLA-C*05:01, or HLA-C*07:01-positive subject. In an embodiment, the subject is an HLA-A*01:01, HLA-B*35:01, HLA-C*03:03, or HLA-C*05:01-positive subject. In an embodiment, the subject is an HLA-A*01:01-positive subject. In an embodiment, the subject is an HLA-B*15:01-positive subject. In an embodiment, the subject is an HLA-B*35:01-positive subject. In an embodiment, the subject is an HLA-C*03:03-positive subject. In an embodiment, the subject is an HLA-C*05:01-positive subject. In an embodiment, the subject is an HLA-C*07:01-positive subject. In an embodiment, the subject is an HLA-C*07:02-positive subject. In an embodiment, the subject is an HLA-A*02:01, HLA-A*02:06, HLA-A*02:07, HLA-A*26:01, HLA-A*01:01, HLA-B*35:01, HLA-C*03:03, HLA-C*05:01, or HLA-C*07:01-positive subject. In an embodiment, the subject is an HLA-A*02:07, HLA-A*26:01, HLA-A*01:01, HLA-B*35:01, HLA-C*03:03, HLA-C*05:01, or HLA-C*07:01-positive subject. In an embodiment, the subject is an HLA-A*02:07, HLA-A*26:01, HLA-A*01:01, HLA-B*35:01, HLA-C*03:03, or HLA-C*05:01-positive subject.

The peptide of the present disclosure may have any length. However, the longer a peptide chain is, the more susceptible it may be to degradation by a proteolytic enzyme, and too small peptide may not successfully be caught in a peptide-binding groove of an MHC molecule. The peptide of the present disclosure typically consists of 9 to 30 amino acid residues, 10 to 25 amino acid residues, 12 to 24 amino acid residues, 15 to 22 amino acid residues, 16 to 20 amino acid residues, or 18 or 19 amino acid residues.

The peptide of the present disclosure may be a peptide consisting of an amino acid sequence of any one of:
WAPVLDFAPPGASAYGSL (SEQ ID NO: 2)
CWAPVLDFAPPGASAYGSL (SEQ ID NO: 3), and
WAPVLDFAPPGASAYGSLC (SEQ ID NO: 4).

In an embodiment, the peptide of the present disclosure is a peptide consisting of the amino acid sequence of SEQ ID NO: 2.

The term "pharmaceutically acceptable salt" as used herein includes an acid addition salt and a base addition salt. The acid addition salt may be an inorganic acid salt, such as hydrochloride, hydrobromide, sulfate, hydroiodide, nitrate, or phosphate, or an organic acid salt such as citrate, oxalate, acetate, formate, propionate, benzoate, trifluoroacetate, maleate, tartrate, methanesulfonate, benzenesulfonate, or p-toluenesulfonate. The base addition salt may be a salt with an inorganic base, such as sodium salt, potassium salt, calcium salt, magnesium salt, or ammonium salt, a salt with an organic base, such as triethylammonium salt, triethanolammonium salt, pyridinium salt, or diisopropylammonium salt. The "pharmaceutically acceptable salt" also includes a salt with a basic or acidic amino acid, such as arginine, aspartic acid, or glutamic acid. The term "peptide" or "compound" used herein includes a peptide or compound in the form of a pharmaceutically acceptable salt, unless the context requires otherwise.

As used herein, the treating a cancer include completely or partially inhibiting progression of a cancer and at least partially reducing one or more symptoms of a cancer. The treating a cancer also include induction of remission, maintenance of remission and suppression of recurrence. The preventing a cancer means preventing development of the cancer in a subject who has not yet developed any cancer. The subject can be, for example, a subject at high risk of developing a cancer. Examples of subjects at high risk of developing a cancer include subjects diagnosed with HBOC (hereditary breast and ovarian cancer syndrome) with mutations in BRCA1 and BRCA2, heavy smokers, elderly persons, and persons infected with hepatitis virus.

In an embodiment, the pharmaceutical composition is a cancer vaccine. In a different embodiment, the pharmaceutical composition is a composition for inducing CTLs in cellular immunotherapy for a cancer.

In an embodiment, the cancer refers to a cancer in which WT1 is expressed or a cancer associated with an elevated expression level of WT1 gene.

In an embodiment, the cancer refers to a blood cancer or a solid cancer. In a further embodiment, the cancer refers to a cancer selected from chronic or acute leukemia including acute myeloid leukemia, chronic myeloid leukemia, acute lymphocytic leukemia, acute lymphoblastic leukemia and chronic lymphocytic leukemia, myelodysplastic syndrome, multiple myeloma, malignant lymphoma, stomach cancer, colon cancer, lung cancer, breast cancer, germ cell carcinoma, liver cancer, skin cancer, bladder cancer, prostate cancer, uterine cancer, cervical cancer, ovarian cancer, brain tumor, glioma, central nervous system primary malignant lymphoma, Hodgkin lymphoma, non-Hodgkin lymphoma, T cell lymphoma, lymphocytic lymphoma, T cell lymphoma, bone cancer, pancreatic cancer, head and neck cancer, skin or intraorbital malignant melanoma, rectal cancer, anal cancer, testicular cancer, carcinoma of fallopian tube, endometrial carcinoma, cervical carcinoma, vaginal carcinoma, vulvar carcinoma, esophageal cancer, small intestinal cancer, endocrine cancer, thyroid cancer, parathyroid cancer, adrenal cancer, sarcoma of soft tissue, urethral cancer, penile cancer, childhood solid tumor, kidney cancer or ureteral cancer, renal pelvic carcinoma, central nervous system tumor, tumor angiogenesis, spinal tumor, brain stem glioma, pituitary adenoma, Kaposi's sarcoma, epidermoid cancer, squamous cell cancer, environmentally induced cancer including asbestos-induced cancer and combinations of these cancers. In a further embodiment, the cancer is selected from acute myeloid leukemia, chronic myeloid leukemia, acute lymphoblastic leukemia, chronic lymphocytic leukemia, myelodysplastic syndrome, multiple myeloma, malignant lymphoma, stomach cancer, colon cancer, lung cancer, breast cancer, germ cell carcinoma, liver cancer, skin cancer, bladder cancer, prostate cancer, uterine cancer, cervical cancer, ovarian cancer, brain tumor and glioma.

The peptide of the present disclosure may be used for treating or preventing a cancer as monotherapy, or in combination with at least one different agent (herein also referred to as concomitant drug).

The peptide of the present disclosure and the concomitant drug may be contained in a single composition or separate compositions. In an embodiment, the peptide of the present disclosure and the concomitant drug may be contained in a single composition. In a different embodiment, the peptide of the present disclosure and the concomitant drug may be contained in separate compositions. A composition comprising any of the active agents may be provided together with instructions of dosage and administration for use of the composition in combination with the other active agent(s). Alternatively, compositions each comprising any of the active agents may be incorporated in a single kit. Such a kit may further comprise instructions of dosage and administration for use of the compositions in combination (including a package insert), and may be packaged in a container. In administration of more than one active agent in combination, the agents may be administered on the same administration schedule or different administration schedules.

In an embodiment, concomitant drugs include different anti-cancer agents, for example, immunomodulators such as immune checkpoint inhibitors and costimulatory molecule agonists, hormone therapy agents, cell growth factors, cell growth factor inhibitors, cell growth factor receptor inhibitors, and chemotherapeutic agents. Examples of cell growth factor inhibitors include vascular endothelial cell growth factor inhibitors (VEGF inhibitors) (e.g., bevacizumab). Examples of immune checkpoint inhibitors include anti-PD-1 antibodies (e.g., nivolumab and pembrolizumab) and anti-PD-L1 antibodies (e.g., durvalumab).

In an embodiment, concomitant drugs include cancer antigen peptides, such as MHC class I-restricted peptides, MHC class II-restricted peptides, conjugates thereof, or pharmaceutically acceptable salts thereof. Examples of cancer antigen peptides include peptides or derivatives thereof, or conjugates thereof described in the following publications: WO2000/006602, WO2000/018795, WO2002/028414, WO2002/079253, WO2003/037060, WO2003/106682, WO2004/026897, WO2005/045027, WO2004/100870, WO2005/053618, WO2007/047764, WO2007/063903, WO2007/120673, WO2010/037395, WO2010/123065, WO2014/157692, WO2014/157704, WO2014/098012, WO2018/181648, and WO2019/131722.

In an embodiment, the concomitant drug is a compound represented by the formula (1) or a pharmaceutically acceptable salt thereof: 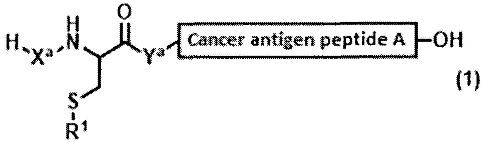 wherein X^{a} and Y^{a} each independently represent a single bond or a divalent peptide group consisting of 1 to 4 amino acid residues, provided that the sum of the numbers of amino acid residues in X^{a} and Y^{a} is an integer of 0 to 4;
cancer antigen peptide A is an MHC class I-restricted peptide consisting of 7 to 30 amino acid residues, wherein an amino group of an N-terminal amino acid of the cancer antigen peptide A binds to Y^{a} in the formula (1), and a carbonyl group of a C-terminal amino acid of the cancer antigen peptide A binds to the hydroxyl group in the formula (1); and
R¹ is a hydrogen atom,
a group represented by the formula (2): 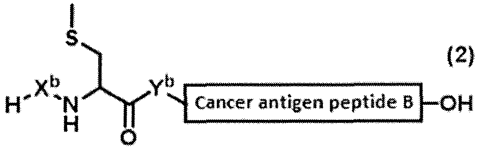
wherein X^{b} and Y^{b} each independently represent a single bond or a divalent peptide group consisting of 1 to 4 amino acid residues, provided that the sum of the numbers of amino acid residues in X^{b} and Y^{b} is an integer of 0 to 4,
cancer antigen peptide B is an MHC class I-restricted peptide consisting of 7 to 30 amino acid residues, wherein an amino group of an N-terminal amino acid of the cancer antigen peptide B binds to Y^{b} in the formula (2), and a carbonyl group of a C-terminal amino acid of the cancer antigen peptide B binds to the hydroxyl group in the formula (2), and
the sulfur atom in the formula (2) binds to the sulfur atom in the formula (1) via a disulfide bond,
or cancer antigen peptide C, wherein the cancer antigen peptide C is an MHC class I-restricted peptide consisting of 7 to 30 amino acid residues including one cysteine residue or an MHC class II-restricted peptide consisting of 7 to 30 amino acid residues including one cysteine residue, and a sulfur atom of the cysteine residue of the cancer antigen peptide C binds to the sulfur atom in the formula (1) via a disulfide bond, and optionally a peptide consisting of 1 to 4 amino acid residues binds to an N-terminus of the cancer antigen peptide C,
provided that when R¹ is the group represented by the formula (2) and the cancer antigen peptide B includes one cysteine residue, a sulfur atom of the cysteine residue of the cancer antigen peptide B optionally binds, via a disulfide bond, to
a sulfur atom in the formula (3): 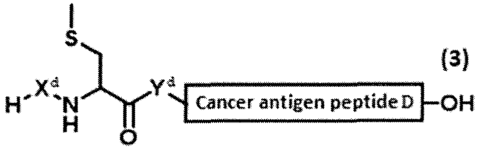
wherein X^{d} and Y^{d} each independently represent a single bond or a divalent peptide group consisting of 1 to 4 amino acid residues, provided that the sum of the numbers of amino acid residues in X^{d} and Y^{d} is an integer of 0 to 4, and cancer antigen peptide D is an MHC class II-restricted peptide consisting of 7 to 30 amino acid residues, wherein an amino group of an N-terminal amino acid of the cancer antigen peptide D binds to Y^{d} in the formula (3), and a carbonyl group of a C-terminal amino acid of the cancer antigen peptide D binds to the hydroxyl group in the formula (3),
or a sulfur atom of a cysteine residue of cancer antigen peptide E, wherein the cancer antigen peptide E is an MHC class II-restricted peptide consisting of 7 to 30 amino acid residues including one cysteine residue; and
when R¹ is the cancer antigen peptide C and a peptide consisting of 1 to 4 amino acid residues including one cysteine residue binds to the N terminus of the cancer antigen peptide C, a sulfur atom of the cysteine residue of the peptide binding to the N terminus of the cancer antigen peptide C optionally binds, via a disulfide bond, to
a sulfur atom in the formula (3): 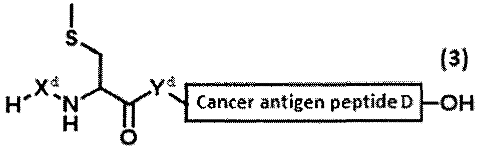
wherein X^{d} and Y^{d} each independently represent a single bond or a divalent peptide group consisting of 1 to 4 amino acid residues, provided that the sum of the numbers of amino acid residues in X^{d} and Y^{d} is an integer of 0 to 4, and cancer antigen peptide D is an MHC class II-restricted peptide consisting of 7 to 30 amino acid residues, wherein an amino group of an N-terminal amino acid of the cancer antigen peptide D binds to Y^{d} in the formula (3), and a carbonyl group of a C-terminal amino acid of the cancer antigen peptide D binds to the hydroxyl group in the formula (3),
or a sulfur atom of a cysteine residue of cancer antigen peptide E, wherein the cancer antigen peptide E is an MHC class II-restricted peptide consisting of 7 to 30 amino acid residues including one cysteine residue.

In an embodiment, the compound represented by the formula (1) or a pharmaceutically acceptable salt thereof is:
a compound of formula (4): 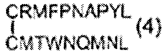 wherein C-C shown in the formula means that the C residues are linked together by a disulfide bond (SEQ ID NOs: 7 and 8, respectively), or a pharmaceutically acceptable salt thereof; or
a compound of formula (5): 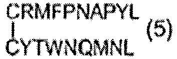 wherein C-C shown in the formula means that the C residues are linked together by a disulfide bond (SEQ ID NOs: 7 and 9, respectively), or a pharmaceutically acceptable salt thereof.

In an embodiment, the compound represented by the formula (1) or a pharmaceutically acceptable salt thereof is:
the compound of formula (5): 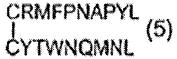 wherein C-C shown in the formula means that the C residues are linked together by a disulfide bond (SEQ ID NOs: 7 and 9, respectively), or a pharmaceutically acceptable salt thereof.

In an embodiment, the pharmaceutical composition of the present disclosure comprises:
the peptide consisting of the amino acid sequence of SEQ ID NO: 2 or a pharmaceutically acceptable salt thereof, and
the compound of formula (5): 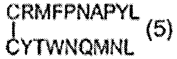 wherein C-C shown in the formula means that the C residues are linked together by a disulfide bond (SEQ ID NOs: 7 and 9, respectively), or a pharmaceutically acceptable salt thereof.

In an embodiment, the pharmaceutical composition of the present disclosure comprises:
the peptide consisting of the amino acid sequence of SEQ ID NO: 2 or an acetate salt thereof, and
the compound of formula (5): 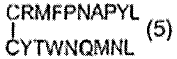 wherein C-C shown in the formula means that the C residues are linked together by a disulfide bond (SEQ ID NOs: 7 and 9, respectively), or a trifluoroacetate salt thereof.

The peptide of the present disclosure may be used for the treatment or prevention of a cancer without other cancer antigen peptides in combination. In an embodiment, the pharmaceutical composition does not comprise a cancer antigen peptide or a pharmaceutically acceptable salt thereof other than the peptide of the present disclosure or a pharmaceutically acceptable salt thereof. In an embodiment, a method for treating or preventing a cancer does not comprise administering a cancer antigen peptide a pharmaceutically acceptable salt thereof other than the peptide of the present disclosure or a pharmaceutically acceptable salt thereof.

A peptide may have modification in amino acid residue(s) in its sequence. Modification may be made by a conventional way, for example by esterification, alkylation, halogenation, phosphorylation, sulfonation, or amidation on a functional group in an amino acid residue. Amino acid modification in a peptide can also be addition of any of various moieties to N-terminus and/or C-terminus of a peptide. A peptide may be modified by addition of such a moiety that would modulate solubility of the peptide, stabilize the peptide against, for example, proteolytic degradation, direct the peptide to a specific tissue or organ, or improve capturing of the peptide by antigen presenting cells.

In a peptide, an amino group of its N-terminal amino acid or a carboxyl group of its C-terminal amino acid may be modified. The amino group may be modified, for example by addition of one to three groups selected from C₁₋₆-alkyl, phenyl, cycloalkyl, or acyl such as C₁₋₆-alkanoyl, phenyl-C₁₋₆-alkanoyl, C₅₋₇-cycloalkyl-carbonyl, C₁₋₆-alkylsulfonyl, phenylsulfonyl, C₂₋₆-alkoxy-carbonyl, phenyl-alkoxycarbonyl, C₅₋₇-cycloalkoxy-carbonyl, or phenoxycarbonyl. The carboxyl group of the C-terminal amino acid may be modified, for example by conversion to an ester, such as a C₁₋₆-alkyl ester, a phenyl-C₀₋₆-alkyl ester, or a C₅₋₇-cycloalkyl ester, or to an amide such as a mono- or di-C₁₋₆-alkylamide, a mono- or di-phenyl-C₀₋₆-alkylamide, or a di-substituted amide wherein the two substituents forms together with the nitrogen atom they attach to a 5- to 7-membered azacycloalkane.

In a peptide, a bond between amino acid residues may be peptide bond or other type of bond such as carbon-carbon bond, carbon-nitrogen bond, or carbon-sulfur bond. A peptide as described herein may comprise one or more D-amino acid residues.

The ability of a peptide to induce CTLs or helper T cells can be confirmed by a conventional method. Induction of CTLs can be confirmed, for example by counting CTLs by HLA tetramer method (Int. J. Cancer: 100, 565-570 (2002)) or limiting dilution method (Nat. Med.: 4, 321-327 (1998)). Induction of helper T cells can be confirmed, for example by a method as described in Cancer Immunol. Immunother. 51: 271 (2002).

The peptide or compound as described herein, or an intermediate peptide for the synthesis thereof may be synthesized by using a conventional technique for peptide synthesis as described, for example, in Peptide Synthesis, Interscience, New York, 1966; The Proteins, Vol 2, Academic Press Inc., New York, 1976; peptide synthesis, Maruzen Co., LTD., 1975; Basics and Experiment of Peptide Synthesis, Maruzen Co., LTD., 1985; or Development of Pharmaceutical Product subsequent vol. 14, Peptide Synthesis, Hirokawa Shoten, 1991. Examples of such a technique include solid phase synthesis by Fmoc method or Boc method, or liquid phase synthesis by sequential condensation of Boc-amino acid or Z-amino acid in a liquid phase (wherein Fmoc means 9-fluorenylmethoxycarbonyl, Boc means t-butoxycarbonyl, and Z means benzyloxycarbonyl). The peptide may be obtained by a genetic technique by using a nucleotide sequence encoding the peptide in accordance with a conventionally known method as described, for example, in Molecular Cloning, T. Maniatis et al., CSH Laboratory (1983), DNA Cloning, DM. Glover, IRL PRESS (1985).

The compound represented by the formula (1) can be prepared by the method described in WO 2014/157692. For example, the compound represented by the formula (1) can be prepared by linking two cancer antigen peptides via a disulfide bond.

The peptide, compound or intermediate synthesized may be purified by any purification method know in the art or in the field of peptide chemistry. Examples of such a purification technique include various types of chromatography (e.g., silica gel column chromatography, ion exchange column chromatography, gel filtration or reversed-phase chromatography), and recrystallization from a solvent, for example an alcohol, such as methanol, ethanol or 2-propanol; an ether, such as diethyl ether; an ester, such as ethyl acetate; an aromatic hydrocarbon, such as benzene or toluene; a ketone, such as acetone; a hydrocarbon, such as hexane; an aprotic solvent, such as dimethylformamide or acetonitrile; water; or a mixture thereof. For further useful purification methods, reference can be made, for example, to Jikken Kagaku Kouza (The Chemical Society of Japan ed., Maruzen) vol. 1. Purification methods for disulfide compounds are also described in Peptide Synthesis, Interscience, New York, 1966; The Proteins, Vol. 2, Academic Press Inc., New York, 1976; peptide synthesis, Maruzen Co., LTD., 1975; Basics and Experiment of peptide synthesis, Maruzen Co., LTD., 1985; or Development of Pharmaceutical Product sequential vol. 14, Peptide Synthesis, Hirokawa Shoten, 1991. Purification by HPLC is preferred.

The pharmaceutical composition may comprise a pharmaceutically acceptable carrier in addition to the peptide of the present disclosure or a pharmaceutically acceptable salt thereof. Also, the pharmaceutical composition may further comprise, or be administered in combination with, an appropriate adjuvant for enhancing the induction of CTLs and/or helper T cells by the composition.

The "pharmaceutically acceptable carrier" refers to a carrier that is non-toxic to a cell or a mammal exposed to the carrier at an amount or concentration it is used. A pH buffered aqueous solution is often used as a pharmaceutically acceptable carrier. Examples of the "pharmaceutically acceptable carrier" include buffering agents (such as phosphate, citrate, lactate, tartrate, trifluoroacetate and other organic acids); antioxidants (such as ascorbic acid); low molecular weight polypeptides (less than about 10 residues); proteins (such as serum albumin, gelatin or immunoglobulin); hydrophilic polymers (such as polyvinylpyrrolidone); amino acids (such as glycine, glutamine, asparagine, arginine, methionine or lysine); monosaccharides, disaccharides and other carbohydrates (such as glucose, mannose or dextrin); chelating agents (such as EDTA); sugar alcohols (such as mannitol, trehalose or sorbitol); stabilizers (such as diethylenetriaminepentaacetic acid); salt forming counterions (such as sodium); solubilizing agents (such as polysorbate 80^{®}), and/or nonionic surfactants (such as TWEEN^{®}, polyethylene glycol (PEG) and PLURONICS^{®}). A macromolecular material that is metabolized slowly, such as a protein, a polypeptide, a liposome, a polysaccharide, polylactide, polyglycolic acid, polymeric amino acids, amino acid copolymers, and inactive virus particles may also be useful as a pharmaceutically acceptable carrier. For administration, the compound or the peptide as described herein may be formulated in a liposome preparation, attached to beads having a diameter of a micrometer order, or associated with a lipid carrier.

The adjuvant may be any of adjuvants as described in Clin. Microbiol. Rev., 7: 277-289, 1994. Specifically, the adjuvant may be a microorganism-derived agent, GM-CSF, a cytokine such as interleukin-2, interleukin-7, or interleukin-12, a plant-derived agent, a marine organism-derived agent, a mineral gel such as aluminum hydroxide, lysolecithin, a surfactant such as pluronic polyol, a polyanion, a peptide, or an oil emulsion (an emulsion preparation). Examples of the microorganism-derived agent include lipid A, monophosphoryl lipid A, which is a derivative of lipid A, killed bacteria (e.g., Mycobacterium bacteria such as BCG bacteria), bacterium-derived proteins, polynucleotides, Freund's incomplete adjuvant, Freund's complete adjuvant, cell wall skeleton components (e.g., BCG-CWS), trehalose dimycolate (TDM).

The adjuvant may also be a sedimentary adjuvant or an oil adjuvant. A sedimentary adjuvant can be a suspension of an inorganic substance to which a peptide can be adsorbed. Examples of the sedimentary adjuvant include sodium hydroxide, aluminum hydroxide (Alum), calcium phosphate, aluminum phosphate, alum, Pepesu, and carboxyvinyl polymer. An oil adjuvant can be an oil emulsifier that is able to emulsify a peptide by forming micelles comprising an aqueous peptide solution phase encapsulated in a mineral oil membrane. Examples of the oil adjuvant include, but are not limited to, liquid paraffin, lanolin, Freund's adjuvant (Freund's complete adjuvant, and Freund's incomplete adjuvant), Montanide, and a W/O emulsion (see WO2006/078059).

The pharmaceutical composition of the present disclosure may be provided as a dosage form for oral administration or parenteral administration, but preferably a dosage form for parenteral administration. Examples of dosage forms for parenteral administration include an injectable preparation, an ointment, a gel, a cream, a plaster, a patch, a liniment, a spray, an inhalant, an aerosol, an eye drop, or a nasal drop, a suppository, an inhalable preparation, or a nasal preparation.

In an embodiment, the pharmaceutical composition is an injectable preparation. An injectable preparation may be in the form of a solution, a suspension, or an emulsion, or may be provided as a solid formulation to be dissolved or dispersed in a liquid before use. An injectable preparation comprises one or more active agents dissolved, dispersed or emulsified in a liquid for injection. Examples of liquids for injection include distilled water for injection, physiological saline, vegetable oils, propylene glycol, polyethylene glycol, alcohols such as ethanol, and combinations thereof. An injectable preparation may additionally comprise a stabilizer, a solubilizing aid (such as glutamic acid, aspartic acid, or polysorbate 80^{®}), a dispersant, an emulsifier, an analgesic, a buffer, a preservative, or other appropriate additive. For providing an injectable preparation as a sterilized preparation, it may be subjected to sterilization in the final step of its production, or produced aseptically throughout its production. An injectable preparation may be provided as a sterilized solid formulation, for example a lyophilized formulation, which may be sterilized or reconstituted in sterilized water for injection or other appropriate sterilized liquid before use.

The peptide of the present disclosure or a pharmaceutically acceptable salt thereof can be administered to a subject by an appropriate method depending on the disease to treat, condition of the subject, target site of the administration, or other factors. For example, parenteral administration, preferably, intravenous, intramuscular, intradermal, or subcutaneous administration by injection or infusion may be useful. Frequency of dose, or dosing interval may be appropriately selected depending on the disease to treat, condition of the subject, route of administration, or other factors. Administration is usually repeated, preferably every few days or few months.

The term effective amount as used herein means an amount of an active agent that can completely or partially inhibit progression of a cancer or at least partially reduce one or more symptoms of a cancer, or that can provide induction of remission, maintenance of remission and/or suppression of recurrence. An effective amount of an agent is determined depending on the age or sex of the subject, the type or severity of condition to treat with the agent, a desired outcome of the treatment with the agent, or other factors. An effective amount for a particular subject can be determined by a person skilled in the art according to any known method. For example, the peptide or compound or a pharmaceutically acceptable salt thereof described herein may usually be administered in an amount of 0.0001 mg to 1000 mg, 0.001 mg to 1000 mg, or 0.1 mg to 10 mg, at one time.

The peptide of the present disclosure or a pharmaceutically acceptable salt thereof may be used in a lymphocyte therapy or a DC (dendritic cell) therapy.

In an embodiment, the present disclosure relates to antigen-presenting cells (for example, dendritic cells, B-lymphocytes, or macrophages) presenting a peptide consisting of the amino acid sequence of SEQ ID NO: 2 or an altered peptide thereof having an ability to induce CTLs via HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02. CTLs can be induced in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject by using such antigen-presenting cells. The antigen-presenting cells presenting the peptide can be obtained by culturing HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive immature antigen-presenting cells in the presence of the peptide.

In a different embodiment, the present disclosure relates to a method of inducing antigen presenting cells, comprising culturing HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive immature antigen presenting cells in the presence of a peptide consisting of the amino acid sequence of SEQ ID NO: 2 or an altered peptide thereof having an ability to induce CTLs. The term "immature antigen presenting cells" herein refers to cells that can mature into antigen presenting cells (for example, dendritic cells, B-lymphocytes or macrophages). Since the immature antigen-presenting cells are contained in peripheral blood mononuclear cells (PBMCs), for example, PBMCs may be cultured in the presence of the peptide. Accordingly, the present disclosure relates to a method of inducing antigen presenting cells, comprising culturing PBMCs of an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject in the presence of a peptide consisting of the amino acid sequence of SEQ ID NO: 2 or an altered peptide thereof having an ability to induce CTLs.

In a different embodiment, the present disclosure relates to a method for treating or preventing a cancer in an HLA-A* 11:0 1, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject, comprising administering antigen-presenting cells presenting a peptide consisting of the amino acid sequence of SEQ ID NO: 2 or an altered peptide thereof having an ability to induce CTLs via HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02 to a subject positive for the corresponding HLA subtype. The antigen presenting cells may be administered by any method appropriately selected depending on the disease to treat, condition of the subject, or target site of the administration, or other factors. The antigen presenting cells may be administered intravenously, intradermally, subcutaneously, intramuscularly, or intranasally, or by other administration route.

In a different embodiment, the present disclosure relates to CTLs induced by a peptide consisting of the amino acid sequence of SEQ ID NO: 2 or an altered peptide thereof having an ability to induce CTLs. The CTLs can damage cancer cells in a HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject.

In a different embodiment, the present disclosure relates to a method of inducing CTLs, comprising culturing PBMCs of an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject in the presence of a peptide consisting of the amino acid sequence of SEQ ID NO: 2 or an altered peptide thereof having an ability to induce CTLs. When PBMCs are cultured in the presence of the peptide, peptide-specific CTLs are induced from precursor cells in the PBMCs. The peptide-specific CTLs obtained by the method can be administered to an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject to treat or prevent a cancer in the subject.

In a different embodiment, the present disclosure relates to a method for treating or preventing a cancer in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject, comprising administering CTLs specific to a peptide consisting of the amino acid sequence of SEQ ID NO: 2 or an altered peptide thereof having an ability to induce CTLs to the subject. The CTLs specific to the peptide can be administered by any method appropriately selected depending on the disease to treat, condition of the subject, or target site of the administration, or other factors. The CTLs may be administered intravenously, intradermally, subcutaneously, intramuscularly, intranasally, or orally, or by other administration route.

In a different embodiment, the present disclosure relates to a kit comprising a peptide consisting of the amino acid sequence of SEQ ID NO: 2 or an altered peptide thereof having an ability to induce CTLs or a pharmaceutically acceptable salt thereof, or a composition comprising the peptide or a pharmaceutically acceptable salt thereof, as a component. In an embodiment, the kit is used for treating or preventing a cancer in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject. In a different embodiment, the kit is used in the method of inducing antigen-presenting cells or the method of inducing CTLs herein described. The kit may comprise, other than the peptide or a pharmaceutically acceptable salt thereof or the composition, a means for taking a sample (for example, PBMCs) from a subject, an adjuvant, a container for reaction, or a package insert. Alternatively, or in addition, the kit may further comprise one or more concomitant drugs or one or more compositions comprising the same. An instruction booklet is usually attached to the kit.

In an aspect, the present disclosure provides a method for treating or preventing a cancer in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject in need thereof, comprising administering a peptide consisting of the amino acid sequence of SEQ ID NO: 2 or an altered peptide thereof having an ability to induce CTLs or a pharmaceutically acceptable salt thereof to the HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject.

In an aspect, the present disclosure provides a peptide consisting of the amino acid sequence of SEQ ID NO: 2 or an altered peptide thereof having an ability to induce CTLs or a pharmaceutically acceptable salt thereof for use in treating or preventing a cancer in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject.

In an aspect, the present disclosure provides use of a peptide consisting of the amino acid sequence of SEQ ID NO: 2 or an altered peptide thereof having an ability to induce CTLs or a pharmaceutically acceptable salt thereof for the manufacture of a medicament for treating a cancer in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject.

In a different aspect, the present disclosure provides a pharmaceutical composition for treating or preventing a benign tumor in an HLA-A* 11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject comprising a peptide consisting of the amino acid sequence of SEQ ID NO: 2 or an altered peptide thereof having an ability to induce CTLs or a pharmaceutically acceptable salt thereof. This embodiment can be carried out in accordance with the above description about the treatment or prevention of a cancer.

The "benign tumor" is a tumor that has no pathologically malignant findings and is distinguished from a malignant tumor or a cancer. It is said that a benign tumor does not show metastasis or infiltration tendency. Diagnosis as a benign tumor does not necessarily mean a good clinical prognosis. For example, a low-grade meningioma that develops in the brain stem is a benign tumor, but it is difficult to treat, and it is clinically malignant because it compresses the brain stem and shows a poor prognosis, and thus often requires treatment or prevention.

In an embodiment, the benign tumor is a benign tumor in which WT1 is expressed or a benign tumor associated with an elevated expression level of WT1 gene.

Benign tumors include, but not limited to, familial adenomatous polyposis, non-hereditary colorectal adenoma, intraductal papillary mucinous neoplasm, meningioma, schwannoma, epithelial adenoma of an organ, papilloma, non-epithelial myoma, lipoma, chondroma, and hemangioma.

In an embodiment, the benign tumor is familial adenomatous polyposis. Familial adenomatous polyposis is a hereditary disease characterized by mutation of a tumor suppressor gene APC (adenoma polyposis coli) and a large number of adenomas formed in the intestinal tract. The terms "familial adenomatous polyposis", "familial polyposis coli", "familial adenomatous polyposis coli" and "FAP" can be used interchangeably. As used herein, familial adenomatous polyposis also includes diseases that coincide with tumors in tissues other than the intestinal tract, such as Gardner's syndrome.

In a further aspect, the present disclosure provides a method for treating or preventing a benign tumor in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject in need thereof, comprising administering a peptide consisting of the amino acid sequence of SEQ ID NO: 2 or an altered peptide thereof having an ability to induce CTLs or a pharmaceutically acceptable salt thereof to the HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject; a peptide consisting of the amino acid sequence of SEQ ID NO: 2 or an altered peptide thereof having an ability to induce CTLs or a pharmaceutically acceptable salt thereof for use in treating or preventing a benign tumor in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject; and use of a peptide consisting of the amino acid sequence of SEQ ID NO: 2 or an altered peptide thereof having an ability to induce CTLs or a pharmaceutically acceptable salt thereof for the manufacture of a medicament for treating a benign tumor in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject.

In a further aspect, the present disclosure provides a method for treating or preventing a benign tumor in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject, comprising administering antigen-presenting cells presenting a peptide consisting of the amino acid sequence of SEQ ID NO: 2 or an altered peptide thereof having an ability to induce CTLs via HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02 to a subject positive for the corresponding HLA subtype; a method for treating or preventing a benign tumor in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject, comprising administering CTLs specific to a peptide consisting of the amino acid sequence of SEQ ID NO: 2 or an altered peptide thereof having an ability to induce CTLs to the subject; and a kit for treating or preventing a benign tumor in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject comprising a peptide consisting of the amino acid sequence of SEQ ID NO: 2 or an altered peptide thereof having an ability to induce CTLs or a pharmaceutically acceptable salt thereof, or a composition comprising the peptide or a pharmaceutically acceptable salt thereof, as a component.

The peptide of SEQ ID NO: 2 has been known to be effective for an intraocular angiogenic disease (WO2016/093326). Accordingly, in a different aspect, the present disclosure provides a pharmaceutical composition for treating or preventing an intraocular angiogenic disease in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject comprising a peptide consisting of the amino acid sequence of SEQ ID NO: 2 or an altered peptide thereof having an ability to induce CTLs or a pharmaceutically acceptable salt thereof. This embodiment can be carried out in accordance with the above description about the treatment or prevention of a cancer.

In an embodiment, the intraocular angiogenic disease is wet-type age-related macular degeneration, myopic macular degeneration, angioid streaks, central serous chorioretinopathy, retinal pigment epitheliopathy, choroidal atrophy, choroideremia, choroidal osteoma, diabetic retinopathy, retinopathy of prematurity, rubeotic glaucoma, or corneal neovascularization.

In a further aspect, the present disclosure provides a method for treating or preventing an intraocular angiogenic disease in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject in need thereof, comprising administering a peptide consisting of the amino acid sequence of SEQ ID NO: 2 or an altered peptide thereof having an ability to induce CTLs or a pharmaceutically acceptable salt thereof to the HLA-A* 11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject; a peptide consisting of the amino acid sequence of SEQ ID NO: 2 or an altered peptide thereof having an ability to induce CTLs or a pharmaceutically acceptable salt thereof for use in treating or preventing an intraocular angiogenic disease in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject; and use of a peptide consisting of the amino acid sequence of SEQ ID NO: 2 or an altered peptide thereof having an ability to induce CTLs or a pharmaceutically acceptable salt thereof for the manufacture of a medicament for treating an intraocular angiogenic disease in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject.

In a further aspect, the present disclosure provides a method for treating or preventing an intraocular angiogenic disease in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject, comprising administering antigen-presenting cells presenting a peptide consisting of the amino acid sequence of SEQ ID NO: 2 or an altered peptide thereof having an ability to induce CTLs via HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02 to a subject positive for the corresponding HLA subtype; a method for treating or preventing an intraocular angiogenic disease in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject, comprising administering CTLs specific to a peptide consisting of the amino acid sequence of SEQ ID NO: 2 or an altered peptide thereof having an ability to induce CTLs to the subject; and a kit for treating or preventing an intraocular angiogenic disease in an HLA-A* 11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject comprising a peptide consisting of the amino acid sequence of SEQ ID NO: 2 or an altered peptide thereof having an ability to induce CTLs or a pharmaceutically acceptable salt thereof, or a composition comprising the peptide or a pharmaceutically acceptable salt thereof, as a component.

In these aspects of the present disclosure described above, the peptide of the present disclosure or a pharmaceutically acceptable salt thereof may be used in combination with a concomitant drug as described above for the treatment or prevention of a cancer. For example, the peptide of the present disclosure or a pharmaceutically acceptable salt thereof may be used in combination with the compound of formula (4) or formula (5) or a pharmaceutically acceptable salt thereof.

In these aspects of the present disclosure described above, HLA is HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02. In an embodiment, HLA is HLA-A*11:01, HLA-A*02:01, HLA-A*02:06, HLA-A*02:07, HLA-A*26:01, or HLA-A*26:03. In an embodiment, HLA is HLA-A*02:01, HLA-A*02:06, HLA-A*02:07, or HLA-A*26:01. In an embodiment, HLA is HLA-A*02:01, HLA-A*02:06, or HLA-A*02:07. In an embodiment, HLA is HLA-A*11:01, HLA-A*26:01, or HLA-A*26:03. In an embodiment, HLA is HLA-A*11:01 or HLA-A*26:03. In an embodiment, HLA is HLA-A* 11:01. In an embodiment, HLA is HLA-A*02:01. In an embodiment, HLA is HLA-A*02:06. In an embodiment, HLA is HLA-A*02:07. In an embodiment, HLA is HLA-A*26:01. In an embodiment, HLA is HLA-A*26:03. In an embodiment, HLA is HLA-A*01:01, HLA-B*15:01, HLA-B*35:01, HLA-C*03:03, HLA-C*05:01, HLA-C*07:01, or HLA-C*07:02. In an embodiment, the subject is an HLA-A*01:01, HLA-B*35:01, HLA-C*03:03, HLA-C*05:01, or HLA-C*07:01-positive subject. In an embodiment, the subject is an HLA-A*01:01, HLA-B*35:01, HLA-C*03:03, or HLA-C*05:01-positive subject. In an embodiment, HLA is HLA-A*01:01. In an embodiment, HLA is HLA-B*15:01. In an embodiment, HLA is HLA-B*35:01. In an embodiment, HLA is HLA-C*03:03. In an embodiment, HLA is HLA-C*05:01. In an embodiment, HLA is HLA-C*07:01. In an embodiment, HLA is HLA-C*07:02. In an embodiment, the subject is an HLA-A*02:01, HLA-A*02:06, HLA-A*02:07, HLA-A*26:01, HLA-A*01:01, HLA-B*35:01, HLA-C*03:03, HLA-C*05:01, or HLA-C*07:01-positive subject. In an embodiment, the subject is an HLA-A*02:07, HLA-A*26:01, HLA-A*01:01, HLA-B*35:01, HLA-C*03:03, HLA-C*05:01, or HLA-C*07:01-positive subject. In an embodiment, the subject is an HLA-A*02:07, HLA-A*26:01, HLA-A*01:01, HLA-B*35:01, HLA-C*03:03, orHLA-C*05:01-positive subject.

Exemplary embodiments of the present disclosure are described below.
1. A pharmaceutical composition for treating or preventing a cancer in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject comprising a peptide consisting of the amino acid sequence of WAPVLDFAPPGASAYGSL (SEQ ID NO: 2) or an altered peptide thereof having an ability to induce CTLs or a pharmaceutically acceptable salt thereof.
2. The pharmaceutical composition according to item 1, wherein the subject is an HLA-A*11:01, HLA-A*02:01, HLA-A*02:06, HLA-A*02:07, HLA-A*26:01, or HLA-A*26:03-positive subject.
3. The pharmaceutical composition according to item 1, wherein the subject is an HLA-A*02:01, HLA-A*02:06, HLA-A*02:07, or HLA-A*26:01-positive subject.
4. The pharmaceutical composition according to item 1, wherein the subject is an HLA-A*02:01, HLA-A*02:06, or HLA-A*02:07-positive subject.
5. The pharmaceutical composition according to item 1, wherein the subject is an HLA-A*11:01-positive subject.
6. The pharmaceutical composition according to item 1, wherein the subject is an HLA-A*02:01-positive subject.
7. The pharmaceutical composition according to item 1, wherein the subject is an HLA-A*02:06-positive subject.
8. The pharmaceutical composition according to item 1, wherein the subject is an HLA-A*02:07-positive subject.
9. The pharmaceutical composition according to item 1, wherein the subject is an HLA-A*26:01-positive subject.
10. The pharmaceutical composition according to item 1, wherein the subject is an HLA-A*26:03-positive subject.
11. The pharmaceutical composition according to item 1, wherein the subject is an HLA-A*01:01, HLA-B*15:01, HLA-B*35:01, HLA-C*03:03, HLA-C*05:01, HLA-C*07:01, or HLA-C*07:02-positive subject.
12. The pharmaceutical composition according to item 1, wherein the subject is an HLA-A*01:01, HLA-B*35:01, HLA-C*03:03, HLA-C*05:01, or HLA-C*07:01-positive subject.
13. The pharmaceutical composition according to item 1, wherein the subject is an HLA-A*01:01-positive subject.
14. The pharmaceutical composition according to item 1, wherein the subject is an HLA-B*15:01-positive subject.
15. The pharmaceutical composition according to item 1, wherein the subject is an HLA-B*35:01-positive subject.
16. The pharmaceutical composition according to item 1, wherein the subject is an HLA-C*03:03-positive subject.
17. The pharmaceutical composition according to item 1, wherein the subject is an HLA-C*05:01-positive subject.
18. The pharmaceutical composition according to item 1, wherein the subject is an HLA-C*07:01-positive subject.
19. The pharmaceutical composition according to item 1, wherein the subject is an HLA-C*07:02-positive subject.
20. The pharmaceutical composition according to item 1, wherein the subject is an HLA-A*02:01, HLA-A*02:06, HLA-A*02:07, HLA-A*26:01, HLA-A*01:01, HLA-B*35:01, HLA-C*03:03, HLA-C*05:01, or HLA-C*07:01-positive subject.
21. The pharmaceutical composition according to any one of items 1 to 20, wherein the pharmaceutical composition comprises the peptide consisting of the amino acid sequence of SEQ ID NO: 2 or a pharmaceutically acceptable salt thereof.
22. The pharmaceutical composition according to any one of items 1 to 21, wherein the pharmaceutical composition further comprises
   a compound of formula (4): 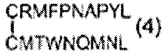 wherein C-C shown in the formula means that the C residues are linked together by a disulfide bond (SEQ ID NOs: 7 and 8, respectively), or a pharmaceutically acceptable salt thereof; or
   a compound of formula (5): 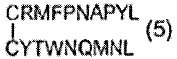 wherein C-C shown in the formula means that the C residues are linked together by a disulfide bond (SEQ ID NOs: 7 and 9, respectively), or a pharmaceutically acceptable salt thereof.
23. The pharmaceutical composition according to item 22, wherein the pharmaceutical composition comprises the compound of formula (5): 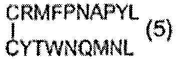 wherein C-C shown in the formula means that the C residues are linked together by a disulfide bond (SEQ ID NOs: 7 and 9, respectively), or a pharmaceutically acceptable salt thereof.
24. The pharmaceutical composition according to any one of items 1 to 21, wherein the pharmaceutical composition is used in combination with
   a compound of formula (4): 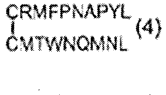 wherein C-C shown in the formula means that the C residues are linked together by a disulfide bond (SEQ ID NOs: 7 and 8, respectively), or a pharmaceutically acceptable salt thereof; or
   a compound of formula (5): 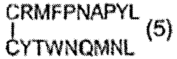 wherein C-C shown in the formula means that the C residues are linked together by a disulfide bond (SEQ ID NOs: 7 and 9, respectively), or a pharmaceutically acceptable salt thereof.
25. The pharmaceutical composition according to item 24, wherein the pharmaceutical composition is used in combination with the compound of formula (5): 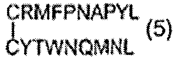 wherein C-C shown in the formula means that the C residues are linked together by a disulfide bond (SEQ ID NOs: 7 and 9, respectively), or a pharmaceutically acceptable salt thereof.
26. The pharmaceutical composition according to any one of items 1 to 25, wherein the pharmaceutical composition comprises
   the peptide consisting of the amino acid sequence of SEQ ID NO: 2 or a pharmaceutically acceptable salt thereof; and
   a compound of formula (5): 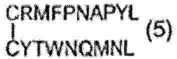 wherein C-C shown in the formula means that the C residues are linked together by a disulfide bond (SEQ ID NOs: 7 and 9, respectively), or a pharmaceutically acceptable salt thereof.
27. The pharmaceutical composition according to any one of items 1 to 26, wherein the cancer is a cancer in which WT1 is expressed or a cancer associated with an elevated expression level of WT1 gene.
28. The pharmaceutical composition according to any one of items 1 to 27, wherein the cancer is acute myeloid leukemia, chronic myeloid leukemia, acute lymphoblastic leukemia chronic lymphocytic leukemia, myelodysplastic syndrome, multiple myeloma, malignant lymphoma, stomach cancer, colon cancer, lung cancer, breast cancer, germ cell carcinoma, liver cancer, skin cancer, bladder cancer, prostate cancer, uterine cancer, cervical cancer, ovarian cancer, brain tumor or glioma.
29. A kit for treating or preventing a cancer in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject, comprising a peptide consisting of the amino acid sequence of WAPVLDFAPPGASAYGSL (SEQ ID NO: 2) or an altered peptide thereof having an ability to induce CTLs, or a pharmaceutically acceptable salt thereof.
30. The kit according to item 29, wherein the subject is an HLA-A*11:01, HLA-A*02:01, HLA-A*02:06, HLA-A*02:07, HLA-A*26:01, or HLA-A*26:03-positive subject.
31. The kit according to item 29, wherein the subject is an HLA-A*02:01, HLA-A*02:06, HLA-A*02:07, or HLA-A*26:01-positive subject.
32. The kit according to item 29, wherein the subject is an HLA-A*02:01, HLA-A*02:06, or HLA-A*02:07-positive subject.
33. The kit according to item 29, wherein the subject is an HLA-A*11:01-positive subject.
34. The kit according to item 29, wherein the subject is an HLA-A*02:01-positive subject.
35. The kit according to item 29, wherein the subject is an HLA-A*02:06-positive subject.
36. The kit according to item 29, wherein the subject is an HLA-A`02:07-positive subject.
37. The kit according to item 29, wherein the subject is an HLA-A*26:01-positive subject.
38. The kit according to item 29, wherein the subject is an HLA-A*26:03-positive subject.
39. The kit according to item 29, wherein the subject is an HLA-A*01:01, HLA-B*15:01, HLA-B*35:01, HLA-C*03:03, HLA-C*05:01, HLA-C*07:01, or HLA-C*07:02-positive subject.
40. The kit according to item 29, wherein the subject is an HLA-A*01:01, HLA-B*35:01, HLA-C*03:03, HLA-C*05:01, or HLA-C*07:01-positive subject.
41. The kit according to item 29, wherein the subject is an HLA-A*01:01-positive subject.
42. The kit according to item 29, wherein the subject is an HLA-B*15:01-positive subject.
43. The kit according to item 29, wherein the subject is an HLA-B*35:01-positive subject.
44. The kit according to item 29, wherein the subject is an HLA-C*03:03-positive subject.
45. The kit according to item 29, wherein the subject is an HLA-C*05:01-positive subject.
46. The kit according to item 29, wherein the subject is an HLA-C*07:01-positive subject.
47. The kit according to item 29, wherein the subject is an HLA-C*07:02-positive subject.
48. The kit according to item 29, wherein the subject is an HLA-A*02:01, HLA-A*02:06, HLA-A*02:07, HLA-A*26:01, HLA-A*01:01, HLA-B*35:01, HLA-C*03:03, HLA-C*05:01, or HLA-C*07:01-positive subject.
49. The kit according to any one of items 29 to 48, wherein the kit comprises the peptide consisting of the amino acid sequence of SEQ ID NO: 2 or a pharmaceutically acceptable salt thereof.
50. The kit according to any one of items 29 to 49, wherein the kit further comprises
   a compound of formula (4): 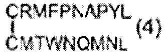 wherein C-C shown in the formula means that the C residues are linked together by a disulfide bond (SEQ ID NOs: 7 and 8, respectively), or a pharmaceutically acceptable salt thereof; or
   a compound of formula (5): 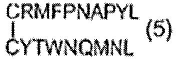 wherein C-C shown in the formula means that the C residues are linked together by a disulfide bond (SEQ ID NOs: 7 and 9, respectively), or a pharmaceutically acceptable salt thereof.
51. The kit according to item 50, wherein the kit comprises the compound of formula (5): 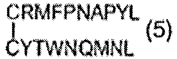 wherein C-C shown in the formula means that the C residues are linked together by a disulfide bond (SEQ ID NOs: 7 and 9, respectively), or a pharmaceutically acceptable salt thereof.
52. The kit according to any one of items 29 to 51, wherein the kit comprises
   the peptide consisting of the amino acid sequence of SEQ ID NO: 2 or a pharmaceutically acceptable salt thereof; and
   the compound of formula (5): 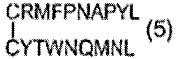 wherein C-C shown in the formula means that the C residues are linked together by a disulfide bond (SEQ ID NOs: 7 and 9, respectively), or a pharmaceutically acceptable salt thereof.
53. The kit according to any one of items 29 to 52, wherein the cancer is a cancer in which WT1 is expressed or a cancer associated with an elevated expression level of WT1 gene.
54. The kit according to any one of items 29 to 53, wherein the cancer is acute myeloid leukemia, chronic myeloid leukemia, acute lymphoblastic leukemia chronic lymphocytic leukemia, myelodysplastic syndrome, multiple myeloma, malignant lymphoma, stomach cancer, colon cancer, lung cancer, breast cancer, germ cell carcinoma, liver cancer, skin cancer, bladder cancer, prostate cancer, uterine cancer, cervical cancer, ovarian cancer, brain tumor or glioma.
55. A method for treating or preventing a cancer, comprising administering a peptide consisting of the amino acid sequence of WAPVLDFAPPGASAYGSL (SEQ ID NO: 2) or an altered peptide thereof having an ability to induce CTLs or a pharmaceutically acceptable salt thereof to an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C`03:03, HLA-C*05:01, HLA-C*07:01, or HLA-C*07:02-positive subject in need thereof.
56. The method according to item 55, wherein the subject is an HLA-A*11:01, HLA-A*02:01, HLA-A*02:06, HLA-A*02:07, HLA-A*26:01, or HLA-A*26:03-positive subject.
57. The method according to item 55, wherein the subject is an HLA-A*02:01, HLA-A*02:06, HLA-A*02:07, or HLA-A*26:01-positive subject.
58. The method according to item 55, wherein the subject is an HLA-A*02:01, HLA-A*02:06, or HLA-A*02:07-positive subject.
59. The method according to item 55, wherein the subject is an HLA-A*11:01-positive subject.
60. The method according to item 55, wherein the subject is an HLA-A*02:01-positive subject.
61. The method according to item 55, wherein the subject is an HLA-A*02:06-positive subject.
62. The method according to item 55, wherein the subject is an HLA-A*02:07-positive subject.
63. The method according to item 55, wherein the subject is an HLA-A*26:01-positive subject.
64. The method according to item 55, wherein the subject is an HLA-A*26:03-positive subject.
65. The method according to item 55, wherein the subject is an HLA-A*01:01, HLA-B*15:01, HLA-B*35:01, HLA-C*03:03, HLA-C*05:01, HLA-C*07:01, or HLA-C*07:02-positive subject.
66. The method according to item 55, wherein the subject is an HLA-A*01:01, HLA-B*35:01, HLA-C*03:03, HLA-C*05:01, or HLA-C*07:01-positive subject.
67. The method according to item 55, wherein the subject is an HLA-A*01:01-positive subject.
68. The method according to item 55, wherein the subject is an HLA-B*15:01-positive subject.
69. The method according to item 55, wherein the subject is an HLA-B*35:01-positive subject.
70. The method according to item 55, wherein the subject is an HLA-C*03:03-positive subject.
71. The method according to item 55, wherein the subject is an HLA-C*05:01-positive subject.
72. The method according to item 55, wherein the subject is an HLA-C*07:01-positive subject.
73. The method according to item 55, wherein the subject is an HLA-C*07:02-positive subject.
74. The method according to item 55, wherein the subject is an HLA-A*02:01, HLA-A*02:06, HLA-A*02:07, HLA-A*26:01, HLA-A*01:01, HLA-B*35:01, HLA-C*03:03, HLA-C*05:01, or HLA-C*07:01-positive subject.
75. The method according to any one of items 55 to 74, wherein the method comprises administering the peptide consisting of the amino acid sequence of SEQ ID NO: 2 or a pharmaceutically acceptable salt thereof.
76. The method according to any one of items 55 to 75, wherein the method further comprises administering
   a compound of formula (4): 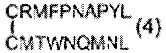 wherein C-C shown in the formula means that the C residues are linked together by a disulfide bond (SEQ ID NOs: 7 and 8, respectively), or a pharmaceutically acceptable salt thereof; or
   a compound of formula (5): 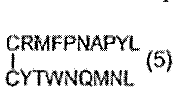 wherein C-C shown in the formula means that the C residues are linked together by a disulfide bond (SEQ ID NOs: 7 and 9, respectively), or a pharmaceutically acceptable salt thereof.
77. The method according to item 76, wherein the method comprises administering the compound of formula (5): 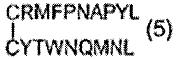 wherein C-C shown in the formula means that the C residues are linked together by a disulfide bond (SEQ ID NOs: 7 and 9, respectively), or a pharmaceutically acceptable salt thereof.
78. The method according to any one of items 55 to 77, wherein the method comprises administering
   the peptide consisting of the amino acid sequence of SEQ ID NO: 2 or a pharmaceutically acceptable salt thereof; and
   a compound of formula (5): 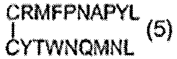 wherein C-C shown in the formula means that the C residues are linked together by a disulfide bond (SEQ ID NOs: 7 and 9, respectively), or a pharmaceutically acceptable salt thereof.
79. The method according to any one of items 55 to 78, wherein the cancer is a cancer in which WT1 is expressed or a cancer associated with an elevated expression level of WT1 gene.
80. The method according to any one of items 55 to 79, wherein the cancer is acute myeloid leukemia, chronic myeloid leukemia, acute lymphoblastic leukemia chronic lymphocytic leukemia, myelodysplastic syndrome, multiple myeloma, malignant lymphoma, stomach cancer, colon cancer, lung cancer, breast cancer, germ cell carcinoma, liver cancer, skin cancer, bladder cancer, prostate cancer, uterine cancer, cervical cancer, ovarian cancer, brain tumor or glioma.
81. A pharmaceutical composition for treating or preventing a benign tumor in an HLA-A*11:01, HLA-A*02:01, HLA-A*02:06, HLA-A*02:07, HLA-A*26:01, HLA-A*26:03, HLA-A*01:01, HLA-B* 15:01, HLA-B*35:01, HLA-C*03:03, HLA-C*05:01, HLA-C*07:01, or HLA-C*07:02-positive subject comprising a peptide consisting of the amino acid sequence of WAPVLDFAPPGASAYGSL (SEQ ID NO: 2) or an altered peptide thereof having an ability to induce CTLs or a pharmaceutically acceptable salt thereof.
82 The pharmaceutical composition according to item 81, wherein the subject is an HLA-A*11:01, HLA-A*02:01, HLA-A*02:06, HLA-A*02:07, HLA-A*26:01, or HLA-A*26:03-positive subject.
83. The pharmaceutical composition according to item 81, wherein the subject is an HLA-A*02:01, HLA-A*02:06, HLA-A*02:07, or HLA-A*26:01-positive subject.
84. The pharmaceutical composition according to item 81, wherein the subject is an HLA-A*02:01, HLA-A*02:06, or HLA-A*02:07-positive subject.
85. The pharmaceutical composition according to item 81, wherein the subject is an HLA-A*11:01-positive subject.
86. The pharmaceutical composition according to item 81, wherein the subject is an HLA-A*02:01-positive subject.
87. The pharmaceutical composition according to item 81, wherein the subject is an HLA-A*02:06-positive subject.
88. The pharmaceutical composition according to item 81, wherein the subject is an HLA-A*02:07-positive subject.
89 The pharmaceutical composition according to item 81, wherein the subject is an HLA-A*26:01-positive subject.
90. The pharmaceutical composition according to item 81, wherein the subject is an HLA-A*26:03-positive subject.
91. The pharmaceutical composition according to item 81, wherein the subject is an HLA-A*01:01, HLA-B*15:01, HLA-B*35:01, HLA-C*03:03, HLA-C*05:01, HLA-C*07:01, or HLA-C*07:02-positive subject.
92. The pharmaceutical composition according to item 81, wherein the subject is an HLA-A*01:01, HLA-B*35:01, HLA-C*03:03, HLA-C*05:01, or HLA-C*07:01-positive subject.
93. The pharmaceutical composition according to item 81, wherein the subject is an HLA-A*01:01-positive subject.
94. The pharmaceutical composition according to item 81, wherein the subject is an HLA-B*15:01-positive subject.
95. The pharmaceutical composition according to item 81, wherein the subject is an HLA-B*35:01-positive subject.
96. The pharmaceutical composition according to item 81, wherein the subject is an HLA-C*03:03-positive subject.
97. The pharmaceutical composition according to item 81, wherein the subject is an HLA-C*05:01-positive subject.
98. The pharmaceutical composition according to item 81, wherein the subject is an HLA-C*07:01-positive subject.
99. The pharmaceutical composition according to item 81, wherein the subject is an HLA-C*07:02-positive subject.
100. The pharmaceutical composition according to item 81, wherein the subject is an HLA-A*02:01, HLA-A*02:06, HLA-A*02:07, HLA-A*26:01, HLA-A*01:01, HLA-B*35:01, HLA-C*03:03, HLA-C*05:01, or HLA-C*07:01-positive subject.
101. The pharmaceutical composition according to any one of items 81 to 100, wherein the pharmaceutical composition comprises the peptide consisting of the amino acid sequence of SEQ ID NO: 2 or a pharmaceutically acceptable salt thereof.
102. The pharmaceutical composition according to any one of items 81 to 101, wherein the pharmaceutical composition further comprises
   a compound of formula (4): 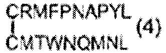 wherein C-C shown in the formula means that the C residues are linked together by a disulfide bond (SEQ ID NOs: 7 and 8, respectively), or a pharmaceutically acceptable salt thereof; or
   a compound of formula (5): 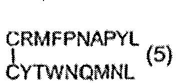 wherein C-C shown in the formula means that the C residues are linked together by a disulfide bond (SEQ ID NOs: 7 and 9, respectively), or a pharmaceutically acceptable salt thereof.
103. The pharmaceutical composition according to item 102, wherein the pharmaceutical composition comprises the compound of formula (5): 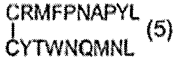 wherein C-C shown in the formula means that the C residues are linked together by a disulfide bond (SEQ ID NOs: 7 and 9, respectively), or a pharmaceutically acceptable salt thereof.
104. The pharmaceutical composition according to any one of items 81 to 101, wherein the pharmaceutical composition is used in combination with
   a compound of formula (4): 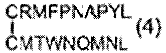 wherein C-C shown in the formula means that the C residues are linked together by a disulfide bond (SEQ ID NOs: 7 and 8, respectively), or a pharmaceutically acceptable salt thereof; or
   a compound of formula (5): 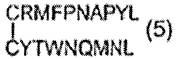 wherein C-C shown in the formula means that the C residues are linked together by a disulfide bond (SEQ ID NOs: 7 and 9, respectively), or a pharmaceutically acceptable salt thereof.
105. The pharmaceutical composition according to item 104, wherein the pharmaceutical composition is used in combination with the compound of formula (5): 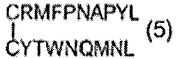 wherein C-C shown in the formula means that the C residues are linked together by a disulfide bond (SEQ ID NOs: 7 and 9, respectively), or a pharmaceutically acceptable salt thereof.
106. The pharmaceutical composition according to any one of items 81 to 105, wherein the pharmaceutical composition comprises
   the peptide consisting of the amino acid sequence of SEQ ID NO: 2 or a pharmaceutically acceptable salt thereof; and
   a compound of formula (5): 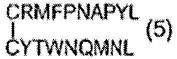 wherein C-C shown in the formula means that the C residues are linked together by a disulfide bond (SEQ ID NOs: 7 and 9, respectively), or a pharmaceutically acceptable salt thereof.
107. The pharmaceutical composition according to any one of items 81 to 106, wherein the benign tumor is a benign tumor in which WT1 is expressed or a benign tumor associated with an elevated expression level of WT1 gene.
108. The pharmaceutical composition according to any one of items 81 to 107, wherein the benign tumor is familial adenomatous polyposis, non-hereditary colorectal adenoma, intraductal papillary mucinous neoplasm, meningioma, schwannoma, epithelial adenoma of an organ, papilloma, non-epithelial myoma, lipoma, chondroma, or hemangioma.
109. The pharmaceutical composition according to items 108, wherein the benign tumor is familial adenomatous polyposis.
110. A pharmaceutical composition for treating or preventing an intraocular angiogenic disease in an HLA-A*11:01, HLA-A*02:01, HLA-A*02:06, HLA-A*02:07, HLA-A*26:01, HLA-A*26:03, HLA-A*01:01, HLA-B*15:01, HLA-B*35:01, HLA-C*03:03, HLA-C*05:01, HLA-C*07:01, or HLA-C*07:02-positive subject comprising a peptide consisting of the amino acid sequence of WAPVLDFAPPGASAYGSL (SEQ ID NO: 2) or an altered peptide thereof having an ability to induce CTLs or a pharmaceutically acceptable salt thereof.
111. The pharmaceutical composition according to item 110, wherein the subject is an HLA-A*11:01, HLA-A*02:01, HLA-A*02:06, HLA-A*02:07, HLA-A*26:01, or HLA-A*26:03-positive subject.
112. The pharmaceutical composition according to item 110, wherein the subject is an HLA-A*02:01, HLA-A*02:06, HLA-A*02:07, or HLA-A*26:01-positive subject.
113. The pharmaceutical composition according to item 110, wherein the subject is an HLA-A*02:01, HLA-A*02:06, or HLA-A*02:07-positive subject.
114. The pharmaceutical composition according to item 110, wherein the subject is an HLA-A*11:01-positive subject.
115. The pharmaceutical composition according to item 110, wherein the subject is an HLA-A*02:01-positive subject.
116. The pharmaceutical composition according to item 110, wherein the subject is an HLA-A*02:06-positive subject.
117. The pharmaceutical composition according to item 110, wherein the subject is an HLA-A*02:07-positive subject.
118 The pharmaceutical composition according to item 110, wherein the subject is an HLA-A*26:01-positive subject.
119. The pharmaceutical composition according to item 110, wherein the subject is an HLA-A*26:03-positive subject.
120. The pharmaceutical composition according to item 110, wherein the subject is an HLA-A*01 :01, HLA-B*15:01, HLA-B*35:01, HLA-C*03:03, HLA-C*05:01, HLA-C*07:01, or HLA-C*07:02-positive subject.
121. The pharmaceutical composition according to item 110, wherein the subject is an HLA-A*01:01, HLA-B*35:01, HLA-C*03:03, HLA-C*05:01, or HLA-C*07:01-positive subject.
122. The pharmaceutical composition according to item 110, wherein the subject is an HLA-A*01:01-positive subject.
123. The pharmaceutical composition according to item 110, wherein the subject is an HLA-B* 15:01-positive subject.
124. The pharmaceutical composition according to item 110, wherein the subject is an HLA-B*35:01-positive subject.
125 The pharmaceutical composition according to item 110, wherein the subject is an HLA-C*03:03-positive subject.
126. The pharmaceutical composition according to item 110, wherein the subject is an HLA-C*05:01 -positive subject.
127. The pharmaceutical composition according to item 110, wherein the subject is an HLA-C*07:01 -positive subject.
128. The pharmaceutical composition according to item 110, wherein the subject is an HLA-C*07:02-positive subject.
129. The pharmaceutical composition according to item 110, wherein the subject is an HLA-A*02:01, HLA-A*02:06, HLA-A*02:07, HLA-A*26:01, HLA-A*01:01, HLA-B*35:01, HLA-C`03:03, HLA-C*05:01, or HLA-C*07:01-positive subject.
130. The pharmaceutical composition according to any one of items 110 to 129, wherein the pharmaceutical composition comprises the peptide consisting of the amino acid sequence of SEQ ID NO: 2 or a pharmaceutically acceptable salt thereof.
131. The pharmaceutical composition according to any one of items 110 to 130, wherein the pharmaceutical composition further comprises
   a compound of formula (4): 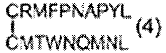 wherein C-C shown in the formula means that the C residues are linked together by a disulfide bond (SEQ ID NOs: 7 and 8, respectively), or a pharmaceutically acceptable salt thereof; or
   a compound of formula (5): 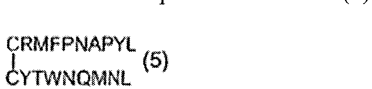 wherein C-C shown in the formula means that the C residues are linked together by a disulfide bond (SEQ ID NOs: 7 and 9, respectively), or a pharmaceutically acceptable salt thereof.
132. The pharmaceutical composition according to item 131, wherein the pharmaceutical composition comprises the compound of formula (5): 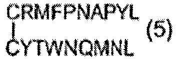 wherein C-C shown in the formula means that the C residues are linked together by a disulfide bond (SEQ ID NOs: 7 and 9, respectively), or a pharmaceutically acceptable salt thereof.
133. The pharmaceutical composition according to any one of items 110 to 130, wherein the pharmaceutical composition is used in combination with
   a compound of formula (4): 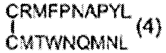 wherein C-C shown in the formula means that the C residues are linked together by a disulfide bond (SEQ ID NOs: 7 and 8, respectively), or a pharmaceutically acceptable salt thereof; or
   a compound of formula (5): 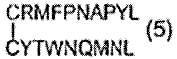 wherein C-C shown in the formula means that the C residues are linked together by a disulfide bond (SEQ ID NOs: 7 and 9, respectively), or a pharmaceutically acceptable salt thereof.
134. The pharmaceutical composition according to item 133, wherein the pharmaceutical composition is used in combination with the compound of formula (5): 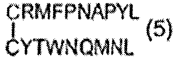 wherein C-C shown in the formula means that the C residues are linked together by a disulfide bond (SEQ ID NOs: 7 and 9, respectively), or a pharmaceutically acceptable salt thereof.
135. The pharmaceutical composition according to any one of items 110 to 134, wherein the pharmaceutical composition comprises
   the peptide consisting of the amino acid sequence of SEQ ID NO: 2 or a pharmaceutically acceptable salt thereof; and
   the compound of formula (5): 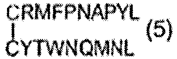 wherein C-C shown in the formula means that the C residues are linked together by a disulfide bond (SEQ ID NOs: 7 and 9, respectively), or a pharmaceutically acceptable salt thereof.
136. The pharmaceutical composition according to any one of items 110 to 135, wherein the intraocular angiogenic disease is wet-type age-related macular degeneration, myopic macular degeneration, angioid streaks, central serous chorioretinopathy, retinal pigment epitheliopathy, choroidal atrophy, choroideremia, choroidal osteoma, diabetic retinopathy, retinopathy of prematurity, rubeotic glaucoma, or corneal neovascularization.

The present invention will be specifically described by way of the following Examples, which should not be construed as limiting the present invention in any sense.

### Examples

### I. Induction of WTl -specific CD8-positive T cells with WT1₃₄₋₅₁

### 1. Methods

### Peptides

WT1₃₄₋₅₁ (WAPVLDFAPPGASAYGSL) (SEQ ID NO: 2), WT1₃₇₋₄₅ (VLDFAPPGA) (SEQ ID NO: 5) and WT1₄₀₋₄₈ (FAPPGASAY) (SEQ ID NO: 6) were used. NetMHC-4.0 (http://www.cbs.dtu.dk/services/NetMHC-4.0) analysis showed that WT1₃₇₋₄₅ had a high affinity to HLA-A*02:01, HLA-A*02:06, and HLA-A*02:07, and WT1₄₀₋₄₈ had a high affinity to HLA -A*26:01 and HLA-A*26:03.

### Preparation of immature dendritic cells (imDC)

Cryopreserved peripheral blood mononuclear cells (PBMCs) of 16 cancer patients, HLA-A*02:01-positive cancer patients (5 cases), HLA-A*02:06-positive cancer patients (7 cases), HLA-A*02:07-positive cancer patients (2 cases) and HLA-A*26:01-positive cancer patients (2 cases) (oropharyngeal cancer, lung cancer, esophageal cancer, stomach cancer, breast cancer, pancreatic cancer, ovarian cancer, gallbladder cancer, cholangiocarcinoma, prostate cancer, and uterine cancer) were thawed and isolated by density gradient centrifugation using Ficoll-Plaque Premium, and then suspended in serum-free AIM-V medium. Cells were seeded onto Primaria cell culture dishes (10 cm) and left in an incubator at 37°C and 5% CO₂ for at least 30 minutes to separate cells into monocytes adhering to the plastic and non-adherent (NAD) cells, and the obtained cells were cryopreserved separately. Also, the adherent monocytes were cultured in serum-free AIM-V medium containing granulocyte macrophage colony-stimulating factor (GM-CSF) (50 ng/ml) and IL-4 (50 ng/ml) in an incubator at 37°C and 5% CO₂ for 5 days to prepare immature dendritic cells (imDC), and the obtained cells were cryopreserved.

### Preparation of WT1 peptide-pulsed mature dendritic cells (mDC) and WT1 peptide-pulsed immature dendritic cells (imDC)

The cryopreserved imDC (1 × 10⁵ cells) were thawed, and 100 µg of WT1₃₄₋₅₁ was added to the cells in the presence of 5 ng/ml GM-CSF, 50 ng/ml prostaglandin E2, and 10 µg/ml OK-432. The cells were then incubated at 37°C and 5% CO₂ for 24 hours to prepare WT1 peptide-pulsed mature dendritic cells (mDC/WT1₃₄₋₅₁). Also, the cryopreserved imDCs (1 × 10⁵ cells) were thawed and incubated in the presence of 5 ng/ml GM-CSF, 50 ng/ml prostaglandin E2, and 10 µg/ml OK-432 at 37°C and 5% CO₂ for 24 hours to prepare WT1 peptide-unpulsed mature dendritic cells (mDC). In addition, 25 µg of WT1₃₄₋₅₁, WT1₃₇₋₄₅, or WT1₄₀₋₄₈ was added to imDC (1 × 10⁴ cells), and then the cells were incubated at 37°C and 5% CO₂ for 24 hours to prepare WT1 peptide-pulsed . immature dendritic cells (imDC/WT1₃₄₋₅₁, imDC/WT1₃₇₋₄₅ or imDC/WT1₄₀₋₄₈).

### Induction of WT1 peptide-specific CD8-positive T cells

The cryopreserved NAD cells were thawed and suspended in RPMI-1640 medium containing 1% nonessential amino acids, 1 mM sodium pyruvate, 50 µM 2-mercaptoethanol, 10% inactivated fetal bovine serum, 20 U/ml of IL-2, and 20 ng/ml IL-7, and cultured in 24-well plates in an incubator at 37°C and 5% CO₂ for 24 hours. Then, mDC/WT1₃₄₋₅₁ (1 × 10⁵ cells) and the NAD cells (1 × 10⁶ cells) were suspended in RPMI-1640 medium containing 1% nonessential amino acids, 1 mM sodium pyruvate, 50 µM 2-mercaptoethanol, 10 U/ml IL -2, 10 ng/ml IL-7 and 10% inactivated fetal bovine serum and cultured in an incubator at 37°C and 5% CO₂. After 7 days (on day 8), mDC/WT1₃₄₋₅₁ (1 × 10⁵ cells) were freshly prepared, and the culturing was continued in a freshly prepared cell culture medium containing 10 U/ml IL-2 and 10 ng/ml IL-7. This operation was performed two more times (on days 15 and 22) for a total of four stimulations (on days 1, 8, 15, and 22). As the control, IL-2 (10 U/ml) and IL-7 (10 ng/ml) were added to NAD cells four times at 7-day intervals (on days 1, 8, 15 and 22).

### Detection of IFN-γ-producing WT1 peptide-specific CD8-positive T cells

For restimulation, T cells induced by culturing with four stimulations with mDC/WT1₃₄₋₅₁ (1 × 10⁵ cells) were seeded onto 96-well U-bottom plates with imDC/WT1₃₄₋₅₁, imDC/WT1₃₇₋₄₅ or imDC/WT1₄₀₋₄₈ (1 × 10⁵ cells), and after GolgiStop was added, the cells were cultured in an incubator at 37°C and 5% CO₂ for 6 hours. A human Fc receptor blocker was then added to the cells, and the cells were incubated at 4°C for 5 minutes. After the cells were incubated with a PE/Cy5-labled anti-human CD8 antibody (clone RPA-T8; eBioscience) and an APC/Cy7-labled anti-human CD4 antibody (clone OKT4; BioLegend) at 4°C for 20 min, the cells were permeabilized with BD Cytofix/Cytoperm Plus Fixation/permeabilized with BD GolgiStop (BD Biosciences). The cells were then incubated with an APC-labeled anti-human IFN-y antibody (clone B27; BioLegend) and analyzed for the frequency of IFN-γ-producing cells among CD8-positive cells.

### Statistical analysis

Paired t-test or Wilcoxon signed-rank test was used to test the results.

### 2. Results

### Induction of HLA-A*02:01-restricted WT1₃₇₋₄₅-specific CD8-positive T cells

NAD cells of HLA-A*02:01 -positive subjects (5 cases) were stimulated with mDC/WT1₃₄₋₅₁ every 7 days a total of four times, and then restimulated with imDC/WT1₃₇₋₄₅ or imDC. The frequency of IFN-γ-producing CD8-positive T cells was then analyzed. The results showed that in 3 of 5 cases (60%), the frequency of IFN-γ-producing CD8-positive T cells among CD8-positive T cells was increased by imDC/WT1₃₇₋₄₅ restimulation greater than by imDC restimulation by 10% or more.

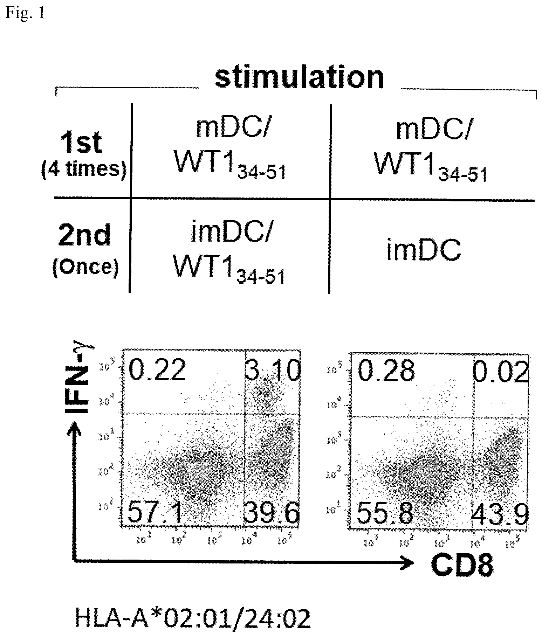

The frequency of IFN-γ-producing CD8-positive T cells among CD8-positive T cells were analyzed when NAD cells from an HLA-A*02:01/24:02-positive subject (1 case) were stimulated with mDC/WT1₃₄₋₅₁ four times and then restimulated with imDC/WT1₃₄₋₅₁ or imDC. The frequency of IFN-γ-producing CD8-positive T cells after restimulation with imDC/WT1₃₄₋₅₁ and that with imDC were approximately 7.3% and 0.05%, respectively (Fig. 1).

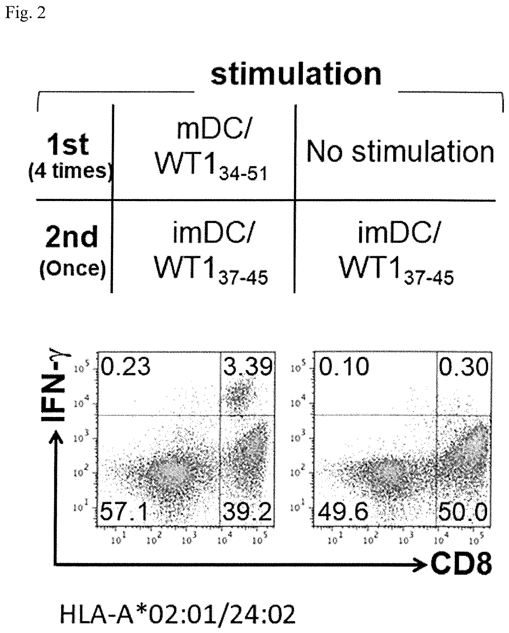

The frequency of IFN-γ-producing CD8-positive T cells among CD8-positive T cells was analyzed when NAD cells from the same patient were stimulated four times with mDC/WT1₃₄₋₅₁ and then restimulated with imDC/WT1₃₇₋₄₅, or when the NAD cells were stimulated with imDC/WT1₃₇₋₄₅ only once, without the four stimulations. The frequency of IFN-γ-producing CD8-positive T cells with the four stimulations was approximately 8.0% and that without the four stimulations was approximately 0.6% (Fig. 2).

The above results demonstrated that WT1₃₄₋₅₁ can induce IFN-γ-producing HLA-A*02:01-restricted WT1₃₇₋₄₅-specific CD8-positive T cells.

### Induction of HLA-A*02:06-restricted WT1₃₇₋₄₅-specific CD8-positive T cells

NAD cells of HLA-A*02:06-positive subjects (7 cases) were stimulated with mDC/WT1₃₄₋₅₁ every 7 days a total of four times, and then restimulated with imDC/WT1₃₇₋₄₅ or imDC. The frequency of IFN-γ-producing CD8-positive T cells was then analyzed. The results showed that in 2 of 7 cases (28.6%), the frequency of IFN-γ-producing CD8-positive T cells among CD8-positive T cells was increased by imDC/WT1₃₇₋₄₅ restimulation greater than by imDC restimulation by 10% or more.

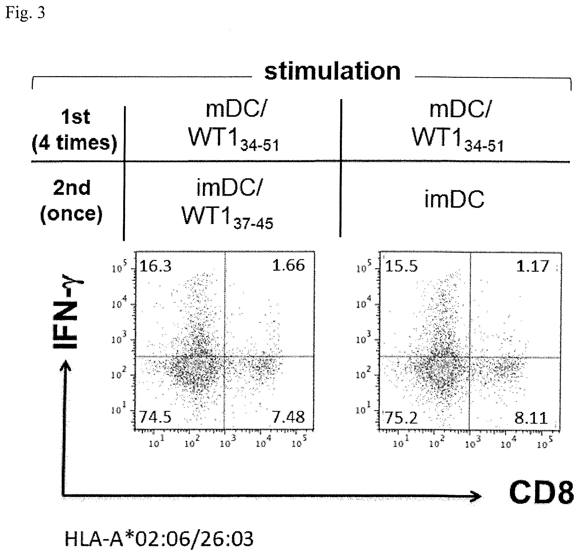

The frequency of IFN-γ-producing CD8-positive T cells among CD8-positive T cells were analyzed when NAD cells from an HLA-A*02:06/26:03-positive subject (1 case) were stimulated with mDC/WT1₃₄₋₅₁ four times and then restimulated with imDC/WT1₃₇₋₄₅ or imDC. The frequency of IFN-γ-producing CD8-positive T cells after restimulation with imDC/WT1₃₇₋₄₅ and that with imDC were approximately 18.2% and 12.6%, respectively (Fig. 3).

The above results demonstrated that WT1₃₄₋₅₁ can induce IFN-γ-producing HLA-A*02:06-restricted WT1₃₇₋₄₅-specific CD8-positive T cells.

### Induction of HLA-A*02:07-restricted WT1₃₇₋₄₅-specific CD8-positive T cells

NAD cells of HLA-A*02:07-positive subjects (2 cases) were stimulated with mDC/WT1₃₄₋₅₁ every 7 days a total of four times, and then restimulated with imDC/WT1₃₇₋₄₅ or imDC. The frequency of IFN-γ-producing CD8-positive T cells was then analyzed. The results showed that in 1 of 2 cases (50%), the frequency of IFN-γ-producing CD8-positive T cells among CD8-positive T cells was increased by imDC/WT1₃₇₋₄₅ restimulation greater than by imDC restimulation by 10% or more.

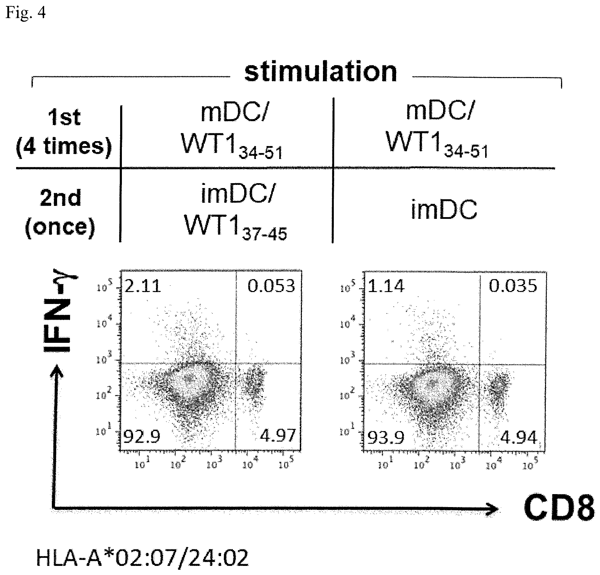

The frequency of IFN-γ-producing CD8-positive T cells were analyzed when NAD cells from an HLA-A*02:07/24:02-positive subject (1 case) were stimulated with mDC/WT1₃₄₋₅₁ four times and then restimulated with imDC/WT1₃₇₋₄₅ or imDC. The frequency of IFN-γ-producing CD8-positive T cells among CD8-positive T cells after restimulation with imDC/WT1₃₇₋₄₅ and that with imDC were approximately 1.1% and 0.7%, respectively (Fig. 4).

The above results demonstrated that WT1₃₄₋₅₁ can induce IFN-γ-producing HLA-A*02:07-restricted WT1₃₇₋₄₅-specific CD8-positive T cells.

### Induction of HLA-A*26:01-restricted WT1₄₀₋₄₈-specific CD8-positive T cells

NAD cells of HLA-A*26:01-positive subjects (2 cases) were stimulated with mDC/WT1₃₄₋₅₁ every 7 days a total of four times, and then restimulated with imDC/WT1₄₀₋₄₈. The frequency of IFN-γ-producing CD8-positive T cells was then analyzed. The results showed that in 2 of 2 cases (100%), IFN-γ-producing CD8-positive T cells were observed.

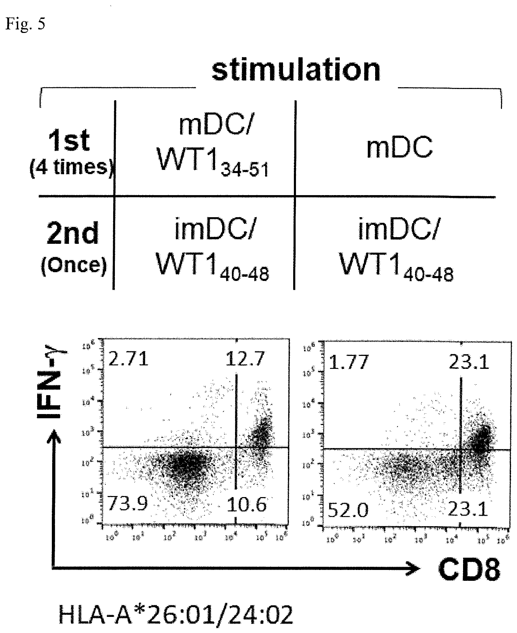

The frequency of IFN-γ-producing CD8-positive T cells were analyzed when NAD cells from an HLA-A*26:01/24:02-positive subject (1 case) were stimulated with mDC/WT1₃₄₋₅₁ four times and then restimulated with imDC/WT1₄₀₋₄₈ or when the NAD cells were stimulated with WT1 peptide-unpulsed mature dendritic cells (mDC) four times and then restimulated with imDC/WT1₄₀₋₄₈. The frequency of IFN-γ-producing CD8-positive T cells among CD8-positive T cells after four stimulations with mDC/WT1₃₄₋₅₁ and that with mDC were approximately 54.5% and 50%, respectively (Fig. 5).

The above results demonstrated that WT1₃₄₋₅₁ can induce IFN-γ-producing HLA-A*26:01-restricted WT1₄₀₋₄₈-specific CD8-positive T cells.

II. Induction of WT1-specific CD8-positive T cells with WT1₃₄₋₅₁ and the compound of formula (5) or with WT1₃₄₋₅₁ (1)

### 1. Methods

### Stimulation of PBMCs

Peripheral blood was collected from HLA-A*02:01/24:02-positive subjects (3 cases), an HLA-A*02:06/24:02-positive subject (1 case), HLA-A*02:07/24:02-positive subjects (2 cases) and an HLA-A*24:02/24:02-positive subject (1 case) (each case was a cancer patient or healthy subject) and a mononuclear cell fraction was isolated by density gradient centrifugation using a lymphocyte separation solution (Nacalai Tesque) to obtain PBMCs.

PBMCs were suspended in human complete medium (herein after referred to as hCM), which was a mixture of equal volumes of RPMI 1640 (Nacalai Tesque) and AIM-V (Gibco) containing 10% inactivated AB human serum, 1× MEM nonessential amino acid solution (Nacalai Tesque), 100 µM 2-mercaptoethanol (Fujifilm Wako Pure Chemical Corporation), 50 IU/ml penicillin G (Meiji Seika Pharma), and 50 mg/ml streptomycin (Meiji Seika Pharma) at 3 × 10⁶ cells/ml. To this PBMC/hCM suspension, the compound of formula (5) (27 µg/ml) and WT1₃₄₋₅₁ (20 µg/ml) (ratio of 1 :0.75) or WT1_{34.51} (20 µg/ml) alone was added and the resulting suspension was dispensed in 1 ml portions into 24-well plates. Two to four wells were prepared per condition. On days 2 to 3, 500 µl of hCM containing 40 IU/ml IL-2 (Imunace, Kyowa Pharmaceutical Industry Co., Ltd.) was added to the cells. Two to three days later, 500 µl of the medium was removed from each well and 500 µl of hCM containing 40 IU/ml IL-2 was added (the half of the medium was changed). The half of the medium was changed in the same manner every 2 to 3 days while the color of the medium was observed.

In some experiments, the second stimulation was performed 7 to 10 days after the first stimulation described above. Autologous PBMCs were used as antigen-presenting cells (APCs). For APC preparation, PBMCs of 3 to 4 × 10⁶ cells were suspended in 1 ml of RPMI 1640 (serum-free), and the compound of formula (5) (27 µg/ml) and WT1₃₄₋₅₁ (20 µg/ml) or WT1₃₄₋₅₁ (20 µg/ml) alone was added to the suspension and allowed to react for 2 hours at 37°C. The cells were irradiated with 30 Gy radiation, and the irradiated cells were washed twice with PBS and suspended with hCM at 1 × 10⁶ cells/500 µl to provide an APC suspension. Seven to twelve days after the first stimulation, 500 µl of the medium was removed from each well and the APC suspension of 1 × 10⁶ cells/500 µl was added to the well. Two to three days later, 500 µl of the medium was removed from each well and 500 µl of hCM containing 40 IU/ml IL-2 was added (the half of the medium was changed). The half of the medium was changed in the same manner every 2 to 3 days.

### Intracellular cytokine assay

Seven to twelve days after each stimulation, the cells and the medium in each well of the 24-well plate were mixed well by pipetting and 500 µl of the cell-containing medium was collected. After centrifugation to remove the supernatant, 800 µl of hCM containing 10 µg/ml brefeldin A (BFA, SIGMA) was added to the cells to prepare a cell suspension. WT1₃₇₋₄₅ or WT1₄₀₋₄₈ were added to wells of 96-well plates at a final concentration of 20 µg/ml, respectively. Wells without peptide addition were also prepared as controls. To these wells, 200 µl of the prepared cell suspension was added per well and allowed to react for 4 hours at 37°C.

The cells were collected from each well, centrifuged to remove the supernatant, and washed twice with FACS Buffer (PBS with 2% FCS). For staining of surface antigens, the cells were reacted with an eFluor450-labeled anti-human CD3 antibody (clone UCHT1, eBioscience), an APC/Cy7-labeled anti-human CD8 antibody (clone RPA-T8, BioLegend), and FITC-labeled anti-human CD4 antibody (clone OKT4, eBioscience) for 30 minutes on ice and then washed twice with FACS Buffer.

Then, 50 µl of BD Fixation/Permeabilization solution (BD Biosciences) was added to each well for cell fixation and membrane permeabilization and allowed to react for 30 minutes on ice. After washed twice with 10-fold diluted BD Perm/Wash Buffer (BD Biosciences), the cells were reacted with a PE-labeled anti-human IFN-y antibody (clone 4S.B3, eBioscience) and an APC-labeled anti-human TNF-α antibody (clone MAb11, eBioscience) for 30 minutes on ice for intracellular cytokine staining.

The cells reacted with antibodies were suspended in FACS Buffer and measured with BD FACSCanto II flow cytometer (BD Biosciences). The results were analyzed with FLOWJO software (BD Biosciences).

### Tetramer assay

Seven to twelve days after each stimulation, the cells and the medium in each well of the 24-well plate were mixed well by pipetting, and 500 µl of the cell-containing medium was collected and divided into two equal portions and placed in two FACS tubes. After the cells were washed twice with FACS Buffer, Clear Back (Human FcR blocking reagent, MBL) was added to the cells and allowed to react for 5 minutes at room temperature. To one of the two tubes, PE-labeled HLA-A*02:01 WT1₃₇₋₄₅ Tetramer (MBL) was added and allowed to react for 60 minutes on ice. The other tube was a tetramer-free tube. To these tubes, a Pacific Blue-labeled anti-human CD3 antibody (clone UCHT1, BD Biosciences) and an FITC-labeled anti-human CD8 antibody (clone T8, Cytostat/Coulter clone, BECKMAN COULTER) were added and allowed to react for 30 minutes on ice. The cells were washed twice with FACS Buffer, suspended in FACS Buffer containing 7-AAD Viability Staining Solution (BioLegend), and measured with BD FACSCanto II fluorometer (BD Biosciences). The results were analyzed by FLOWJO software (BD Biosciences).

### 2. Results

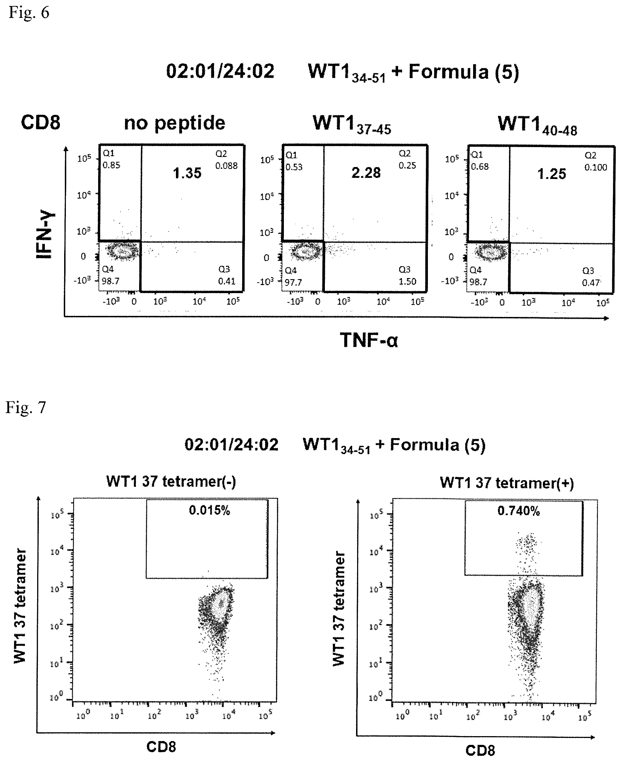

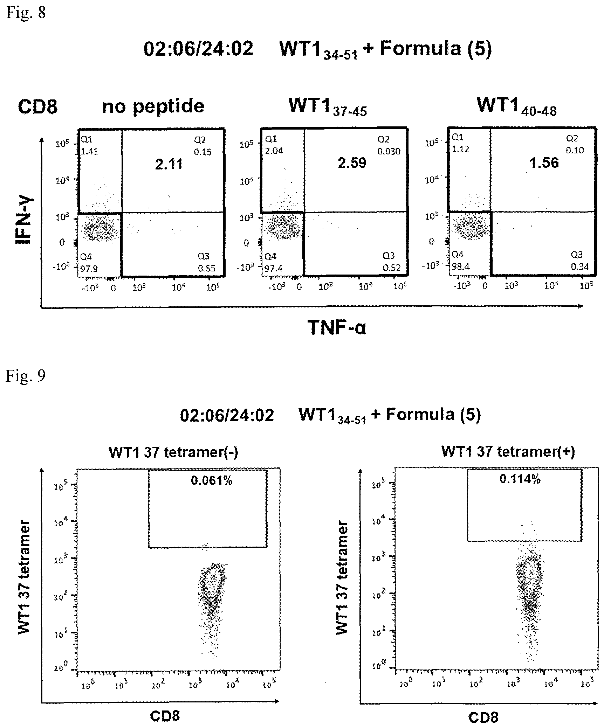

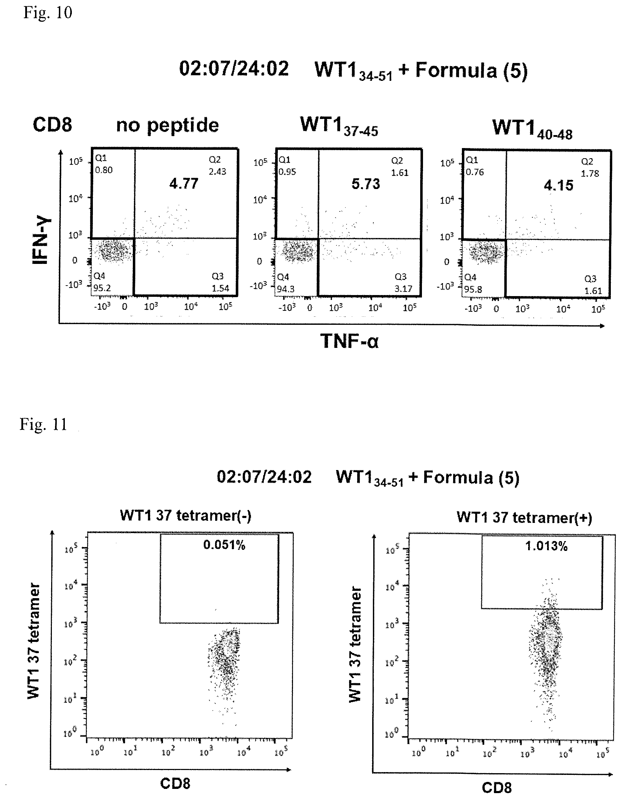

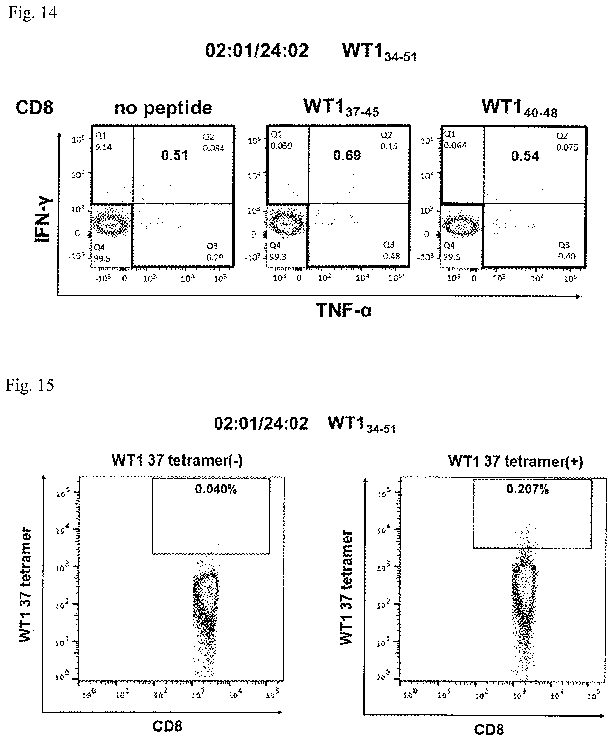

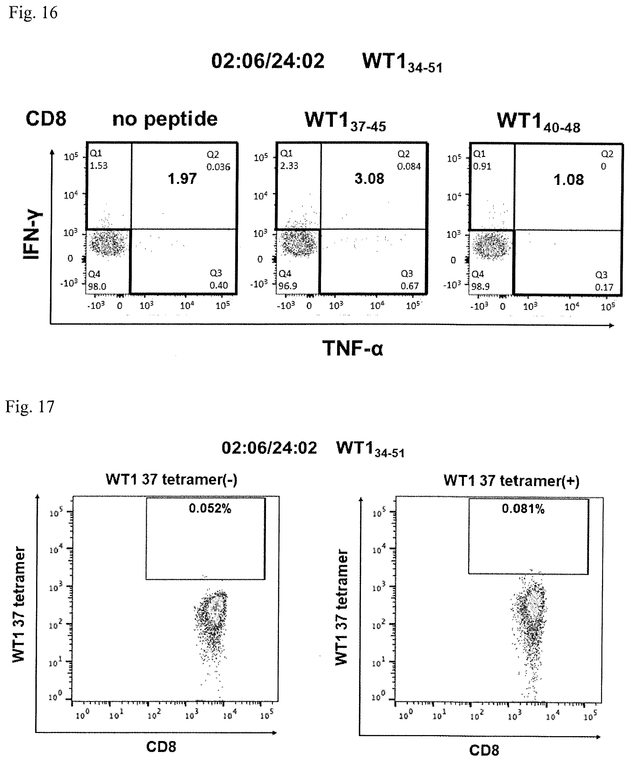

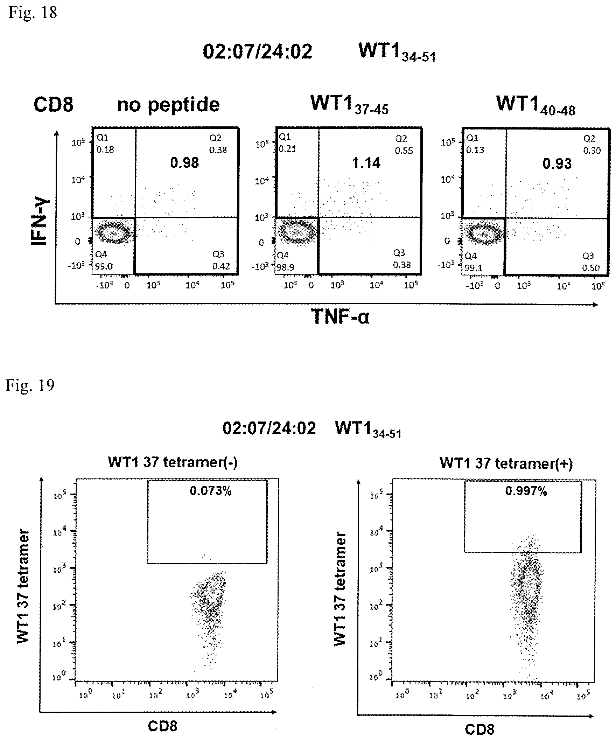

The following results are shown in Figs. 6 to 21.

**Table 1**

| HLA-A | | Peptide(s) for stimulation | | | |
|---|---|---|---|---|---|
| | | WT1₃₄₋₅₁ + Formula (5) | | WT1₃₄₋₅₁ | |
| 02:01/24:02 | | | | | |
| | Cytokine assay | Fig. 6 | (8 days after the first stimulation) | Fig. 14 | (8 days after the first stimulation) |
| | Tetramer assay | Fig. 7 | (8 days after the first stimulation) | Fig. 15 | (8 days after the first stimulation) |
| 02:06/24:02 | | | | | |
| | Cytokine assay | Fig. 8 | (12 days after the first stimulation) | Fig. 16 | (12 days after the first stimulation) |
| | Tetramer assay | Fig. 9 | (8 days after the first stimulation) | Fig. 17 | (8 days after the first stimulation) |
| 02:07/24:02 | | | | | |
| | Cytokine assay | Fig. 10 | (8 days afterthe first stimulation) | Fig. 18 | (8 days after the first stimulation) |
| | Tetramer assay | Fig. 11 | (8 days afterthe first stimulation) | Fig. 19 | (8 days afterthe first stimulation) |
| 24:02/24:02 | | | | | |
| | Cytokine assay | Fig. 12 | (9 days afterthe second stimulation) | Fig. 20 | (9 days afterthe second stimulation) |
| | Tetramer assay | Fig. 13 | (9 days after the second stimulation) | Fig. 21 | (9 days after the second stimulation) |

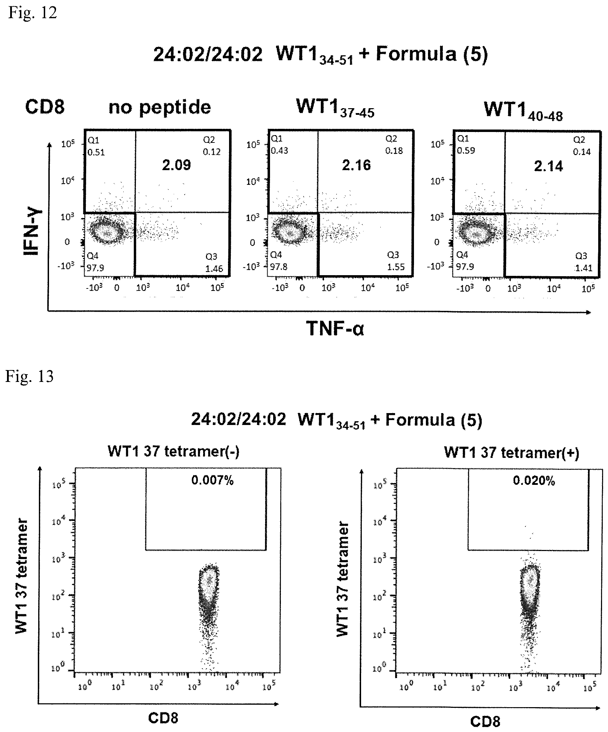

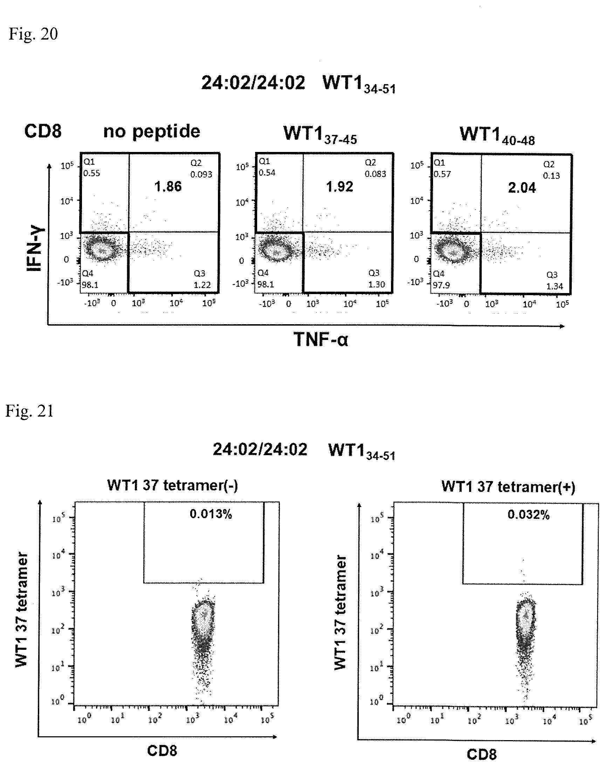

From PBMCs of an HLA-A*02:01/24:02-positive subject, HLA-A*02:06/24:02-positive subject or HLA-A*02:07/24:02-positive subject stimulated with WT1₃₄₋₅₁ and the compound of formula (5) or WT1₃₄₋₅₁ alone, TNF-α- and/or INF-γ-producing CD8-positive cells were induced by WT1₃₇₋₄₅ stimulation, indicating that WT1₃₇₋₄₅-specific CD8-positive T cells were induced (Figs. 6, 8, 10, 14, 16, and 18). In contrast, no induction of WT1₃₇₋₄₅-specific CD8-positive T cells was observed in PBMCs from HLA-A*24:02/24:02-positive subject even after two rounds of stimulation (Figs. 12 and 20). No TNF-α- and/or INF-γ-producing CD8-positive cells were observed upon stimulation with WT1₄₀₋₄₈, which was used as a negative control in this experiment. The tetramer used in the tetramer assay was considered to detect, not only HLA-A*02:01-restricted WT1₃₇₋₄₅-specific CD8-positive T cells, but also HLA-A*02:06- or HLA-A*02:07-restricted WT1₃₇₋₄₅-specific CD8-positive T cells, those restricted to an HLA type belonging to the HLAA2 subtype, although the sensitivity was lower. In the tetramer assay, PBMCs of an HLA-A*02:01/24:02-positive subject, HLA-A*02:06/24:02-positive subject, or HLA-A*02:07/24:02-positive subject stimulated with WT1₃₄₋₅₁ and the compound of formula (5) or WT1₃₄₋₅₁ alone showed increased tetramer positive cells (Figs. 7, 9, 11, 15, 17, and 19). The tetramer used in this experiment did not show specific staining in PBMCs of an HLA-A*24:02/24:02-positive subject, which did not have any HLA-A2 subtype (Figs. 13 and 21). These results confirmed that PBMCs stimulated with WT1₃₄₋₅₁ and the compounds of formula (5) induced HLA-A*0 2:01, HLA-A*02:06, or HLA-A*02:07-restricted WT1₃₇₋₄₅-specific CD8-positive T cells but did not induce HLA-A*24:02-restricted cells.

III. Induction of WT1-specific CD8-positive T cells with WT1₃₄₋₅₁ and the compound of formula (5) or with WT1₃₄₋₅₁ (2)

### 1. Methods

### Stimulation of PBMCs

Peripheral blood was collected from an HLA-A*01:01 -positive subject, an HLA-A*26:01-positive subject, an HLA-B*35:01-positive subject, an HLA-C*03:03-positive subject, an HLA-C*05:01-positive subject, or HLA-C*07:01-positive subject (1 case each) (cancer patient or healthy subject) and a mononuclear cell fraction was isolated by density gradient centrifugation using a lymphocyte separation solution (Nacalai Tesque) to obtain PBMCs.

PBMCs were suspended in human complete medium (herein after referred to as hCM), which was a mixture of equal volumes of RPMI 1640 (Nacalai Tesque) and AIM-V (Gibco) containing 10% inactivated AB human serum, 1 × MEM nonessential amino acid solution (Nacalai Tesque), 100 µM 2-mercaptoethanol (Fujifilm Wako Pure Chemical Corporation), 50 IU/ml penicillin G (Meiji Seika Pharma), and 50 mg/ml streptomycin (Meiji Seika Pharma) at 2 to 3 × 10⁶ cells/ml. To this PBMC/hCM suspension, the compound of formula (5) (27 µg/ml) and WT1₃₄₋₅₁ (20 µg/ml) (ratio of 1:0.75) or WT1₃₄₋₅₁ (20 µg/ml) alone was added and the resulting suspension was dispensed in 1 ml portions into 24-well plates. Two to three wells were prepared per condition. On days 2 to 3, 500 µl of hCM containing 40 IU/ml IL-2 (Recombinant Human IL-2, PeproTech) was added to the cells. Two to three days later, 750 µl of the medium was removed from each well and 750 µl of hCM containing 40 IU/ml IL-2 was added (the half of the medium was changed). The half of the medium was changed in the same manner every 2 to 3 days while the color of the medium was observed.

The second and third stimulations were performed 7 to 10 days and 14 to 20 days after the first stimulation described above, respectively. Autologous PBMCs were used as APCs. For APC preparation, PBMCs of 1 × 10⁶ cells were suspended in 800 µl of AIM-V (serum-free), and the compound of formula (5) (27 µg/ml) and WT1₃₄₋₅₁ (20 µg/ml) or WT1₃₄₋₅₁ (20 µg/ml) alone was added to the suspension and allowed to react for 2 hours at 37°C. The cells were irradiated with 30 Gy radiation to provide an APC suspension. Seven to ten days after the first stimulation and fourteen to twenty days after the first stimulation, 800 µl of the medium was removed from each well and the APC suspension of 1 × 10⁶ cells/800 µl was added to the well. Two to three days later, 750 µl of the medium was removed from each well and 750 µl of hCM containing 40 lU/ml IL-2 was added (the half of the medium was changed). The half of the medium was changed in the same manner every 2 to 3 days.

### Intracellular cytokine assay

PBMCs that differed in only one HLA class I allele from PBMCs used for the preceding three stimulations and that were pulsed with WT1₃₇₋₄₅ or WT1₄₀₋₄₈ or not pulsed with any peptide (see Table 4) were used as APCs for the intracellular cytokine and tetramer assay. For APC preparation, PBMCs of 3 × 10⁵ cells were suspended in 300 µl of RPMI 1640 (serum-free), and WT1₃₇₋₄₅ (20 µg/ml) or WT1₄₀₋₄₈ (20 µg/ml) or no peptide was added to the suspension and allowed to react for 1 hour at 37°C. The cells were washed twice with PBS, and hCM containing brefeldin A (BFA) (SIGMA) was added to the cells at 1×10⁵ cells/100 µl to prepare an APC suspension. Twenty-one days after the first stimulation, the stimulated cells and the medium were mixed well by pipetting and 900 µl of the cell-containing medium was collected from each well. After the supernatant was removed by centrifugation, hCM containing BFA was added to the cells to prepare a cell suspension. In a round-bottom 96-well plate, 100 µl each of the APC suspension and the cell suspension thus prepared were added and co-cultured at 37°C and 5% CO₂ for 4 hours. The reaction was performed in triplicate.

After the stimulation, the supernatant was removed by centrifugation and the cells were washed twice with FACS Buffer (PBS with 2% FCS). For staining of surface antigens, the cells were reacted with a PE/Cy7-labeled anti-human CD3 antibody (clone SK-7, BioLegend), an APC/Cy7-labeled anti-human CD8 antibody (clone RPA-T8, BioLegend), and an FITC-labeled anti-human CD4 antibody (clone RPA-T, BioLegend) for 30 minutes on ice and then washed twice with FACS Buffer.

Then, 50 µl of BD Fixation/Permeabilization solution (BD Biosciences) was added to each well for cell fixation and membrane permeabilization and allowed to react for 20 minutes on ice. After washed twice with 10-fold diluted BD Perm/Wash Buffer (BD Biosciences), the cells were reacted with a PE-labeled anti-human IFN-γ antibody (clone 4S.B3, eBioscience) and an APC-labeled anti-human TNF-α antibody (clone MAb11, eBioscience) for 30 minutes on ice for intracellular cytokine staining.

The cells reacted with antibodies were suspended in FACS Buffer and measured with BD FACSCanto II flow cytometer (BD Biosciences). The results were analyzed with FLOWJO software (BD Biosciences).

For tetramer assay, the stimulated cells and the medium were mixed well by pipetting, and 500 µl of the cell-containing medium was collected and divided into two equal portions and placed in two FACS tubes. After the cells were washed twice with FACS Buffer, Clear Back (Human FcR blocking reagent, MBL) was added to the cells and allowed to react for 5 minutes at room temperature. To one of the two tubes, PE-labeled HLA-A*01:01 WT1₄₀₋₄₈ Tetramer (MBL) or PE-labeled HLA-B*35:01 WT1₄₀₋₄₈ Tetramer (MBL) was added and allowed to react for 60 minutes on ice. The other tube was a tetramer-free tube. To these tubes, a Pacific Blue-labeled anti-human CD3 antibody (clone UCHT1, BD Biosciences) and an FITC-labeled anti-human CD8 antibody (clone T8, Cytostat/Coulter clone, BECKMAN COULTER) were added and allowed to react for 30 minutes on ice. The cells were washed twice with FACS Buffer, suspended in FACS Buffer containing 7-AAD Viability Staining Solution (BioLegend), and measured with BD FACSCanto II fluorometer (BD Biosciences). The results were analyzed by FLOWJO software (BD Biosciences).

### 2. Results

The 9-mer peptides capable of binding to MHC class I molecules in the sequence of WT1₃₄₋₅₁ (WAPVLDFAPPGASAYGSL) (SEQ ID NO: 2) were analyzed in NetMHC-4.0 (http://www.cbs.dtu.dk/services/NetMHC-4.0). The analyzed peptides are shown in Table 2, and the predicted binding results are shown in Tables 3-1 to 3-32. In Tables 3-1 to 3-32, The column "Rank" is the %Rank compared to 400,000 random natural peptides, "H_Avg _Rank" is the harmonic mean of the %Rank calculated for all specified alleles, and "N_binders" is the number of alleles covered by that peptide. The symbol "WB" means a weak binder and "SB" means a strong binder.

**Table 2**

| Positions | Sequence | SEQ ID NO: |
|---|---|---|
| 34-42 | WAPVLDFAP | 10 |
| 35-43 | APVLDFAPP | 11 |
| 36-44 | PVLDFAPPG | 12 |
| 37-45 | VLDFAPPGA | 5 |
| 38-46 | LDFAPPGAS | 13 |
| 39-47 | DFAPPGASA | 14 |
| 40-48 | FAPPGASAY | 6 |
| 41-49 | APPGASAYG | 15 |
| 42-50 | PPGASAYGS | 16 |
| 43-51 | PGASAYGSL | 17 |

**Table 3-1**

| | HLA-A*01:01 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 5080.9 | 1.6 | 1.6 | 1 | WB |
| 37-45 | VLDFAPPGA (SEQ ID NO: 5) | 9674.1 | 3 | 3.0 | 0 | |
| 43-51 | PGASAYGSL (SEQ ID NO: 17) | 23637.4 | 16 | 16.0 | 0 | |
| 39-47 | DFAPPGASA (SEQ ID NO: 14) | 27828.7 | 28 | 28.0 | 0 | |
| 34-42 | WAPVLDFAP (SEQ ID NO: 10) | 28442.4 | 30 | 30.0 | 0 | |
| 38-46 | LDFAPPGAS (SEQ ID NO: 13) | 31534.7 | 43 | 43.0 | 0 | |
| 36-44 | PVLDFAPPG (SEQ ID NO: 12) | 32070.5 | 46 | 46.0 | 0 | |
| 42-50 | PPGASAYGS (SEQ ID NO: 16) | 32645.7 | 49 | 49.0 | 0 | |
| 35-43 | APVLDFAPP (SEQ ID NO: 11) | 33882.9 | 60 | 60.0 | 0 | |
| 41-49 | APPGASAYG (SEQ ID NO: 15) | 36290.7 | 70 | 70.0 | 0 | |

**Table 3-2**

| | HLA-A*02:01 | | | | | |
|---|---|---|---|---|---|---|
| Pos | Peptide | nM | Rank | H_Avg_Ranks | N_binders | BindLevel |
| 37-45 | VLDFAPPGA (SEQ ID NO: 5) | 170.8 | 1.5 | 1.5 . | 1 | VVB |
| 34-42 | WAPVLDFAP (SEQ ID NO: 10) | 25644.6 | 37 | 37.0 | 0 | |
| 35-42 | APVLDFAPP (SEQ ID NO: 11) | 28152.1 | 42 | 42.0 | 0 | |
| 40-48 | FAPPGASAY (SEQ ID NO: 6) | 28809.7 | 44 | 44.0 | 0 | |
| 36-44 | PVLDFAPPG (SEQ ID NO: 12) | 30541.1 | 48 | 48.0 | 0 | |
| 38-46 | LDFAPPGAS (SEQ ID NO: 13) | 37979.2 | 75 | 75.0 | 0 | |
| 39-47 | DFAPPGASA (SEQ ID NO: 14) | 38383.6 | 75 | 75.0 | 0 | |
| 41-49 | APPGASAYG (SEQ ID NO: 15) | 39072.5 | 80 | 80.0 | 0 | |
| 43-51 | PGASAYGSL (SEQ ID NO: 17) | 41187.0 | 90 | 90.0 | 0 | |
| 42-50 | PPGASAYGS (SEQ ID NO: 16) | 43104.1 | 95 | 95.0 | 0 | |

**Table 3-3**

| | HLA-A*02:07 | | | | | |
|---|---|---|---|---|---|---|
| Pos | Peptide | nM | Rank | H_Avg_Ranks | N_binders | BindLevel |
| 37-45 | VLDFAPPGA (SEQ ID NO: 5) | 18851.0 | 1.6 | 1.6 | 1 | WB |
| 36-44 | PVLDFAPPG (SEQ ID NO: 12) | 37981.2 | 18 | 18.0 | 0 | |
| 40-48 | FAPPGASAY (SEQ ID NO: 6) | 38061.9 | 18 | 18.0 | 0 | |
| 41-49 | APPGASAYG (SEQ ID NO: 15) | 38315.6 | 19 | 19.0 | 0 | |
| 35-43 | APVLDFAPP (SEQ ID NO: 11) | 38482.2 | 19 | 19.0 | 0 | |
| 34-42 | WAPVLDFAP (SEQ ID NO: 10) | 38485.9 | 19 | 19.0 | 0 | |
| 43-51 | PGASAYGSL (SEQ ID NO: 17) | 39604.5 | 23 | 23.0 | 0 | |
| 39-47 | DFAPPGASA (SEQ ID NO: 14) | 44170.6 | 55 | 55.0 | 0 | |
| 38-46 | LDFAPPGAS (SEQ ID NO: 13) | 44726.1 | 60 | 60.0 | 0 | |
| 42-50 | PPGASAYGS (SEQ ID NO: 16) | 45456.0 | 75 | 75.0 | 0 | |

| Table 3-4 | | | | | | |
|---|---|---|---|---|---|---|
| | HLA-A*02:11 | | | | | |
| Pos | Peptide | nM | Rank | H_Avg_Ranks | N_binders | BindLevel |
| 37-45 | VLDFAPPGA (SEQ ID NO: 5) | 23.8 | 0.7 | 0.7 | 1 | WB |
| 36-44 | PVLDFAPPG (SEQ ID NO: 12) | 18019.6 | 13 | 13 | 0 | |
| 38-46 | LDFAPPGAS (SEQ ID NO: 13) | 28928.7 | 21 | 21 | 0 | |
| 43-51 | PGASAYGSL (SEQ ID NO: 17) | 29330.2 | 21 | 21 | 0 | |
| 40-48 | FAPPGASAY (SEQ ID NO: 6) | 29655.7 | 22 | 22 | 0 | |
| 41-49 | APPGASAYG (SEQ ID NO: 15) | 34917.2 | 29 | 29 | 0 | |
| 39-47 | DFAPPGASA (SEQ ID NO: 14) | 35923.5 | 30 | 30 | 0 | |
| 42-50 | PPGASAYGS (SEQ ID NO: 16) | 38327.2 | 35 | 35 | 0 | |
| 34-42 | WAPVLDFAP (SEQ ID NO: 10) | 41670.2 | 46 | 46 | 0 | |
| 35-43 | APVLDFAPP (SEQ ID NO: 11) | 44215.6 | 60 | 60 | 0 | |

**Table 3-5**

| | HLA-A*02:12 | | | | | |
|---|---|---|---|---|---|---|
| Pos | Peptide | nM | Rank | H_Avg_Ranks | N_binders | BindLevel |
| 37-45 | VLDFAPPGA (SEQ ID NO: 5) | 682.5 | 1.4 | 1.4 | 1 | WB |
| 36-44 | PVLDFAPPG (SEQ ID NO: 12) | 27176.7 | 17 | 17 | 0 | |
| 35-43 | APVLDFAPP (SEQ ID NO: 11) | 31889.5 | 22 | 22 | 0 | |
| 39-47 | DFAPPGASA (SEQ ID NO: 14) | 35522.3 | 28 | 28 | 0 | |
| 40-48 | FAPPGASAY (SEQ ID NO: 6) | 35974.5 | 29 | 29 | 0 | |
| 41-49 | APPGASAYG (SEQ ID NO: 15) | 37031.7 | 32 | 32 | 0 | |
| 34-42 | WAPVLDFAP (SEQ ID NO: 10) | 37205.6 | 32 | 32 | 0 | |
| 38-46 | LDFAPPGAS (SEQ ID NO: 13) | 38490.1 | 36 | 36 | 0 | |
| 42-50 | PPGASAYGS (SEQ ID NO: 16) | 40765.8 | 44 | 44 | 0 | |
| 43-51 | PGASAYGSL (SEQ ID NO: 17) | 42513.1 | 55 | 55 | 0 | |

**Table 3-6**

| | HLA-A"02:16 | | | | | |
|---|---|---|---|---|---|---|
| Pos | Peptide | nM | Rank | H_Avg_Ranks | N_binders | BindLevel |
| 37-45 | VLDFAPPGA (SEQ ID NO: 5) | 80.2 | 0.6 | 0.6 | 1 | WB |
| 36-44 | PVLDFAPPG (SEQ ID NO: 12) | 9799.8 | 6 | 6.0 | 0 | |
| 39-47 | DFAPPGASA (SEQ ID NO: 14) | 24076.2 | 13 | 13.0 | 0 | |
| 43-51 | PGASAYGSL (SEQ ID NO: 17) | 27809.7 | 16 | 16.0 | 0 | |
| 42-50 | PPGASAYGS (SEQ ID NO: 16) | 34328.7 | 24 | 24.0 | 0 | |
| 40-48 | FAPPGASAY (SEQ ID NO: 6) | 36436.3 | 27 | 27.0 | 0 | |
| 41-49 | APPGASAYG (SEQ ID NO: 15) | 36509.3 | 27 | 27.0 | 0 | |
| 34-42 | WAPVLDFAP (SEQ ID NO: 10) | 42477.7 | 46 | 46.0 | 0 | |
| 38-46 | LDFAPPGAS (SEQ ID NO: 13) | 43132.6 | 49 | 49.0 | 0 | |
| 35-43 | APVLDFAPP (SEQ ID NO: 11) | 43377.4 | 55 | 55.0 | 0 | |

**Table 3-7**

| | HLA-A*02:19 | | | | | |
|---|---|---|---|---|---|---|
| Pos | Peptide | nM | Rank | H_Avg_Ranks | N_binders | BindLevel |
| 37-45 | VLDFAPPGA (SEQ ID NO: 5) | 240.2 | 0.6 | 0.6 | 1 | WB |
| 36-44 | PVLDFAPPG (SEQ ID NO: 12) | 23721.4 | 11 | 11 | 0 | |
| 39-47 | DFAPPGASA (SEQ ID NO: 14) | 31326.6 | 19 | 19 | 0 | |
| 40-48 | FAPPGASAY (SEQ ID NO: 6) | 36755.9 | 29 | 29 | 0 | |
| 43-51 | PGASAYGSL (SEQ ID NO: 17) | 36859.8 | 29 | 29 | 0 | |
| 42-50 | PPGASAYGS (SEQ ID NO: 16) | 37818.0 | 32 | 32 | 0 | |
| 38-46 | LDFAPPGAS (SEQ ID NO: 13) | 41791.6 | 55 | 55 | 0 | |
| 35-43 | APVLDFAPP (SEQ ID NO: 11) | 42082.5 | 55 | 55 | 0 | |
| 41-49 | APPGASAYG (SEQ ID NO: 15) | 42320.4 | 55 | 55 | 0 | |
| 34-42 | WAPVLDFAP (SEQ ID NO: 10) | 43760.6 | 70 | 70 | 0 | |

**Table 3-8**

| | HLA-A*25:01 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 1530.5 | 0.18 | 0.175 | 1 | SB |
| 43-51 | PGASAYGSL (SEQ ID NO: 17) | 22656 | 6.5 | 6.5 | 0 | |
| 39-47 | DFAPPGASA (SEQ ID NO: 14) | 24996.2 | 8.5 | 8.5 | 0 | |
| 35-43 | APVLDFAPP (SEQ ID NO: 11) | 30535.5 | 17 | 17 | 0 | |
| 34-42 | WAPVLDFAP (SEQ ID NO: 10) | 32569.8 | 22 | 22 | 0 | |
| 38-46 | LDFAPPGAS (SEQ ID NO: 13) | 32722.1 | 23 | 23 | 0 | |
| 36-44 | PVLDFAPPG (SEQ ID NO: 12) | 33333.1 | 25 | 25 | 0 | |
| 41-49 | APPGASAYG (SEQ ID NO: 15) | 33955.2 | 27 | 27 | 0 | |
| 42- 50 | PPGASAYGS (SEQ ID NO: 16) | 37712.6 | 48 | 48 | 0 | |
| 37-45 | VLDFAPPGA (SEQ ID NO: 5) | 38347.5 | 55 | 55 | 0 | |

**Table 3-9**

| | HLA-A*26:01 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 274.6 | 0.25 | 0.25 | 1 | SB |
| 34-42 | WAPVLDFAP (SEQ ID NO: 10) | 24808.4 | 18 | 18 | 0 | |
| 35-43 | APVLDFAPP (SEQ ID NO: 11) | 25129.4 | 18 | 18 | 0 | |
| 41-49 | APPGASAYG (SEQ ID NO: 15) | 25890.8 | 20 | 20 | 0 | |
| 39-47 | DFAPPGASA (SEQ ID NO: 14) | 25942.7 | 20 | 20 | 0 | |
| 43-51 | PGASAYGSL (SEQ ID NO: 17) | 27888.7 | 26 | 26 | 0 | |
| 38-46 | LDFAPPGAS (SEQ ID NO: 13) | 28702.0 | 28 | 28 | 0 | |
| 36-44 | PVLDFAPPG (SEQ ID NO: 12) | 31038.5 | 38 | 38 | 0 | |
| 37-45 | VLDFAPPGA (SEQ ID NO: 5) | 32163.3 | 43 | 43 | 0 | |
| 42-50 | PPGASAYGS (SEQ ID NO: 16) | 37270.1 | 75 | 75 | 0 | |

**Table 3-10**

| | HLA-A*26:02 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 355.4 | 0.6 | 0.6 | 1 | WB |
| 39-47 | DFAPPGASA (SEQ ID NO: 14) | 18575.6 | 9.5 | 9.5 | 0 | |
| 35-43 | APVLDFAPP (SEQ ID NO: 11) | 28483.6 | 18 | 18 | 0 | |
| 43-51 | PGASAYGSL (SEQ ID NO: 17) | 29706.1 | 19 | 19 | 0 | |
| 34-42 | WAPVLDFAP (SEQ ID NO: 10) | 30748.7 | 21 | 21 | 0 | |
| 38-46 | LDFAPPGAS (SEQ ID NO: 13) | 36460.0 | 32 | 32 | 0 | |
| 41-49 | APPGASAYG (SEQ ID NO: 15) | 37220.5 | 34 | 34 | 0 | |
| 36-44 | PVLDFAPPG (SEQ ID NO: 12) | 37561.5 | 35 | 35 | 0 | |
| 37-45 | VLDFAPPGA (SEQ ID NO: 5) | 41870.8 | 55 | 55 | 0 | |
| 42-50 | PPGASAYGS (SEQ ID NO: 16) | 42111.6 | 60 | 60 | 0 | |

**Table 3-11**

| | HLA-A*26:03 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 5518.5 | 1.1 | 1.1 | 1 | WB |
| 36-44 | PVLDFAPPG (SEQ ID NO: 12) | 18744.2 | 6 | 6 | 0 | |
| 35-43 | APVLOFAPP (SEQ ID NO: 11) | 19291.9 | 6.5 | 6.5 | 0 | |
| 39-47 | DFAPPGASA (SEQ ID NO: 14) | 22907.7 | 9 | 9 | 0 | |
| 43-51 | PGASAYGSL (SEQ ID NO: 17) | 24537.5 | 11 | 11 | 0 | |
| 34-42 | WAPVLDFAP (SEQ ID NO: 10) | 32073.6 | 22 | 22 | 0 | |
| 38-46 | LDFAPPGAS (SEQ ID NO: 13) | 32823.1 | 23 | 23 | 0 | |
| 41-49 | APPGASAYG (SEQ ID NO: 15) | 33832.4 | 26 | 26 | 0 | |
| 37-45 | VLDFAPPGA (SEQ ID NO: 5) | 35956.6 | 32 | 32 | 0 | |
| 42-50 | PPGASAYGS (SEQ ID NO: 16) | 36744.7 | 35 | 35 | 0 | |

**Table 3-12**

| | HLA-A*29:02 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 184 | 0.8 | 0.8 | 1 | WB |
| 39-47 | DFAPPGASA (SEQ ID NO: 14) | 19614.6 | 17 | 17 | 0 | |
| 37-45 | VLDFAPPGA (SEQ ID NO: 5) | 24281 | 24 | 24 | 0 | |
| 34-42 | WAPVLDFAP (SEQ ID NO: 10) | 25989.9 | 27 | 27 | 0 | |
| 36-44 | PVLDFAPPG (SEQ ID NO: 12) | 27778.7 | 31 | 31 | 0 | |
| 38-46 | LDFAPPGAS (SEQ ID NO: 13) | 29371.2 | 35 | 35 | 0 | |
| 43-51 | PGASAYGSL (SEQ ID NO: 17) | 30400 | 38 | 38 | 0 | |
| 42-50 | PPGASAYGS (SEQ ID NO: 16) | 32896.4 | 45 | 45 | 0 | |
| 35-43 | APVLDFAPP (SEQ ID NO: 11) | 34482.1 | 55 | 55 | 0 | |
| 41-49 | APPGASAYG (SEQ ID NO: 15) | 37238.6 | 65 | 65 | 0 | |

**Table 3-13**

| | HLA-A*30:02 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 476.3 | 1.2 | - 1.2 | 1 | WB |
| 43-51 | PGASAYGSL (SEQ ID NO: 17) | 9498.8 | 9 | 9 | 0 | |
| 37-45 | VLDFAPPGA (SEQ ID NO: 5) | 16240.6 | 19 | 19 | 0 | |
| 39-47 | DFAPPGASA (SEQ ID NO: 14) | 19390.7 | 25 | 25 | 0 | |
| 38-46 | LDFAPPGAS (SEQ ID NO: 13) | 20319.5 | 27 | 27 | 0 | |
| 41-49 | APPGASAYG (SEQ ID NO: 15) | 28217.7 | 55 | 55 | 0 | |
| 34-42 | WAPVLDFAP (SEQ ID NO: 10) | 29702.00 | 60 | 60 | 0 | |
| 36-44 | PVLDFAPPG (SEQ ID NO: 12) | 34189.3 | 80 | 80 | 0 | |
| 35-43 | APVLDFAPP (SEQ ID NO: 11) | 34784.1 | 80 | 80 | 0 | |
| 42-50 | PPGASAYGS (SEQ ID NO: 16) | 36229.1 | 85 | 85 | 0 | |

**Table 3-14**

| | HLA-A*32:15 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 3800 | 1 | 1 | 1 | WB |
| 34-42 | WAPVLDFAP (SEQ ID NO: 10) | 24071.2 | 11 | 11 | 0 | |
| 43-51 | PGASAYGSL (SEQ ID NO: 17) | 27792.6 | 14 | 14 | 0 | |
| 38-46 | LDFAPPGAS (SEQ ID NO: 13) | 28299.3 | 15 | 15 | 0 | |
| 36-44 | PVLDFAPPG (SEQ ID NO: 12) | 30592.1 | 17 | 17 | 0 | |
| 42-50 | PPGASAYGS (SEQ ID NO: 16) | 32223.5 | 20 | 20 | 0 | |
| 39-47 | DFAPPGASA (SEQ ID NO: 14) | 34561.3 | 24 | 24 | 0 | |
| 35-43 | APVLDFAPP (SEQ ID NO: 11) | 35026.6 | 24 | 24 | 0 | |
| 41-49 | APPGASAYG (SEQ ID NO: 15) | 35085.7 | 25 | 25 | 0 | |
| 37-45 | VLDFAPPGA (SEQ ID NO: 5) | 38286.1 | 32 | 32 | 0 | |

**Table 3-15**

| | HLA-A*68:23 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 42.5 | 0.09 | 0.09 | 1 | SB |
| 41-49 | APPGASAYG (SEQ ID NO: 15) | 9780 | 6 | 6 | 0 | |
| 35-43 | APVLDFAPP (SEQ ID NO: 11) | 14088.9 | 8 | 8 | 0 | |
| 34-42 | WAPVLDFAP (SEQ ID NO: 10) | 15965.6 | 9 | 9 | 0 | |
| 36-44 | PVLDFAPPG (SEQ ID NO: 12) | 23246.8 | 14 | 14 | 0 | |
| 38-46 | LDFAPPGAS (SEQ ID NO: 13) | 27813.3 | 18 | 18 | 0 | |
| 39-47 | DFAPPGASA (SEQ ID NO: 14) | 29240.6 | 20 | 20 | 0 | |
| 43-51 | PGASAYGSL (SEQ ID NO: 17) | 30832.3 | 21 | 21 | 0 | |
| 37-45 | VLDFAPPGA (SEQ ID NO: 5) | 38707.3 | 36 | 36 | 0 | |
| 42-50 | PPGASAYGS (SEQ ID NO: 16) | 41372.3 | 44 | 44 | 0 | |

**Table 3-16**

| | HLA-B*14:02 | | | | | |
|---|---|---|---|---|---|---|
| Pos | Peptide | nM | Rank | H_Avg_Ranks | N_binders | BindLevel |
| 39-47 | DFAPPGASA (SEQ ID NO: 14) | 2908.6 | 0.8 | 0.8 | 1 | WB |
| 40-48 | FAPPGASAY (SEQ ID NO: 6) | 4578.2 | 1.4 | 1.4 | 1 | WB |
| 43-51 | PGASAYGSL (SEQ ID NO: 17) | 8827.2 | 3.5 | 3.5 | 0 | |
| 38-46 | LDFAPPGAS (SEQ ID NO: 13) | 14592.8 | 7 | 7 | 0 | |
| 34-42 | WAPVLDFAP (SEQ ID NO: 10) | 18030.5 | 9.5 | 9.5 | 0 | |
| 35-43 | APVLDFAPP (SEQ ID NO: 11) | 30029 | 25 | 25 | 0 | |
| 37-45 | VLDFAPPGA (SEQ ID NO: 5) | 32746.5 | 31 | 31 | 0 | |
| 41-49 | APPGASAYG (SEQ ID NO: 15) | 37051.3 | 45 | 45 | 0 | |
| 36-44 | PVLDFAPPG (SEQ ID NO: 12) | 37097.9 | 46 | 46 | 0 | |
| 42-50 | PPGASAYGS (SEQ ID NO: 16) | 37168.6 | 46 | 46 | 0 | |

**Table 3-17**

| | HLA-8*15:01 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 259 | 1.5 | 1.5 | 1 | WB |
| 38-46 | LDFAPPGAS (SEQ ID NO: 13) | 24096.2 | 34 | 34 | 0 | |
| 43-51 | PGASAYGSL (SEQ ID NO: 17) | 25957.6 | 38 | 38 | 0 | |
| 36-44 | PVLDFAPPG (SEQ ID NO: 12) | 26720.4 | 40 | 40 | 0 | |
| 34-42 | WAPVLDFAP (SEQ ID NO: 10) | 26783.8 | 40 | 40 | 0 | |
| 35-43 | APVLDFAPP (SEQ ID NO: 11) | 27957.5 | 43 | 43 | 0 | |
| 39-47 | DFAPPGASA (SEQ ID NO: 14) | 28298.7 | 43 | 43 | 0 | |
| 41-49 | APPGASAYG (SEQ ID NO: 15) | 28770.5 | 45 | 45 | 0 | |
| 37-45 | VLDFAPPGA (SEQ ID NO: 5) | 30315.9 | 49 | 49 | 0 | |
| 42-50 | PPGASAYGS (SEQ ID NO: 16) | 42135.8 | 95 | 95 | 0 | |

**Table 3-18**

| | HLA-B*15:02 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 102 | 0.06 | 0.06 | 1 | SB |
| 38-46 | LDFAPPGAS (SEQ ID NO: 13) | 14206.1 | 6 | 6 | 0 | |
| 39-47 | DFAPPGASA (SEQ ID NO: 14) | 19197.2 | 8.5 | 8.5 | 0 | |
| 34-42 | WAPVLDFAP (SEQ ID NO: 10) | 20878.4 | 10 | 10 | 0 | |
| 43-51 | PGASAYGSL (SEQ ID NO: 17) | 30270.4 | 19 | 19 | 0 | |
| 37-45 | VLDFAPPGA (SEQ ID NO: 5) | 34108.4 | 26 | 26 | 0 | |
| 36-44 | PVLDFAPPG (SEQ ID NO: 12) | 34217.5 | 26 | 26 | 0 | |
| 42-50 | PPGASAYGS (SEQ ID NO: 16) | 35431.4 | 28 | 28 | 0 | |
| 41-49 | APPGASAYG (SEQ ID NO: 15) | 36043 | 30 | 30 | 0 | |
| 35-43 | APVLDFAPP (SEQ ID NO: 11) | 39098.3 | 38 | 38 | 0 | |

**Table 3-19**

| | HLA-B*15:03 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 27.5 | 0.4 | 0.4 | 1 | SB |
| 38-46 | LDFAPPGAS (SEQ ID NO: 13) | 16691.3 | 19 | 19 | 0 | |
| 39-47 | DFAPPGASA (SEQ ID NO: 14) | 18287.3 | 21 | 21 | 0 | |
| 43-51 | PGASAYGSL (SEQ ID NO: 17) | 19303 | 22 | 22 | 0 | |
| 35-43 | APVLDFAPP (SEQ ID NO: 11) | 24333.1 | 28 | 28 | 0 | |
| 36-44 | PVLDFAPPG (SEQ ID NO: 12) | 24529 | 29 | 29 | 0 | |
| 34-42 | WAPVLDFAP (SEQ ID NO: 10) | 31768.6 | 42 | 42 | 0 | |
| 37-45 | VLDFAPPGA (SEQ ID NO: 5) | 34247.8 | 48 | 48 | 0 | |
| 41-49 | APPGASAYG (SEQ ID NO: 15) | 40294.4 | 70 | 70 | 0 | |
| 42-50 | PPGASAYGS (SEQ ID NO: 16) | 40970.5 | 75 | 75 | 0 | |

**Table 3-20**

| | HLA-B*15:17 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 334.1 | 1.2 | 1.2 | 1 | WB |
| 34-42 | WAPVLDFAP (SEQ ID NO: 10) | 31342.9 | 21 | 21 | 0 | |
| 43-51 | PGASAYGSL (SEQ ID NO: 17) | 33915.2 | 25 | 25 | 0 | |
| 36-44 | PVLDFAPPG (SEQ ID NO: 12) | 34381.5 | 26 | 26 | 0 | |
| 37-45 | VLDFAPPGA (SEQ ID NO: 5) | 36854.2 | 31 | 31 | 0 | |
| 38-46 | LDFAPPGAS (SEQ ID NO: 13) | 38877.7 | 37 | 37 | 0 | |
| 35-43 | APVLDFAPP (SEQ ID NO: 11) | 39501.8 | 40 | 40 | 0 | |
| 41-49 | APPGASAYG (SEQ ID NO: 15) | 40664 | 45 | 45 | 0 | |
| 39-47 | DFAPPGASA (SEQ ID NO: 14) | 43002.1 | 60 | 60 | 0 | |
| 42-50 | PPGASAYGS (SEQ ID NO: 16) | 43983.7 | 65 | 65 | 0 | |

**Table 3-21**

| | HLA-B*27:20 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 2671.1 | 1.6 | 1.6 | 1 | WB |
| 36-44 | PVLDFAPPG (SEQ ID NO: 12) | 19402.1 | 7.5 | 7.5 | 0 | |
| 43-51 | PGASAYGSL (SEQ ID NO: 17) | 22128.9 | 8.5 | 8.5 | 0 | |
| 38-46 | LDFAPPGAS (SEQ ID NO: 13) | 26299.1 | 11 | 11 | 0 | |
| 35-43 | APVLDFAPP (SEQ ID NO: 11) | 29886 | 13 | 13 | 0 | |
| 39-47 | DFAPPGASA (SEQ ID NO: 14) | 30243.2 | 13 | 13 | 0 | |
| 42-50 | PPGASAYGS (SEQ ID NO: 16) | 39310 | 24 | 24 | 0 | |
| 34-42 | WAPVLDFAP (SEQ ID NO: 10) | 39469.8 | 24 | 24 | 0 | |
| 37-45 | VLDFAPPGA (SEQ ID NO: 5) | 42790.5 | 32 | 32 | 0 | |
| 41-49 | APPGASAYG (SEQ ID NO: 15) | 44427.1 | 38 | 38 | 0 | |

**Table 3-22**

| | HLA-B*35:01 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 4.1 | 0.01 | 0.01 | . 1 | SB |
| 34-42 | WAPVLDFAP (SEQ ID NO: 10) | 2405.9 | 3 | 3 | 0 | |
| 35-43 | APVLDFAPP (SEQ ID NO: 11) | 3175.3 | 3.5 | 3.5 | 0 | |
| 41-49 | APPGASAYG (SEQ ID NO: 15) | 10857.1 | 8.5 | 8.5 | 0 | |
| 36-44 | PVLDFAPPG (SEQ ID NO: 12) | 14715.2 | 12 | 12 | 0 | |
| 38-46 | LDFAPPGAS (SEQ ID NO: 13) | 18001.8 | 15 | 15 | 0 | |
| 42-50 | PPGASAYGS (SEQ ID NO: 16) | 18182.3 | 15 | 15 | 0 | |
| 39-47 | DFAPPGASA (SEQ ID NO: 14) | 24061.3 | 22 | 22 | 0 | |
| 43-51 | PGASAYGSL (SEQ ID NO: 17) | 24318.6 | 23 | 23 | 0 | |
| 37-45 | VLDFAPPGA (SEQ ID NO: 5) | 28029.3 | 29 | 29 | 0 | |

**Table 3-23**

| | HLA-B*35:03 | | | | | |
|---|---|---|---|---|---|---|
| Pos | Peptide | nM | Rank | H_Avg_Ranks | N_binders | BindLevel |
| 35-43 | APVLDFAPP (SEQ ID NO: 11) | 14423.9 | 0.9 | 0.9 | 1 | WB |
| 40-48 | FAPPGASAY (SEQ ID NO: 6) | 25043.6 | 2.5 | 2.5 | 0 | |
| 42-50 | PPGASAYGS (SEQ ID NO: 16) | 29702 | 4 | 4 | 0 | |
| 43-51 | PGASAYGSL (SEQ ID NO: 17) | 33894.7 | 6 | 6 | 0 | |
| 41-49 | APPGASAYG (SEQ ID NO: 15) | 34221.1 | 6 | 6 | 0 | |
| 36-44 | PVLDFAPPG (SEQ ID NO: 12) | 39520.6 | 12 | 12 | 0 | |
| 39-47 | DFAPPGASA (SEQ ID NO: 14) | 40719.5 | 14 | 14 | 0 | |
| 38-46 | LDFAPPGAS (SEQ ID NO: 13) | 43353 | 22 | 22 | 0 | |
| 34-42 | WAPVLDFAP (SEQ ID NO: 10) | 46695.7 | 55 | 55 | 0 | |
| 37-45 | VLDFAPPGA (SEQ ID NO: 5) | 47311.1 | 65 | 65 | 0 | |

**Table 3-24**

| | HLA-B*46:01 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 75 | 0.01 | 0.01 | 1 | SB |
| 34-42 | WAPVLDFAP (SEQ ID NO: 10) | 24567.2 | 12 | 12 | 0 | |
| 39-47 | DFAPPGASA (SEQ ID NO: 14) | 27516.4 | 18 | 18 | 0 | |
| 43-51 | PGASAYGSL (SEQ ID NO: 17) | 29515.8 | 25 | 25 | 0 | |
| 41-49 | APPGASAYG (SEQ ID NO: 15) | 31863.3 | 36 | 36 | 0 | |
| 35-43 | APVLDFAPP (SEQ ID NO: 11) | 32321.3 | 39 | 39 | 0 | |
| 37-45 | VLDFAPPGA (SEQ ID NO: 5) | 32954.1 | 43 | 43 | 0 | |
| 38-46 | LDFAPPGAS (SEQ ID NO: 13) | 33568 | 48 | 48 | 0 | |
| 36-44 | PVLDFAPPG (SEQ ID NO: 12) | 35027.7 | 60 | 60 | 0 | |
| 42-50 | PPGASAYGS (SEQ ID NO: 16) | 35837.3 | 70 | 70 | 0 | |

**Table 3-25**

| | HLA-B*83:01 | | | | | |
|---|---|---|---|---|---|---|
| Pos | Peptide | nM | Rank | H_Avg_Ranks | N_binders | BindLevel |
| 35-43 | APVLDFAPP (SEQ ID NO: 11) | 4754.6 | 0.9 | 0.9 | 1 | WB |
| 41-49 | APPGASAYG (SEQ ID NO: 15) | 5166.5 | 1 | 1 | 1 | WB |
| 40-48 | FAPPGASAY (SEQ ID NO: 6) | 8771.5 | 2.5 | 2.5 | 0 | |
| 39-47 | DFAPPGASA (SEQ ID NO: 14) | 13992.8 | 5 | 5 | 0 | |
| 43-51 | PGASAYGSL (SEQ ID NO: 17) | 17578.4 | 7.5 | 7.5 | 0 | |
| 42-50 | PPGASAYGS (SEQ ID NO: 16) | 17948.0 | 8 | 8 | 0 | |
| 36-44 | PVLDFAPPG (SEQ ID NO: 12) | 27860.9 | 18 | 18 | 0 | |
| 37-45 | VLDFAPPGA (SEQ ID NO: 5) | 29065.5 | 20 | 20 | 0 | |
| 38-46 | LDFAPPGAS (SEQ ID NO: 13) | 29511.7 | 21 | 21 | 0 | |
| 34-42 | WAPVLDFAP (SEQ ID NO:10) | 31473.4 | 23 | 23 | 0 | |

**Table 3-26**

| | HLA-C*03:03 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 5 | 0.03 | 0.03 | 1 | SB |
| 43-51 | PGASAYGSL (SEQ ID NO: 17) | 2210.3 | 2.5 | 2.5 | 0 | |
| 39-47 | DFAPPGASA (SEQ ID NO: 14) | 7040.2 | 4 | 4 | 0 | |
| 34-42 | WAPVLDFAP (SEQ ID NO: 10) | 11919.4 | 6 | 6 | 0 | |
| 41-49 | APPGASAYG (SEQ ID NO: 15) | 19973.3 | 9.5 | 9.5 | 0 | |
| 38-46 | LDFAPPGAS (SEQ ID NO: 13) | 21386.4 | 10 | 10 | 0 | |
| 36-44 | PVLDFAPPG (SEQ ID NO: 12) | 25003 | 12 | 12 | 0 | |
| 35-43 | APVLDFAPP (SEQ ID NO: 11) | 25582.6 | 13 | 13 | 0 | |
| 37-45 | VLDFAPPGA (SEQ ID NO: 5) | 43090.1 | 39 | 39 | 0 | |
| 42-50 | PPGASAYGS (SEQ ID NO: 16) | 45391.6 | 55 | 55 | 0 | |

**Table 3-27**

| | HLA-C*05:01 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 217.7 | 0.2 | 0.2 | 1 | SB |
| 37-45 | VLDFAPPGA (SEQ ID NO: 5) | 3969.3 | 1.3 | 1.3 | 1 | WB |
| 39-47 | DFAPPGASA (SEQ ID NO: 14) | 38919.3 | 22 | 22 | 0 | |
| 34-42 | WAPVLDFAP (SEQ ID NO: 10) | 40165.9 | 25 | 25 | 0 | |
| 36-44 | PVLDFAPPG (SEQ ID NO: 12) | 40587.5 | 26 | 26 | 0 | |
| 38-46 | LDFAPPGAS (SEQ ID NO: 13) | 41594.5 | 29 | 29 | 0 | |
| 43-51 | PGASAYGSL (SEQ ID NO: 17) | 42404.7 | 32 | 32 | 0 | |
| 41-49 | APPGASAYG (SEQ ID NO: 15) | 43017 | 35 | 35 | 0 | |
| 35-43 | APVLDFAPP (SEQ ID NO: 11) | 44302.3 | 41 | 41 | 0 | |
| 42-50 | PPGASAYGS (SEQ ID NO: 16) | 46246.2 | 60 | 60 | 0 | |

**Table 3-28**

| | HLA-C*07:01 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 3224.9 | 1.2 | 1.2 | 1 | WB |
| 43-51 | PGASAYGSL (SEQ ID NO: 17) | 35792.8 | 29 | 29 | 0 | |
| 41-49 | APPGASAYG (SEQ ID NO: 15) | 38247.6 | 36 | 36 | 0 | |
| 38-46 | LDFAPPGAS (SEQ ID NO: 13) | 39083.5 | 39 | 39 | 0 | |
| 39-47 | DFAPPGASA (SEQ ID NO: 14) | 41372.8 | 49 | 49 | 0 | |
| 37-45 | VLDFAPPGA (SEQ ID NO: 5) | 42518.2 | 55 | 55 | 0 | |
| 36-44 | PVLDFAPPG (SEQ ID NO: 12) | 43512.7 | 65 | 65 | 0 | |
| 34-42 | WAPVLDFAP (SEQ ID NO: 10) | 43854.0 | 65 | 65 | 0 | |
| 42-50 | PPGASAYGS (SEQ ID NO: 16) | 44885.6 | 75 | 75 | 0 | |
| 35-43 | APVLDFAPP (SEQ ID NO: 11) | 47243.1 | 95 | 95 | 0 | |

**Table 3-29**

| | HLA-C*07:02 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 373 | 0.13 | 0.125 | 1 | SB |
| 43-51 | PGASAYGSL (SEQ ID NO: 17) | 26322.1 | 13 | 13 | 0 | |
| 34-42 | WAPVLDFAP (SEQ ID NO: 10) | 30773.6 | 18 | 18 | 0 | |
| 38-46 | LDFAPPGAS (SEQ ID NO: 13) | 31055.3 | 18 | 18 | 0 | |
| 36-44 | PVLDFAPPG (SEQ ID NO: 12) | 32458.7 | 20 | 20 | 0 | |
| 39-47 | DFAPPGASA (SEQ ID NO: 14) | 33470.5 | 21 | 21 | 0 | |
| 37-45 | VLDFAPPGA (SEQ ID NO: 5) | 42048.3 | 46 | 46 | 0 | |
| 42-50 | PPGASAYGS (SEQ ID NO: 16) | 43404.1 | 55 | 55 | 0 | |
| 35-43 | APVLDFAPP (SEQ ID NO: 11) | 43974.7 | 60 | 60 | 0 | |
| 41-49 | APPGASAYG (SEQ ID NO: 15) | 45342.5 | 70 | 70 | 0 | |

**Table 3-30**

| | HLA-C:08:02 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 1331.8 | 0.13 | 0.125 | 1 | SB |
| 37-45 | VLDFAPPGA (SEQ ID NO: 5) | 14016.8 | 3 | 3 | 0 | |
| 43-51 | PGASAYGSL (SEQ ID NO: 17) | 31050.9 | 17 | 17 | 0 | |
| 34-42 | WAPVLDFAP (SEQ ID NO: 10) | 31738.1 | 18 | 18 | 0 | |
| 36-44 | PVLDFAPPG (SEQ ID NO: 12) | 33728.6 | 22 | 22 | 0 | |
| 38-46 | LDFAPPGAS (SEQ ID NO: 13) | 38716.5 | 37 | 37 | 0 | |
| 39-47 | DFAPPGASA (SEQ ID NO: 14) | 38999.4 | 38 | 38 | 0 | |
| 35-43 | APVLDFAPP (SEQ ID NO: 11) | 39089.4 | 38 | 38 | 0 | |
| 41-49 | APPGASAYG (SEQ ID NO: 15) | 41701.7 | 55 | 55 | 0 | |
| 42-50 | PPGASAYGS (SEQ ID NO: 16) | 42162.7 | 55 | 55 | 0 | |

**Table 3-31**

| | HLA-C:12:03 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 5.6 | 0.01 | 0.01 | 1 | SB |
| 39-47 | DFAPPGASA (SEQ ID NO: 14) | 11822.9 | 7.5 | 7.5 | 0 | |
| 38-46 | LDFAPPGAS (SEQ ID NO: 13) | 12684.9 | 8 | 8.0 | 0 | |
| 34-42 | WAPVLDFAP (SEQ ID NO: 10) | 27653.1 | 20 | 20 | 0 | |
| 43-51 | PGASAYGSL (SEQ ID NO: 17) | 28838.1 | 21 | 21 | 0 | |
| 36-44 | PVLDFAPPG (SEQ ID NO: 12) | 28911.2 | 21 | 21 | 0 | |
| 37-45 | VLDFAPPGA (SEQ ID NO: 5) | 38038.0 | 38 | 38 | 0 | |
| 41-49 | APPGASAYG (SEQ ID NO: 15) | 39806.4 | 43 | 43 | 0 | |
| 35-43 | APVLDFAPP (SEQ ID NO: 11) | 40463.4 | 45 | 45 | 0 | |
| 42-50 | PPGASAYGS (SEQ ID NO: 16) | 45790.7 | 75 | 75 | 0 | |

**Table 3-32**

| | HLA-C*14:02 | | | | | |
|---|---|---|---|---|---|---|
| Pos | Peptide | nM | Rank | H_Avg_Ranks | N_binders | BindLevel |
| 40-48 | FAPPGASAY (SEQ ID NO: 6) | 12.5 | 0.04 | 0.04 | 1 | SB |
| 43-51 | PGASAYGSL (SEQ ID NO: 17) | 9241.8 | 5.5 | 5.5 | 0 | |
| 39-47 | DFAPPGASA (SEQ ID NO: 14) | 17984.1 | 10 | 10 | 0 | |
| 36-44 | PVLDFAPPG (SEQ ID NO: 12) | 20920.5 | 12 | 12 | 0 | |
| 41-49 | APPGASAYG (SEQ ID NO: 15) | 24021.3 | 14 | 14 | 0 | |
| 38-46 | LDFAPPGAS (SEQ ID NO: 13) | 27454.8 | 17 | 17 | 0 | |
| 34-42 | WAPVLDFAP (SEQ ID NO: 10) | 30321.5 | 20 | 20 | 0 | |
| 35-43 | APVLDFAPP (SEQ ID NO: 11) | 31834.0 | 22 | 22 | 0 | |
| 37-45 | VLDFAPPGA (SEQ ID NO: 5) | 39194.4 | 37 | 37 | 0 | |
| 42-50 | PPGASAYGS (SEQ ID NO: 16) | 41316.9 | 44 | 44 | 0 | |

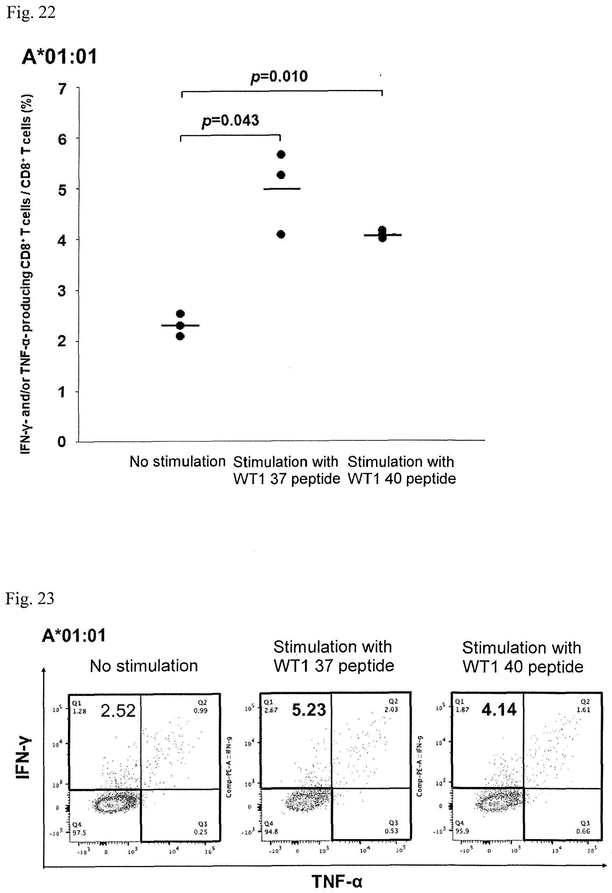

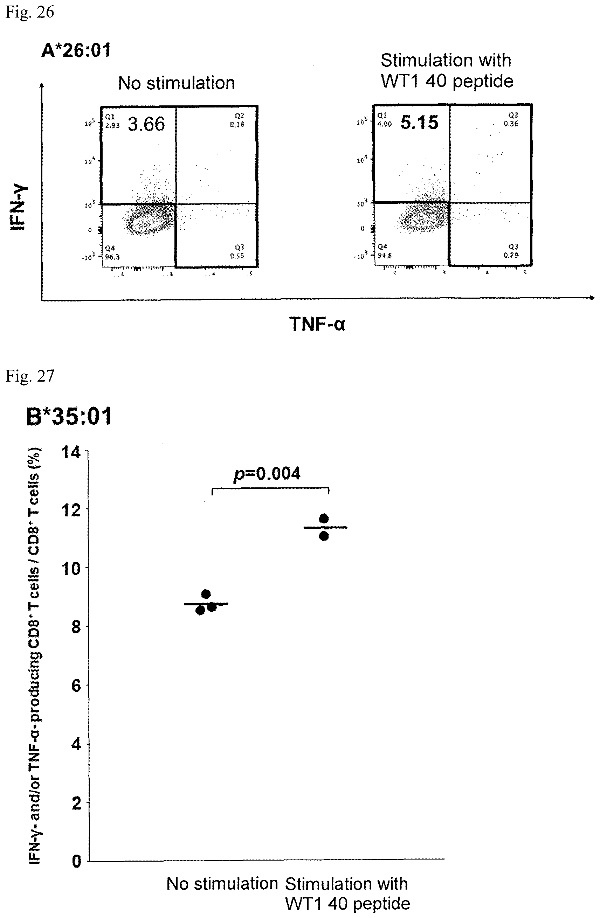

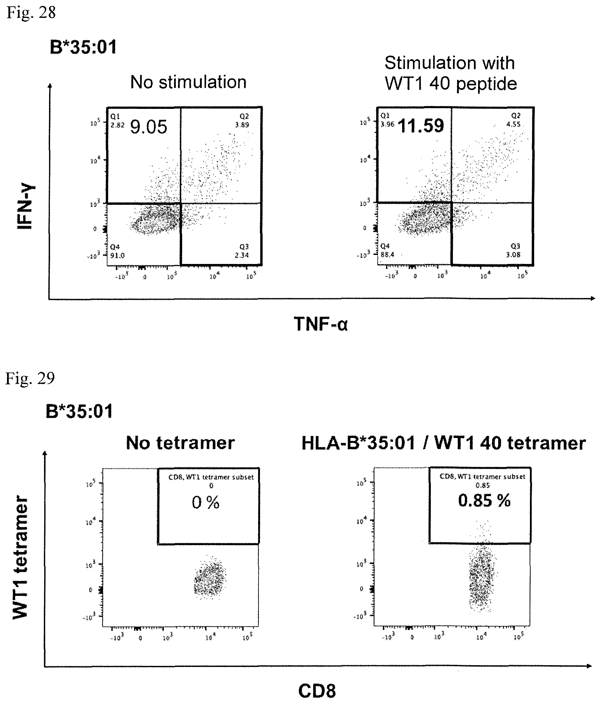

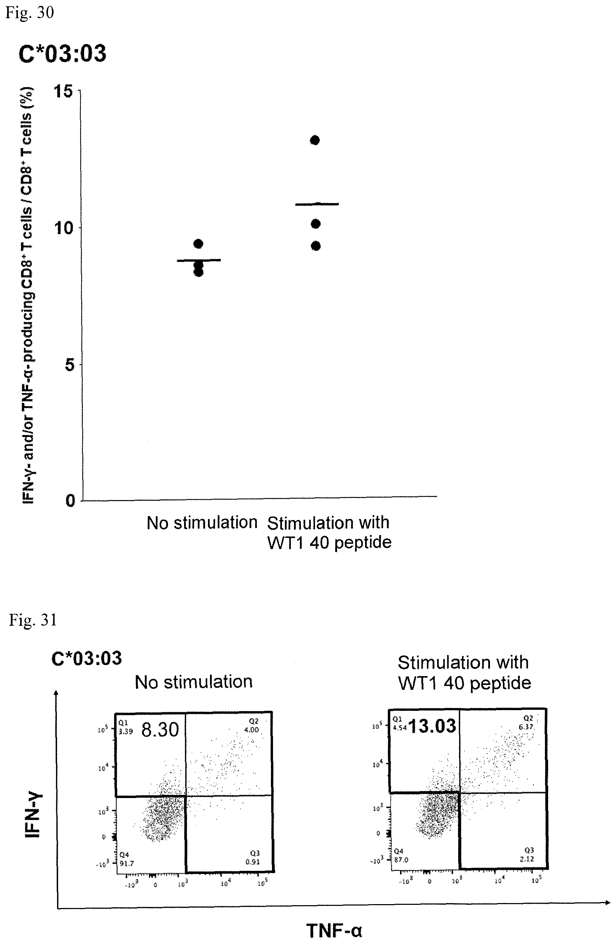

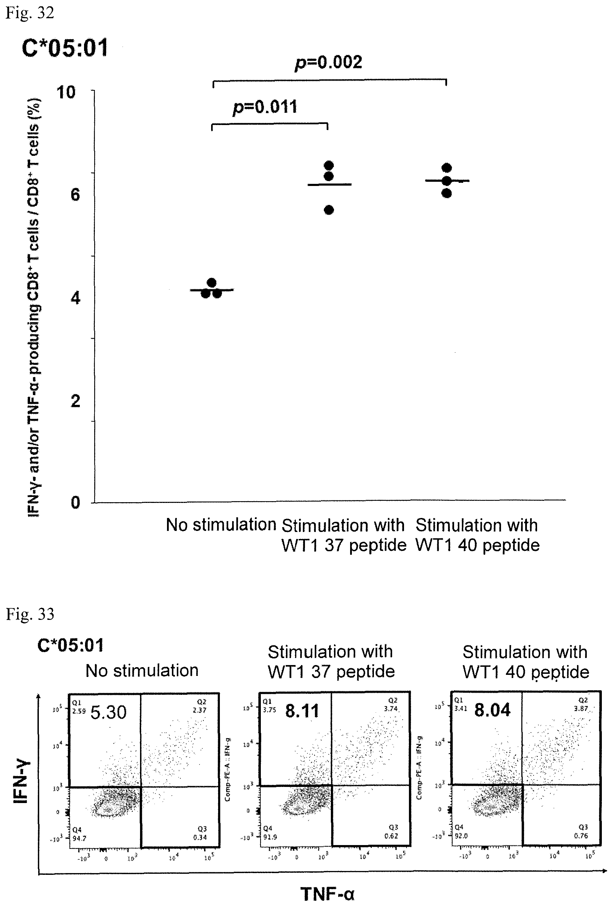

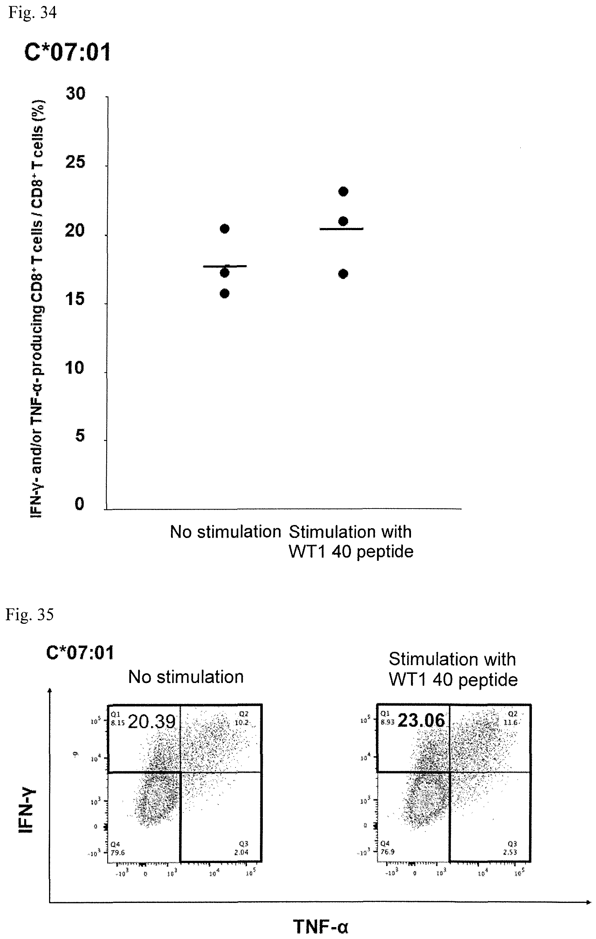

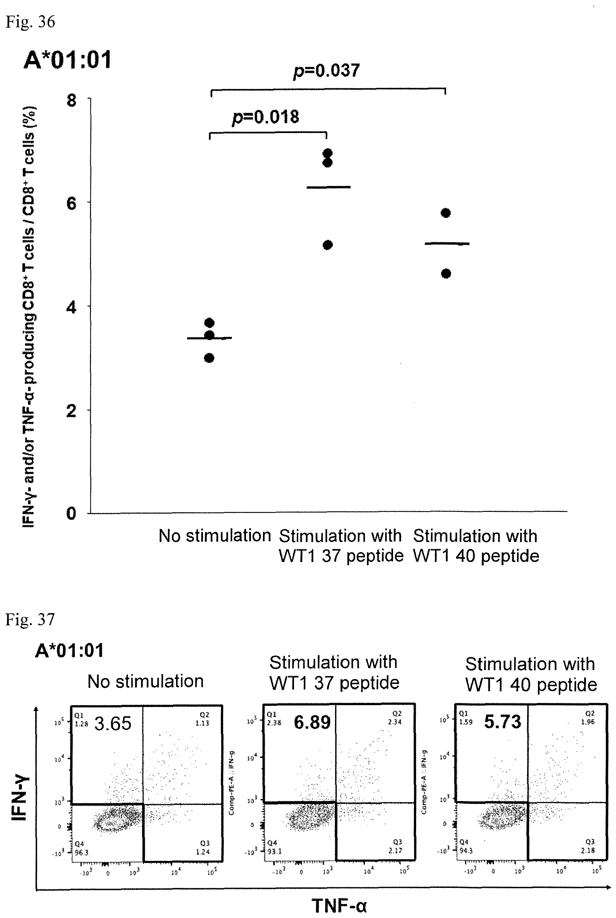

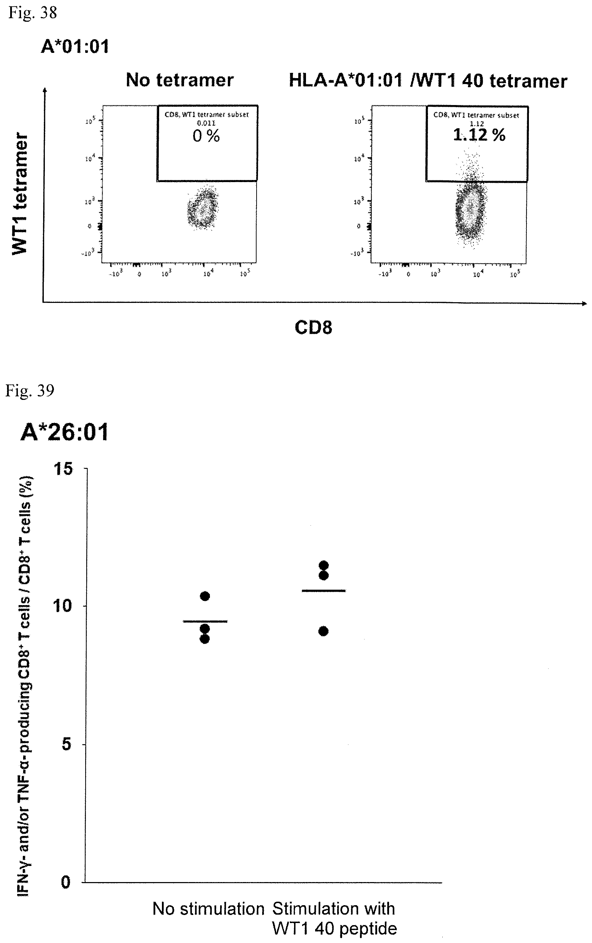

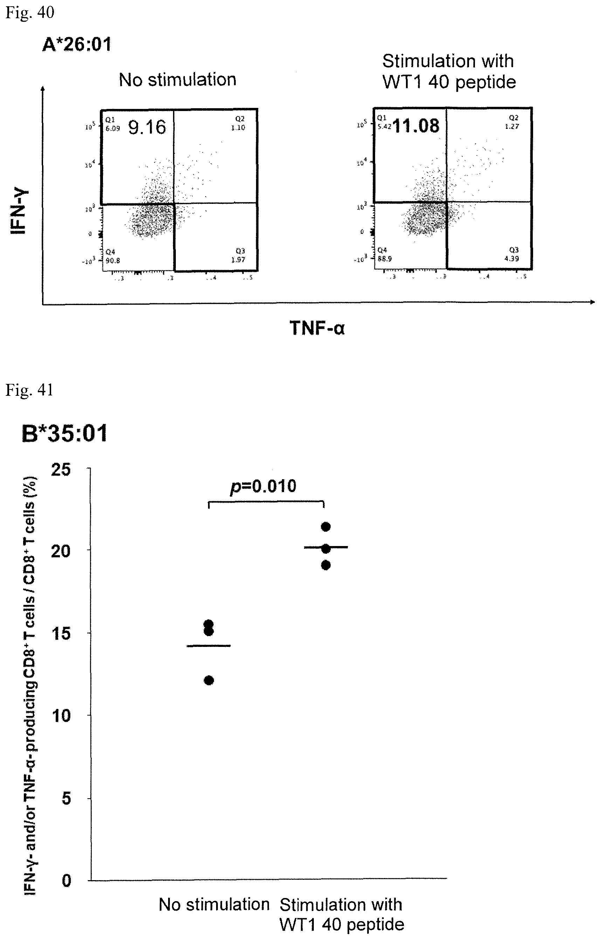

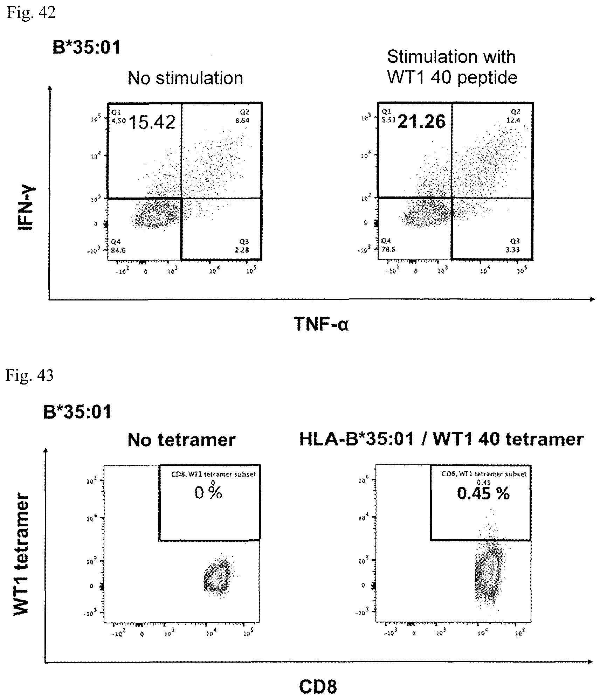

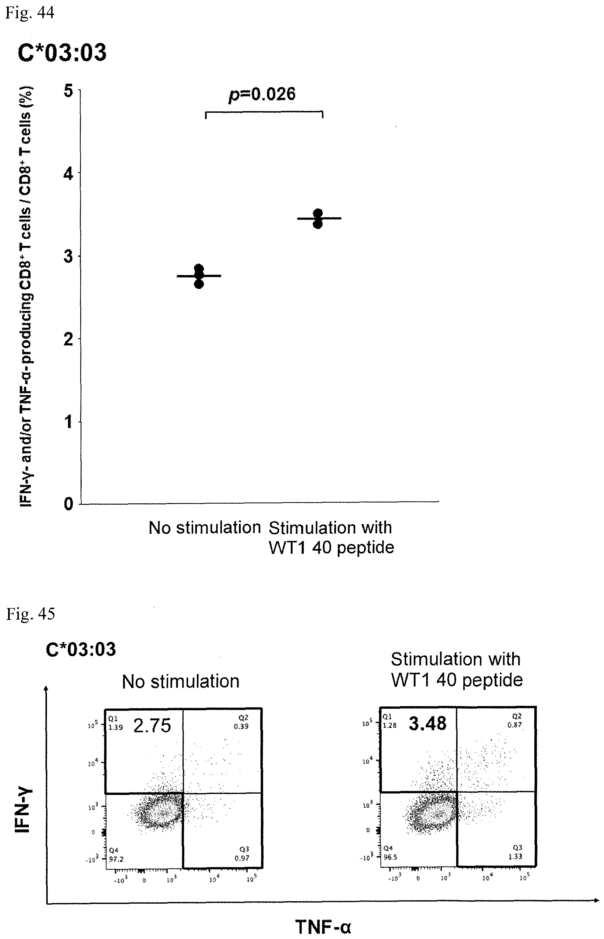

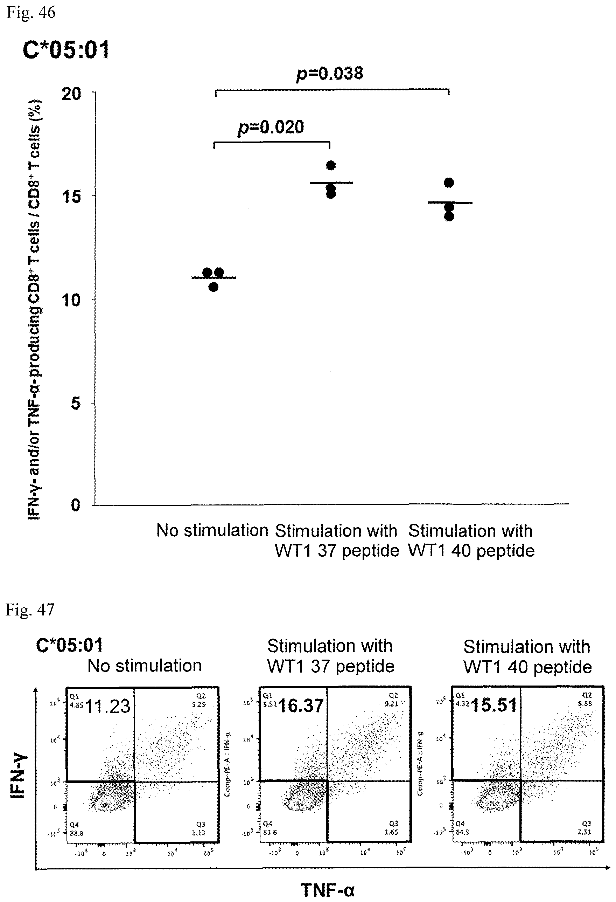

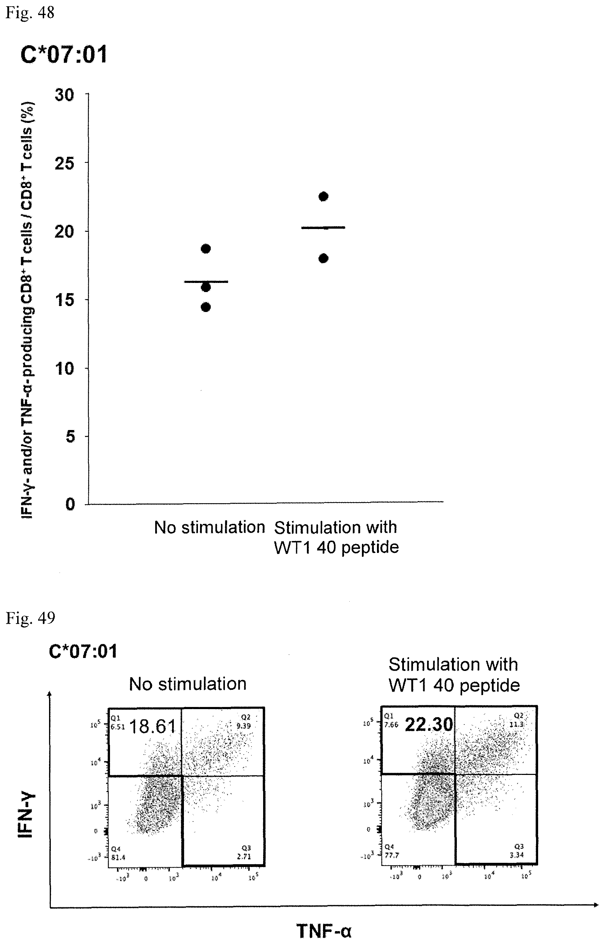

The results of Tables 4-1 and 4-2 are shown in Figs. 22 to 49.

**Table 4-1**

| **HLA class I-WT1 peptide** | | **HLA class I** | | **WT1 peptide used for three stimulations with PBMCs** | | | | | |
|---|---|---|---|---|---|---|---|---|---|
| | | | | **WT1 34 + Formula (5)** | | | **WT1 34** | | |
| **HLA class I** | **WT peptide** | **PBMCs for three stimulations** | **PBMCs for assay** | **Cytokine** | | **Tetramer** | **Cytokine** | | **Tetramer** |
| | | | | **WT1 37** | **WT1 40** | **WT1 40** | **WT1 37** | **WT1 40** | **WT1 40** |
| **A*01:01** | **WT1 37** | **A*01:01/02:01** | **A*01:01/29:02** | **Fig. 22** | **Fig. 23** | **Fig. 24** | **Fig. 36** | **Fig. 37** | **Fig. 38** |
| | **WT1 40** | **B*44:02158;01** | **B*13:02/57:01** | | | | | | |
| | | **C*05:01107:18** | **C*06:02/06:02** | | | | | | |
| **A*26:01** | **WT1 40** | **A*02:06/26:01** | **A*20:02/26:01** | **Fig.25** | **Fig.26** | | **Fig.39** | **Fig.40** | |
| | | **B*40:02/54:01** | **B*44:02/52:01** | | | | | | |
| | | **C*01:02/03:04** | **C`05:01116:01** | | | | | | |
| **B*35'01** | **WT140** | **A*02:02/03:01** | **A*31:01168:02** | **Fig. 27** | **Fig. 28** | **Fig. 29** | **Fig. 41** | **Fig.42** | **Fig.43** |
| | | **8*35:01/44:02** | **8*35:01/47:03** | | | | | | |
| | | **C*04:01/05:01** | **C*07:18/15:05** | | | | | | |

**Table 4-2**

| **HLA class I** - **WT1 peptide** | | **HLA class I** | | **WT1 peptide used for three stimulations with PBMCs** | | | | | |
|---|---|---|---|---|---|---|---|---|---|
| | | | | **WT1 34 + Formula (5)** | | | **WT1 34** | | |
| **HLA class I** | **WT peptide** | **PBMCs for three stimulations** | **PBMCs for assay** | **Cytokine** | | **Tetramer** | **Cytokine** | | **Tetramer** |
| | | | | **WT1 37** | **WT1 40** | **WT1 40** | **WT1 37** | **WT1 40** | **WT1 40** |
| **C*03:03** | **WT140** | A*01:01/68:01 | A*02:01103:01 | **Fig. 30** | **Fig. 31** | | **Fig. 44** | **Fig. 45** | |
| | | B*08:01/15:40 | B*27:05/55:01 | | | | | | |
| | | C*03:03/07:01 | C*01:02/03:03 | | | | | | |
| **C*05:01** | **WT1 37** | A*02:02/03:01 | A*23:01/30:02 | **Fig. 32** | **Fig. 33** | | **Fig. 46** | **Fig. 47** | |
| | | 8*35:01/44:02 | 8*18:01/53:01 | | | | | | |
| | **WT1 40** | C*04:01/05:01 | C*05:01/06:02 | | | | | | |
| **C*07:01** | **WT1 40** | A*33:03/68:02 | A`02:05123:01 | **Fig. 34** | **Fig. 35** | | **Fig. 48** | **Fig. 49** | |
| | | B*44:03/58:01 | B*08:01/53:01 | | | | | | |
| | | C*07:01/14:03 | C*04:01/07:01 | | | | | | |

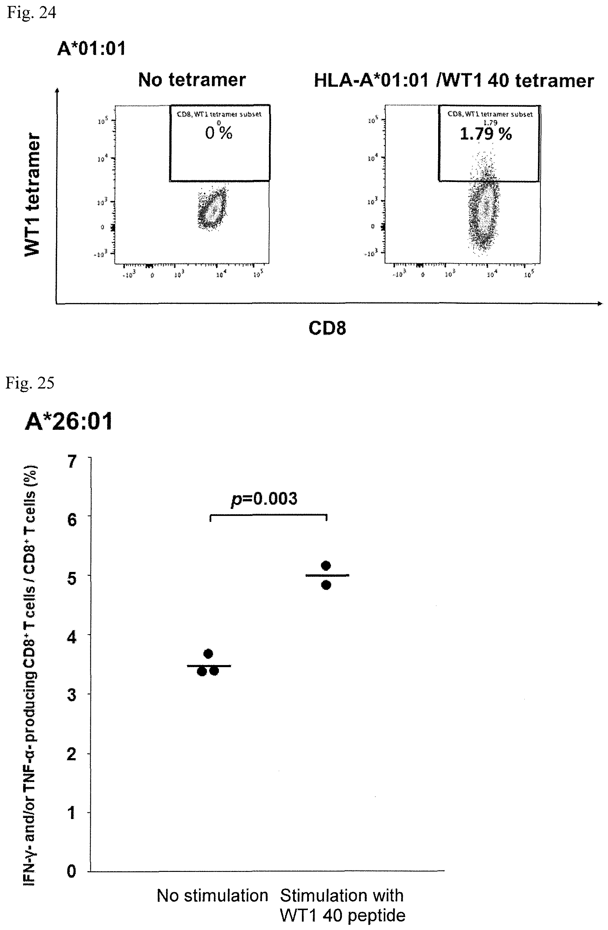

From PBMCs of an HLA-A*01:01-positive subject, HLA-A*26:01-positive subject, HLA-B*35:01-positive subject, HLA-C*03:03-positive subject, HLA-C*05:01-positive subject, or HLA-C*07:01 -positive subject stimulated with WT1₃₄₋₅₁ and the compound of formula (5) or WT1₃₄₋₅₁ alone, TNF-α- and/or INF-γ-producing CD8-positive cells were induced by WT1₃₇₋₄₅ or WT1₄₀₋₄₈ stimulation, indicating that WT1₃₇₋₄₅- or WT1₄₀₋₄₈-specific CD8-positive T cells were induced (Figs. 22, 23, 25, 26, 27, 28, 30 to 37, and 39 to 42). In the tetramer assay, PBMCs of an HLA-A*01:01-positive subject or HLA-B*35:01-positive subject stimulated with WT1₃₄₋₅₁ and the compound of formula (5) or WT1₃₄₋₅₁ alone showed increased tetramer positive cells (Figs. 24, 29, 38, and 43).

These results confirmed that PBMCs stimulated with WT1₃₄₋₅₁ and the compounds of formula (5) or WT1₃₄₋₅₁ alone induced HLA-A*01:01, HLA-A*26:01, HLA-B*35:01, HLA-C*03:03, HLA-C*05:01, or HLA-C*07:01-restricted WT1₃₇₋₄₅-specific CD8-positive T cells and/or WT1₄₀₋₄₈-specific CD8-positive T cells.

IV. Induction of WT1-specific CD8-positive T cells with WT1₃₄₋₅₁ and the compound of formula (5) or with WT1₃₄₋₅₁ (3)

PBMCs are obtained from peripheral blood of an HLA-A* 11:01 -positive subject, HLA-A*26:03-positive subject, HLA-A* 15:01-positive subject, or HLA-C*07:02-positive subject, and the intracellular cytokine assay and/or tetramer assay is performed as described in Section I, II, or III above. The results confirm that PBMCs stimulated with WT1₃₄₋₅₁ and the compound of formula (5) or WT1₃₄₋₅₁ alone induces HLA-A*11:01, HLA-A*26:03, HLA-A*15:01, or HLA-C*07:02-restricted WT1₃₇₋₄₅-specific CD8-positive T cells and/or WT1₄₀₋₄₈-specific CD8-positive T cells.

## Claims

1. A pharmaceutical composition for treating or preventing a cancer in an HLA-A*02:01, HLA-A*02:06, HLA-A*02:07, HLA-A*26:01, HLA-A*01:01, HLA-B*35:01, HLA-C*03:03, HLA-C*05:01, or HLA-C*07:01 -positive subject comprising a peptide consisting of the amino acid sequence of WAPVLDFAPPGASAYGSL (SEQ ID NO: 2) or an altered peptide thereof having an ability to induce CTLs or a pharmaceutically acceptable salt thereof.

2. The pharmaceutical composition according to claim 1, wherein the subject is an HLA-A*02:01, HLA-A*02:06, HLA-A*02:07, or HLA-A*26:01 -positive subject.

3. The pharmaceutical composition according to claim 1, wherein the subject is an HLA-A*02:01, HLA-A*02:06, or HLA-A*02:07-positive subject.

4. The pharmaceutical composition according to claim 1, wherein the subject is an HLA-A*02:01-positive subject.

5. The pharmaceutical composition according to claim 1, wherein the subject is an HLA-A*02:06-positive subject.

6. The pharmaceutical composition according to claim 1, wherein the subject is an HLA-A*02:07-positive subject.

7. The pharmaceutical composition according to claim 1, wherein the subject is an HLA-A*26:01-positive subject.

8. The pharmaceutical composition according to claim 1, wherein the subject is an HLA-A*01:01, HLA-B*35:01, HLA-C*03:03, HLA-C*05:01, or HLA-C*07:01 -positive subject.

9. The pharmaceutical composition according to claim 1, wherein the subject is an HLA-A*01:01-positive subject.

10. The pharmaceutical composition according to claim 1, wherein the subject is an HLA-B*35:01-positive subject.

11. The pharmaceutical composition according to claim 1, wherein the subject is an HLA-C*03:03-positive subject.

12. The pharmaceutical composition according to claim 1, wherein the subject is an HLA-C*05:01 -positive subject.

13. The pharmaceutical composition according to claim 1, wherein the subject is an HLA-C*07:01 -positive subject.

14. The pharmaceutical composition according to any one of claims 1 to 13, wherein the pharmaceutical composition comprises the peptide consisting of the amino acid sequence of SEQ ID NO: 2 or a pharmaceutically acceptable salt thereof.

15. The pharmaceutical composition according to any one of claims 1 to 14, wherein the pharmaceutical composition further comprises
a compound of formula (4): 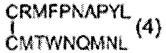 wherein C-C shown in the formula means that the C residues are linked together by a disulfide bond (SEQ ID NOs: 7 and 8, respectively), or a pharmaceutically acceptable salt thereof; or
a compound of formula (5): 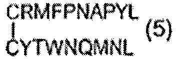 wherein C-C shown in the formula means that the C residues are linked together by a disulfide bond (SEQ ID NOs: 7 and 9, respectively), or a pharmaceutically acceptable salt thereof.

16. The pharmaceutical composition according to claim 15, wherein the pharmaceutical composition comprises the compound of formula (5): 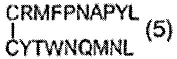 wherein C-C shown in the formula means that the C residues are linked together by a disulfide bond (SEQ ID NOs: 7 and 9, respectively), or a pharmaceutically acceptable salt thereof.

17. The pharmaceutical composition according to any one of claims 1 to 14, wherein the pharmaceutical composition is used in combination with
a compound of formula (4): 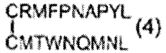 wherein C-C shown in the formula means that the C residues are linked together by a disulfide bond (SEQ ID NOs: 7 and 8, respectively), or a pharmaceutically acceptable salt thereof; or
a compound of formula (5): 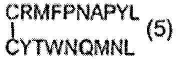 wherein C-C shown in the formula means that the C residues are linked together by a disulfide bond (SEQ ID NOs: 7 and 9, respectively), or a pharmaceutically acceptable salt thereof.

18. The pharmaceutical composition according to claim 17, wherein the pharmaceutical composition is used in combination with the compound of formula (5): 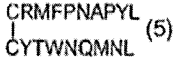 wherein C-C shown in the formula means that the C residues are linked together by a disulfide bond (SEQ ID NOs: 7 and 9, respectively), or a pharmaceutically acceptable salt thereof.

19. The pharmaceutical composition according to any one of claims 1 to 18, wherein the pharmaceutical composition comprises
the peptide consisting of the amino acid sequence of SEQ ID NO: 2 or a pharmaceutically acceptable salt thereof; and
a compound of formula (5): 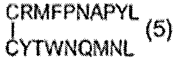 wherein C-C shown in the formula means that the C residues are linked together by a disulfide bond (SEQ ID NOs: 7 and 9, respectively), or a pharmaceutically acceptable salt thereof.

20. The pharmaceutical composition according to any one of claims 1 to 19, wherein the cancer is a cancer in which WT1 is expressed or a cancer associated with an elevated expression level of WT1 gene.

21. The pharmaceutical composition according to any one of claims 1 to 20, wherein the cancer is acute myeloid leukemia, chronic myeloid leukemia, acute lymphoblastic leukemia chronic lymphocytic leukemia, myelodysplastic syndrome, multiple myeloma, malignant lymphoma, stomach cancer, colon cancer, lung cancer, breast cancer, germ cell carcinoma, liver cancer, skin cancer, bladder cancer, prostate cancer, uterine cancer, cervical cancer, ovarian cancer, brain tumor or glioma.
